# EUROPEAN PATENT APPLICATION

(11) **EP 4 737 476 A1**
(43) Date of publication of application: **06.05.2026**
(21) Application number: 24199946.5
(22) Date of filing: 12.09.2024
(51) Int. Cl.: C07K 14/435, A61P 1/00, A61K 38/00, C12N 5/00, A61K 35/00, A61K 35/17, A61K 40/11, A61K 40/32, A61K 40/42, A61P 35/00, C07K 14/705, C07K 16/18, A61K 38/17, C07K 14/725, C07K 16/32, C12N 5/0783

(54) **T-CELL RECEPTOR VARIANT AGAINST MKRAS 7-16 G12D**

(71) Applicant: Medigene Immunotherapies GmbH, 82152 Planegg-Martinsried (DE)
(72) Inventor: Longinotti, Giulia, 81377 Munich (DE); Mohr, Anne-Wiebe, 81667 Munich (DE); Alterauge, Marc Dominik, 81375 Munich (DE); Bittmann, Julia Carola, 80637 Munich (DE)
(74) Representative: Schiweck Weinzierl Koch Patentanwälte Partnerschaft mbB

(57) **Abstract**

The present invention relates to a novel TCR characterized by its CDR3 sequences of the α chain variable region and/or the β chain variable region. The present invention relates to a TCR polypeptide complex and/or a combination comprising a novel TCR characterized by its CDR3 sequences of the α chain variable region and/or the β chain variable region. The present invention further relates to a nucleic acid, a vector as well as a host cell comprising said nucleic acid or said vector. The present invention relates to a method for obtaining said TCR and said chimeric co-stimulatory receptor and to a pharmaceutical or diagnostic composition comprising the abovementioned. The present invention further relates to the abovementioned for use as a medicament or for use in a method of diagnosing, detecting, preventing, and/or treating cancer. Furthermore, the present invention relates to a method of detecting the presence of a cancer in a subject *in vitro,* by applying within said method the abovementioned, and the use of the abovementioned for generating modified lymphocytes and a kit.

## Description

### SEQUENCE LISTING

This application contains a Sequence Listing in computer readable form, which is incorporated herein by reference.

### TECHNICAL FIELD OF THE INVENTION

The present invention relates to a novel TCR (TCR16) characterized by its CDR1-6 sequences of the α chain variable region and/or the β chain variable region. The present invention further relates to a nucleic acid encoding said TCR, a vector comprising said nucleic acid, as well as a host cell comprising said TCR, said nucleic acid or said vector. The present invention further relates to a method for obtaining said TCR and to a pharmaceutical or diagnostic composition comprising said TCR, said nucleic acid molecule, said vector, and/or said host cell. The present invention further relates to said TCR, said nucleic acid, said vector, said host cell and/or said pharmaceutical composition for use as a medicament, or for use in a method of diagnosing, detecting, preventing, and/or treating cancer. Furthermore, the present invention relates to a method of detecting the presence of a cancer in a subject *in vitro,* by applying within said method said TCR, said nucleic acid, said vector, said host cell, and/or said pharmaceutical composition and the use of said TCR, said nucleic acid, and/or said vector for generating modified lymphocytes. Finally, the present invention relates to a kit comprising said TCR, said nucleic acid, said vector, said host cell and/or said pharmaceutical or diagnostic composition.

The present invention further relates to a TCR polypeptide complex and/or a combination comprising a novel TCR characterized by its CDR sequences of the α chain variable region and/or the β chain variable region. The present invention further relates to a nucleic acid, a vector as well as a host cell comprising said nucleic acid or said vector. The present invention relates to a method for obtaining said TCR and said chimeric co-stimulatory receptor and to a pharmaceutical or diagnostic composition comprising the abovementioned. The present invention further relates to the abovementioned for use as a medicament or for use in a method of diagnosing, detecting, preventing, and/or treating cancer. Furthermore, the present invention relates to a method of detecting the presence of a cancer in a subject *in vitro,* by applying within said method the abovementioned, and the use of the abovementioned for generating modified lymphocytes and a kit.

### BACKGROUND OF THE INVENTION

T lymphocytes (or T cells) which form a part of the cell mediated immune system play a major role in the eradication of pathogens. T cells develop in the thymus and express T cell receptor molecules on their surface that allow the recognition of peptides presented on human leukocyte antigen (HLA) molecules which are expressed on nucleated cells (antigen presentation). Antigens of pathogens, i.e. foreign antigens presented by HLA molecules will elicit a powerful T cell response whereas self-antigens usually do not lead to a T cell response due to a negative selection of self-antigen specific T cells in the thymus during the development of such T cells. The immune system can thus discriminate between nucleated cells presenting foreign- or self-antigens or self, but aberrant antigens and specifically target and eradicate infected cells via potent cytokine release and cellular cytotoxicity mechanisms of the T cells.

The power of the immune system has been recognized as a promising tool for future cancer therapies. In the last decade, research has begun to exploit the unique properties of T cells by using adoptive cell transfer (ACT), which involves the administration of tumor infiltrating lymphocytes (TILs) which are patient-derived, expanded ex *vivo.* Although ACT has been shown to be a promising treatment for various types of cancer, its broad application as clinical treatment has been hampered by the need for custom isolation and characterization of tumor-specific T cells from each patient - a process that can be difficult and time-consuming but also often fails to yield high-avidity T cells (Xue et al., Clin Exp Immunol. 2005 February; 139(2): 167-172; Schmitt et al., Hum Gene Ther. 2009 November; 20(11): 1240-1248).

The genetic transfer of tumor antigen-specific TCRs into primary T cells can overcome some of the current limitations of ACT, as it allows for the rapid generation of tumor-reactive T lymphocytes with defined antigen specificity even in immunocompromised patients. However, the identification of suitable T cell clones bearing TCRs that specifically recognize tumor antigens and exhibit the desired anti-tumor effects *in vivo* is still the topic of ongoing research.

Tumors with Kirsten rat sarcoma (KRAS) mutations have been shown to create a tumor microenvironment (TME) that impairs immune cells in the TME and promotes tumor progression and immune escape. In the presence of KRAS mutations, increased levels of inflammatory cytokines necessary for tumorigenesis and progression have been observed in the TME. Under physiological conditions, the KRAS gene controls cell proliferation and survival. Activating mutations in the KRAS gene are highly prevalent oncogenic driver mutations in human cancers associated with tumorigenesis and aggressive tumor growth (Lee, J.K., et al., Precis. Onc., 2002, 6, 91). These mutations lead to the formation of so-called neoantigens, which are tumor-specific antigens (TSAs) that promote tumor growth and maintenance. KRAS has the highest mutation rate in a variety of frequently fatal solid cancers, including pancreatic ductal adenocarcinoma, endometrial cancer, non-small cell lung cancer, and colorectal cancer. The worldwide incidence of solid tumors harboring KRAS mutations is estimated to exceed 300,000 patients (Alexandrov LB, et al., Nature. 2020, 578(7793):94-101). The best-known mutations result from the substitution of only a single amino acid (G=glycine) at position 12 by aspartic acid (D), valine (V), or cysteine (C) (Yang Y, et al. J Clin Med. 2023;12(2):709). Considering that mutant KRAS (mKRAS) has been considered "undruggable" (Huang L, et al., Signal Transduct Target Ther. 2021 ;6(1):386), except for G12C mutations in some targeted therapies, novel and efficient treatment options for mKRAS tumors are urgently needed. It is the object of the present invention to comply with the needs set out above.

### SUMMARY OF THE INVENTION

This objective is *inter alia* accomplished by a novel T-cell receptor (TCR) characterized by its Complementarity Determining Region 1-6(CDR) sequences of the α chain variable region and/or the β chain variable region, such CDR3 which is the prime determinant of antigen recognition and specificity. Such TCR has additionally antigenic specificity to an particular mutated KRAS epitope or a fragment thereof or a variant thereof presented in the context of a major histocompatibility complex (MHC) class I molecule. In detail, it has specificity for the epitope comprising the amino acid sequence of SEQ ID NO: 1 (also referred to as mKRAS ₇₋₁₆ G12D - 10mer), meaning said TCR is capable of recognizing said epitope presented in the context of a MHC class I molecule in tumor.

Furthermore, said objective is *inter alia* accomplished by a T-cell receptor (TCR) polypeptide complex and/or a combination comprising a novel TCR characterized by its Complementarity Determining Region 3 (CDR3) sequences of the α chain variable region and/or the β chain variable region, such CDR3 which is the prime determinant of antigen recognition and specificity; and further comprising a chimeric co-stimulatory receptor. Such complex and/or combination is also expressed by a cell population comprising cells or by a cell per se.

In some aspects, the invention relates to a TCR comprising i) an α chain CDR3 comprising an amino acid sequence having at least 80 % identity to SEQ ID NO: 16; and/or ii) a β chain CDR3 comprising an amino acid sequence having at least 80 % identity to SEQ ID NO: 4.

In some aspects, the present invention relates to a nucleic acid comprising a nucleotide sequence encoding the TCR as mentioned above and defined herein.

In some aspects, the present invention relates to a vector comprising the nucleic acid as mentioned above and defined herein.

In some aspects, the present invention also relates to the host cell comprising the TCR, the nucleic acid or the vector as mentioned above and defined herein.

In some aspects, the present invention relates to a method for obtaining the TCR as mentioned above, comprising (i) incubating the host cell as defined above under conditions causing expression of said TCR; and (ii) purifying said TCR.

In some aspects, the present invention additionally relates to a pharmaceutical or diagnostic composition comprising one or more of: (i) the TCR; (ii) the nucleic acid; (iii) the vector; and/or (iv) the host cell as mentioned above and defined herein, and, optionally, pharmaceutically excipient(s).

In some aspects, the present invention relates to the TCR, the nucleic acid, the vector, the host cell and/or the pharmaceutical composition as mentioned above and defined herein for use as a medicament and/or for use in therapy.

In some aspects, the present invention relates to the TCR, the nucleic acid, the vector, the host cell and/or the pharmaceutical composition as mentioned above and defined herein for use in a method of detecting, diagnosing, prognosing, preventing and/or treating cancer.

In some aspects, the present invention also relates to a method of detecting the presence of a cancer in a subject *in vitro,* comprising (a) contacting a sample obtained from a subject and comprising one or more cells with (i) the TCR; (ii) the nucleic acid; (iii) the vector; (iv) the host cell, and/or (v) the pharmaceutical composition as mentioned above and defined herein, thereby forming a complex, and (b) detecting the complex, wherein detection of the complex is indicative of the presence of the cancer in the subject.

In some aspects, the present invention also relates to the use of the TCR, the nucleic acid and/or the vector as mentioned above and defined herein for generating modified lymphocytes.

In some aspects, the present invention relates to a kit comprising the TCR, the nucleic acid, the vector, the host cell, and/or the pharmaceutical or diagnostic composition as mentioned above and defined herein.

In some further aspects, the invention relates to a TCR polypeptide complex and/or a combination comprising: a) a TCR; and b) a chimeric co-stimulatory receptor, wherein the TCR comprises: i) an α chain CDR3 comprising an amino acid sequence having at least 80 % identity to SEQ ID NO: 16; and/or ii) a β chain CDR3 comprising an amino acid sequence having at least 80 % identity to SEQ ID NO: 4.

In some further aspects, the invention relates to fusion proteins comprising (a) an extracellular domain containing a polypeptide derived from PD-1 or CD40L at its N-terminus; (b) a transmembrane domain (e.g., derived from PD-1); and (c) an intracellular domain containing a polypeptide derived from 4-1BB or CD28 at its C-terminus. Also, fusion proteins with CD28 at the N-terminus and CD40L at the C-terminus are envisaged.

In some further aspects, the invention relates to a cell population comprising cells or a cell expressing: a) a TCR; and b) a chimeric co-stimulatory receptor, wherein the TCR comprises: i) an α chain CDR3 comprising an amino acid sequence having at least 80 % identity to SEQ ID NO: 16; and/or ii) a β chain CDR3 comprising an amino acid sequence having at least 80 % identity to SEQ ID NO: 4.

In some further aspects, the invention relates to a nucleic acid comprising a nucleotide sequence encoding the TCR as mentioned above and defined herein and a nucleotide sequence encoding the chimeric co-stimulatory receptor as mentioned above and defined herein.

In some further aspects, the invention relates to a vector comprising the nucleic acid as mentioned above and defined herein.

In some further aspects, the invention relates to a host cell comprising the nucleic acid or the vector as mentioned above and defined herein.

In some further aspects, the present invention also relates to a method for obtaining the TCR and the chimeric co-stimulatory receptor as mentioned above, comprising (i) incubating the host cell as defined above under conditions causing expression of said TCR and of said chimeric co-stimulatory receptor; and (ii) purifying said TCR and said chimeric co-stimulatory receptor.

In some further aspects, the invention relates to a pharmaceutical or diagnostic composition comprising one or more of: (i) the complex and/or the combination; (ii) the cell population or the cell, (iii) the nucleic acid; (iv) the vector; and/or (v) the host cell as mentioned above and defined herein; and, optionally, pharmaceutically excipient(s).

In some further aspects, the present invention relates to the complex and/or the combination, and/or the cell population or the cell, the nucleic acid, the vector, the host cell and/or the pharmaceutical composition as mentioned above and defined herein for use as a medicament and/or for use in therapy.

In some further aspects, the present invention relates to the complex and/or the combination, and/or the cell population or the cell, the nucleic acid, the vector, the host cell and/or the pharmaceutical composition as mentioned above and defined herein for use in a method of detecting, diagnosing, prognosing, preventing and/or treating cancer.

In some further aspects, the present invention also relates to a method of detecting the presence of a cancer in a subject *in vitro,* comprising (a) contacting a sample obtained from a subject and comprising one or more cells with (i) the complex and/or the combination; (ii) the cell population or the cell, (iii) the nucleic acid; (iv) the vector; (v) the host cell; and/or (vi) the pharmaceutical composition as mentioned above and defined herein, thereby forming a complex, and (b) detecting the complex, wherein detection of the complex is indicative of the presence of the cancer in the subject.

In some further aspects, the present invention also relates to the use of the complex and/or the combination, and/or the cell population or the cell, the nucleic acid and/or the vector as mentioned above and defined herein for generating modified lymphocytes.

In some further aspects, the present invention relates to a kit comprising the complex and/or the combination, and/or the cell population or the cell, the nucleic acid, the vector, the host cell, and/or the pharmaceutical or diagnostic composition as mentioned above and defined herein.

### BRIEF DESCRIPTION OF THE DRAWINGS

The invention will be better understood with reference to the detailed description when considered in conjunction with the non-limiting examples and the drawings, in which:

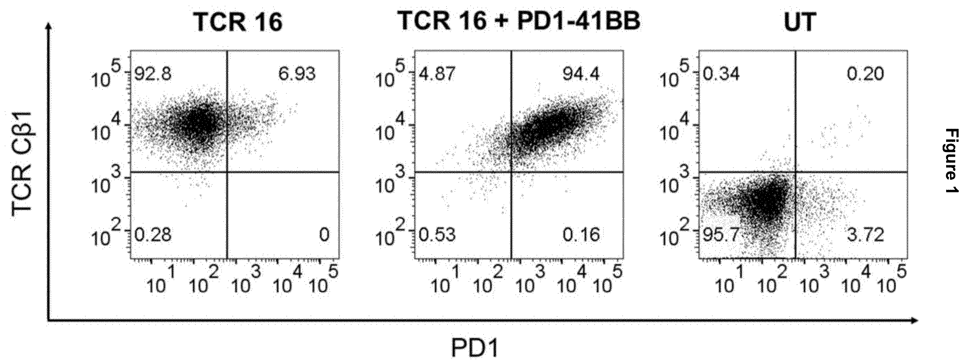

**Figure 1****. High and robust transgenic TCR and PD1-41BB CSP expression.** CD8⁺ T cells were isolated from healthy donors and activated with anti-CD3/CD28 beads in the presence of IL-2. The activated T cells were transduced with retroviral particles containing the transgene encoding either TCR 16 alone (TCR 16) or TCR 16 in combination with PD1-41BB CSP (TCR 16 + PD1-41BB). Untransduced CD8⁺ T cells (UT) were prepared in the same manner and used as controls. After transduction, CD8⁺ T cells were enriched using anti-CD8 and anti-TCR Cβ1 antibodies by fluorescence-activated cell sorting (FACS). To assess transduction efficiency and expression levels of the transgenes, T cell samples were stained with anti-TCR Cβ1, anti-PD1 and anti-CD8 antibodies and analyzed by flow cytometry. Populations shown are pre-gated on live single CD8⁺ T cells. Data of one representative experiment are shown.

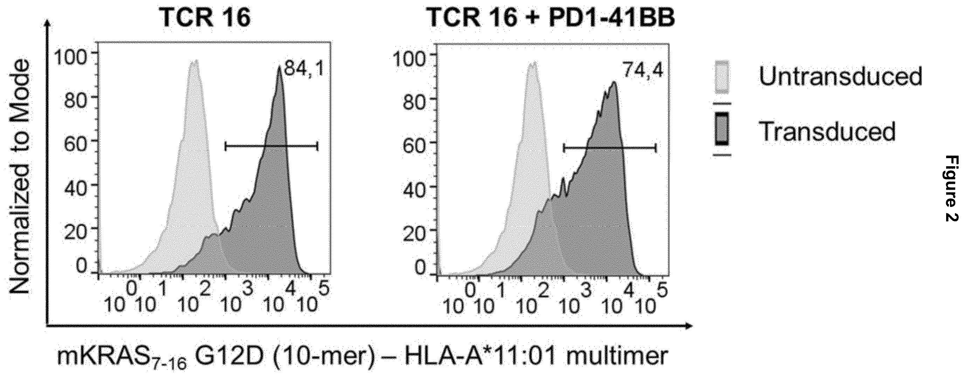

**Figure 2****. High and robust mKRAS₇₋₁₆ G12D (10-mer) multimer binding.** After enrichment and rapid expansion protocol, TCR 16-transduced CD8⁺ T cells (TCR 16) and TCR 16 + PD1-41BB-transduced CD8⁺ T cells (TCR 16 + PD1-41BB) were stained with mKRAS₇₋₁₆ G12D (10-mer) HLA-A*11:01 multimer and analyzed by flow cytometry. Untransduced CD8⁺ T cells were stained and analyzed in parallel as internal controls. Populations shown are pre-gated on live single cells. Data of one representative experiment are shown.

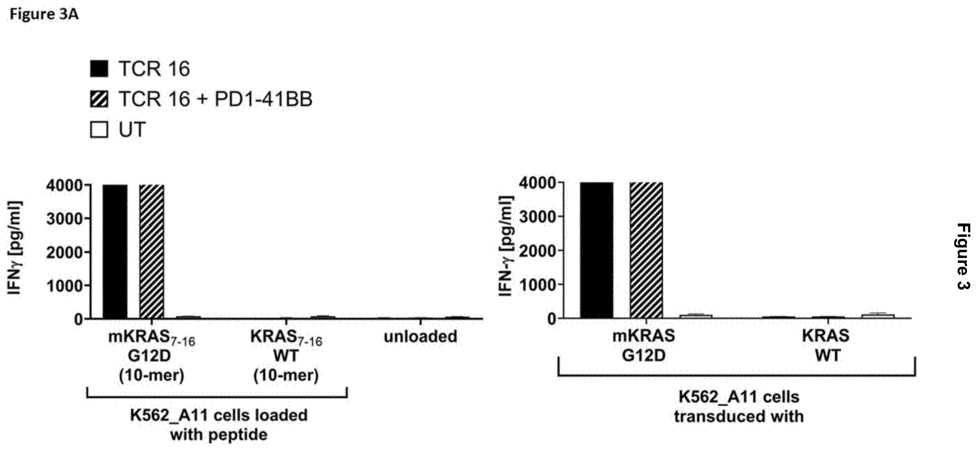

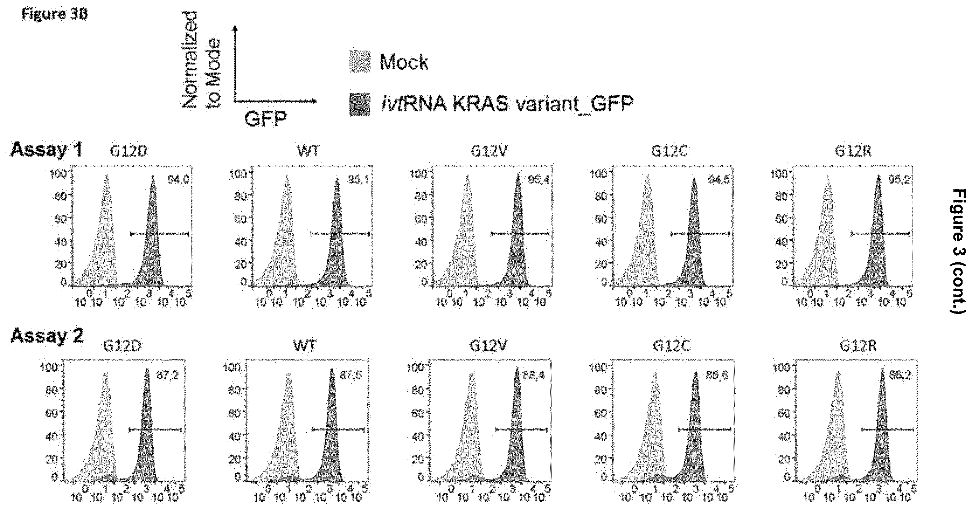

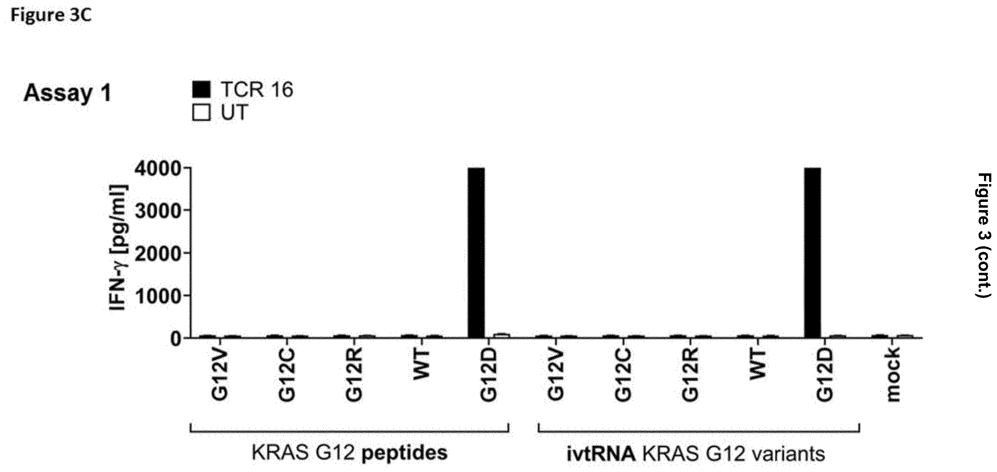

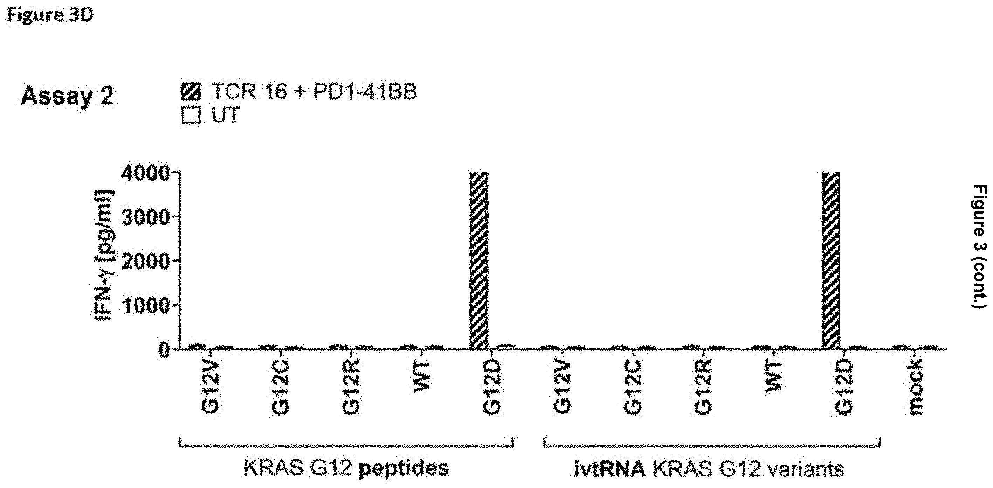

**Figure 3****. Exquisite mKRAS₇₋₁₆ G12D specificity with no KRAS wild-type recognition.** TCR 16-transduced CD8⁺ T cells (TCR 16) and TCR 16 + PD1-41BB-transduced CD8⁺ T cells (TCR 16 + PD1-41BB) were co-cultured with K562 cells expressing transgenic HLA-A*11:01 molecules (K562_A11) loaded with either mKRAS₇₋₁₆ G12D peptide (10-mer) or KRAS₇₋₁₆ wild type (WT, 10-mer) peptide at high concentration (10⁻⁵ M). Additionally, CD8⁺ T cells were also co-cultured with K562_A11 cells transduced with either a transgene encoding a fragment of mKRAS G12D gene (~90 bp) or a transgene encoding a fragment of KRAS G12 wild type (WT) gene (~90 bp). Unloaded K562_A11 cells served as target controls. Untransduced CD8⁺ T cells (UT) were included as negative controls. After 20 hours (h) of co-culture, an ELISA was performed to evaluate IFN-γ secretion by T cells. Shown is the mean value of duplicates with standard deviations (values are cut at 4000 pg/ml, upper limit of quantification). Data of one representative experiment are shown (Figure 3A). TCR 16-transduced CD8⁺ T cells (TCR 16, tested in assay 1) and TCR 16 + PD1-41BB-transduced CD8⁺ T cells (TCR 16 + PD1-41BB, tested in assay 2) were co-cultured with target cells either individually loaded (10⁻⁵ M) with mKRAS₇₋₁₆ G12V, mKRAS₇₋₁₆ G12C, mKRAS₇₋₁₆ G12R peptides or individually transfected with ivtRNA encoding gene fragments (~402 bp fragment spanning the specific mutation at position 12 and linked to GFP) of either mKRAS G12V, mKRAS G12C or mKRAS G12R. Loading of mKRAS₇₋₁₆ G12D peptide (10⁻⁵ M) and transfection with ivtRNA encoding mKRAS G12D gene fragment (~402 bp fragment spanning the specific mutation at position 12 and linked to GFP) served as positive target controls, whereas loading of KRAS₇₋₁₆ wild type peptide (10⁻⁵ M) and transfection with ivtRNA encoding KRAS wild gene fragment (~402 bp fragment spanning the position 12 and linked to GFP) were included as negative target controls. In addition, untransduced CD8⁺ T cells (UT) and water electroporation of target cells (mock) served as internal controls. The transfection efficiency of target cells was evaluated by flow cytometry analyzing GFP signals (data for assay 1 and assay 2, which were performed as independent assays, are shown) (Figure 3B). IFN-γ secretion was evaluated by ELISA 20 h after setting up the co-culture. Shown is the mean value of triplicates with standard deviations (values are cut at 4000 pg/ml, upper limit of quantification). Representative data for assay 1 and for assay 2 are shown. (Figure 3C and 3D).

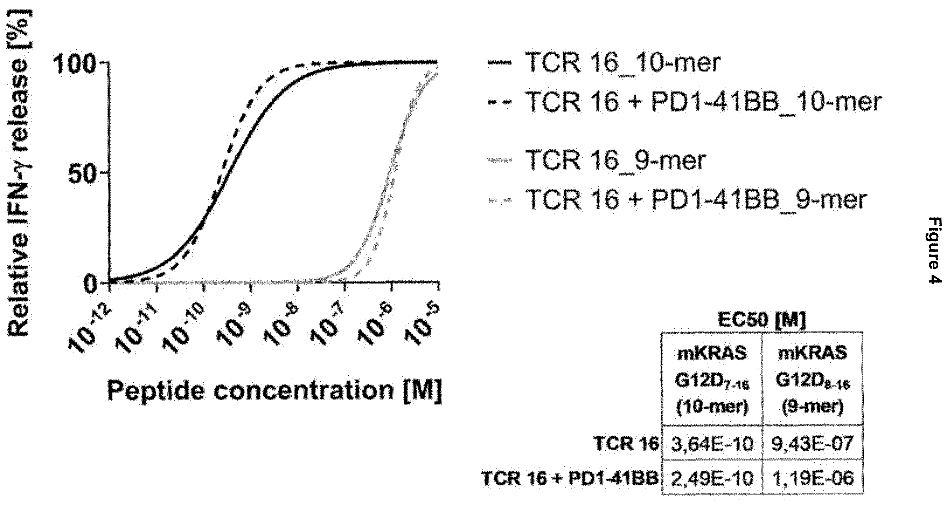

**Figure 4****. High peptide sensitivity for mKRAS₇₋₁₆ G12D (10-mer) epitope.** TCR 16-transduced CD8⁺ T cells (TCR 16) and TCR 16 + PD1-41BB-transduced CD8⁺ T cells (TCR 16 + PD1-41BB) were tested in a co-culture experiment using K562_A11 cells loaded with titrated amounts of either mKRAS₇₋₁₆ G12D (10-mer) peptide or mKRAS₈₋₁₆ G12D (9-mer) peptide (10⁻⁵ M to 10⁻¹² M) for direct comparison of peptide sensitivity for the two peptide length variants. After 20 h of co-culture, an ELISA was performed to evaluate IFN-γ secretion by T cells. Maximal IFN-γ release per effector cell sample was set to 100%. Based on this, the relative IFN-γ release was calculated. A nonlinear regression analysis was used to determine EC50 values (peptide concentration needed for half maximal relative IFN-γ secretion) for TCR16- and TCR 16 + PD1-41BB-transduced CD8⁺ T cells in response to the two mKRAS G12D peptide length variants. Data of one representative experiment are shown.

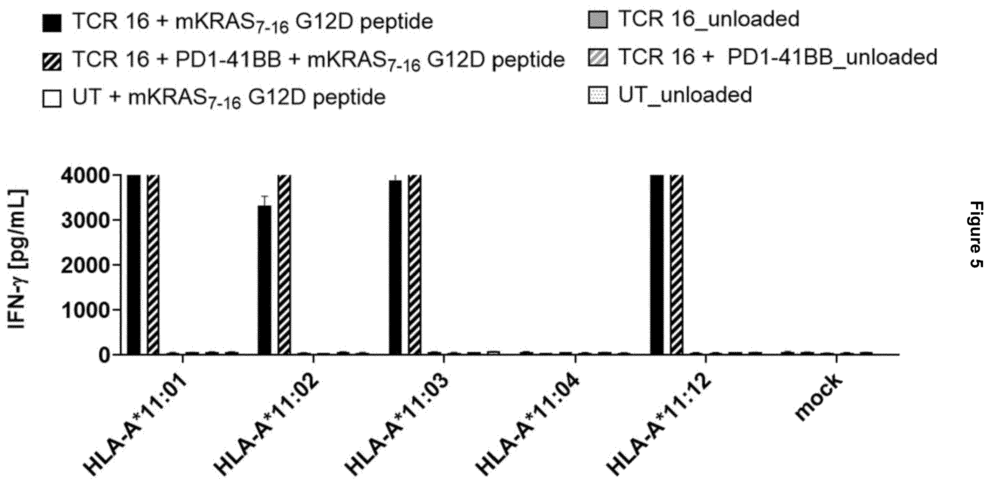

**Figure 5****. Peptide-specific restriction to four different HLA-A*11 subtypes.** CD8⁺ T cells expressing either no transgenic TCR (UT), TCR 16 alone (TCR 16) or the combination of TCR 16 and PD1-41BB CSP (TCR 16 + PD1-41BB) were co-cultured with a set of HLA-transduced K562 cell samples. Each K562 cell sample expressed one of the following transgenic HLA molecules: HLA-A*11:01, HLA-A*11:02, HLA-A*11:03, HLA-A*11:04, HLA-A*11:12. Each HLA-transduced K562 sample was tested either unloaded or after mKRAS₇₋₁₆ G12D peptide loading (10⁻⁵ M). Untransduced K562 cells (mock) were included as controls and tested both unloaded and after mKRAS₇₋₁₆G12D peptide loading. IFN-γ release was evaluated by ELISA 20 h after setting up the co-culture. Shown is the mean value of duplicates with standard deviations (values are cut at 4000 pg/ml, upper limit of quantification). Data of one representative experiment are shown.

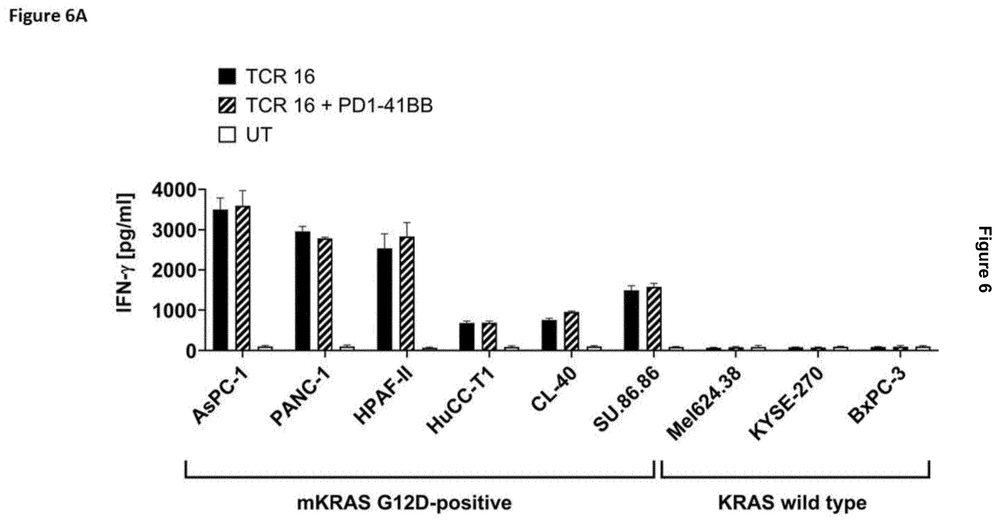

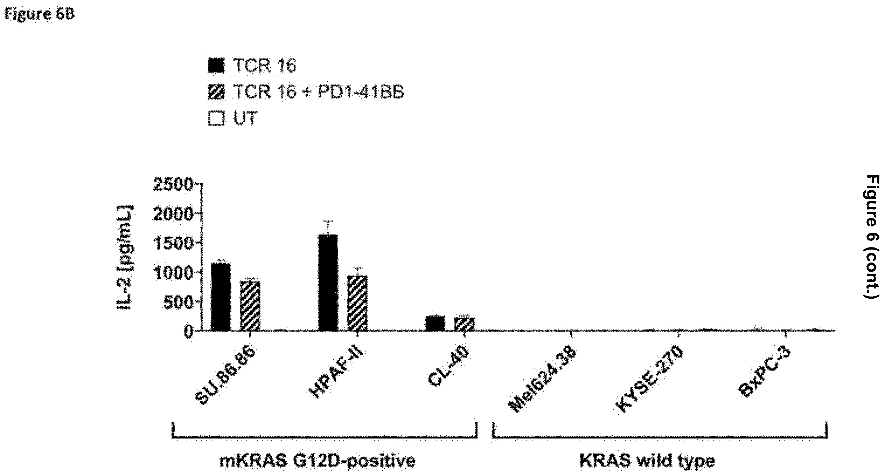

Figure 6. High and specific IFN-γ and IL-2 secretion in response to mKRAS G12D-positive tumor cells. The first experiment was set up using tumor cell lines derived from various indications expressing either mKRAS G12D (AsPC-1, PANC-1, HPAF-II, HuCC-T1, CL-40, SU.86.86) or KRAS wild type (Mel624.38, KYSE-270, BxPC-3) in co-culture with TCR 16- and TCR 16 + PD1-41BB-transduced CD8⁺ T cells (TCR 16 and TCR 16 + PD1-41BB). Untransduced CD8⁺ T cells (UT) served as negative control. IFN-γ secretion was assessed 20 h after setting up the co-culture. Shown is the mean value of triplicates with standard deviations (values are cut at 4000 pg/ml, upper limit of quantification). Data of one representative experiment is shown (Figure 6A). The second experiment was set up using SU.86.86, HPAF-II, CL-40 tumor cells (mKRAS G12D-positive) and Mel624.38, KYSE-270, BxPC-3 tumor cells (KRAS wild type) in co-culture with TCR 16- and TCR 16 + PD1-41BB-transduced CD8⁺ T cells (TCR 16 and TCR 16 + PD1-41BB). Untransduced CD8⁺ T cells (UT) served as negative control. IL-2 secretion was assessed 20 h after setting up the co-culture. Shown is the mean value of duplicates with standard deviations. Data of one representative experiment are shown (**Figure 6B**).

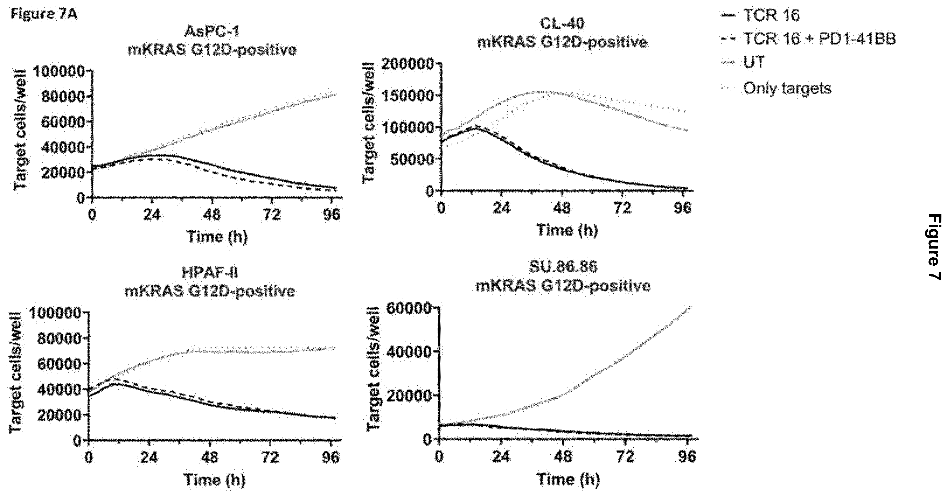

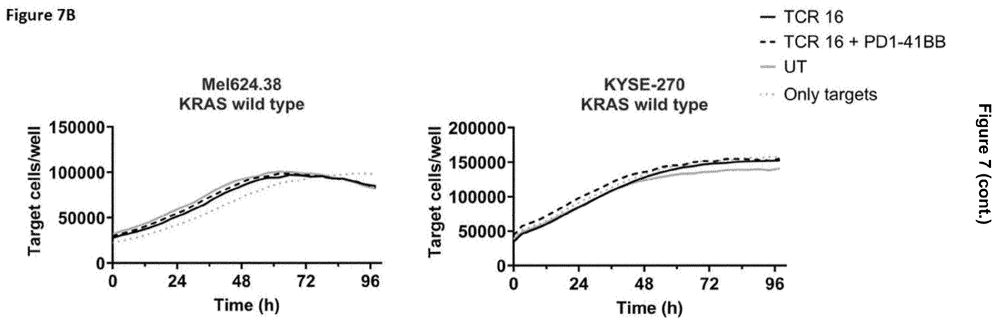

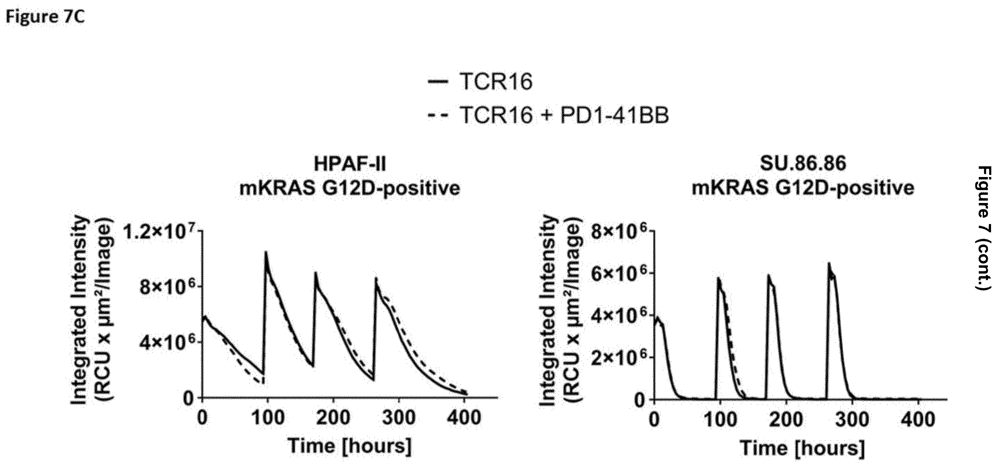

**Figure 7****. Strong and specific cytotoxic response against mKRAS G12D-positive tumor cells even after multiple tumor challenges.** Tumor cell lines derived from various indications expressing either mKRASG12D (AsPC-1, HPAF-II, CL-40, SU.86.86) or KRASwild type (Mel624.38, KYSE-270) were selected for this co-culture experiment. All tumor cells stably expressed a red fluorescent protein for cell tracking. The described tumor cell lines were co-cultured with either TCR16- or TCR 16 + PD1-41BB-transduced CD8⁺ T cells (TCR 16 and TCR 16 + PD1-41BB) or untransduced (UT) CD8⁺ T cells. Tumor cell lines were also kept in culture without any T cells as internal control (only targets). Cytotoxicity mediated by T cells was determined by reduction in the absolute number of red fluorescent tumor cells over time using a live-cell imaging system. Absolute cell numbers of red fluorescent tumor cells per well at every point of the measurement were calculated using the IncuCyte^{®} software. Shown are the target cell numbers (target cells/well) as mean of three replicates. Data of one representative experiment are shown (**Figure 7A** **and** **7B**). To assess T cell performance in a challenging environment, TCR16- and TCR16 + PD1-41BB-transduced CD8⁺ T cells (TCR 16 and TCR 16 + PD1-41 BB) were co-cultured with 3-dimensional (3D) tumor cell spheroids and multiple challenges with fresh tumor cell spheroids were performed by transferring the tumor cells to the co-culture plates at different time points. Hence, red fluorescent mKRAS G12D-positive tumor cells (HPAF-II and SU.86.86) were selected as target cells for this assay. Cytotoxicity mediated by T cells was determined by the reduction of red fluorescence signal over time using a live-cell imaging system. Shown is the red fluorescence integrated intensity (Red Calibrated Unit (RCU) x µm²/image) at every point of measurement as mean of three replicates which was calculated using the IncuCyte^{®} software. Data of one representative experiment are shown (**Figure 7C**).

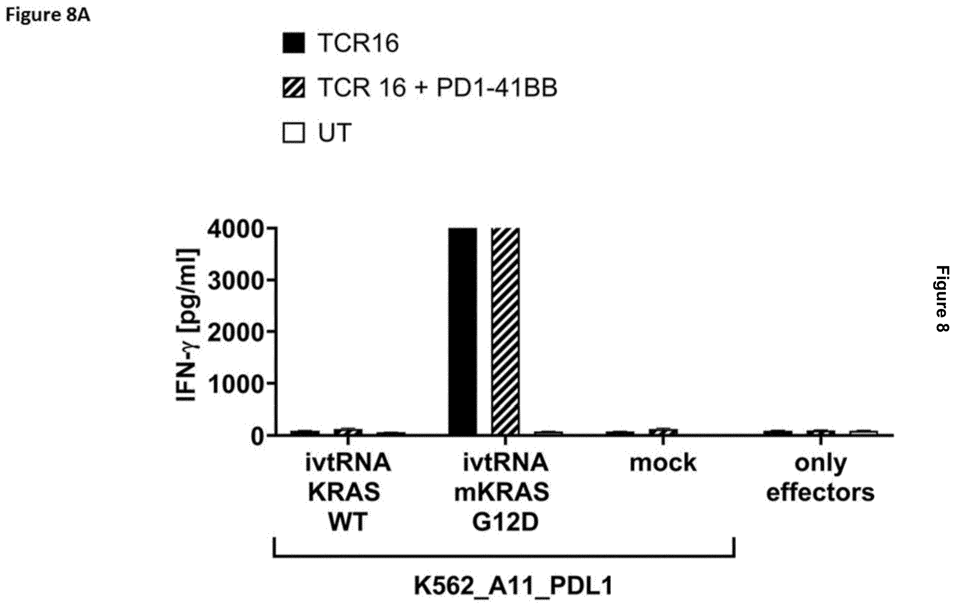

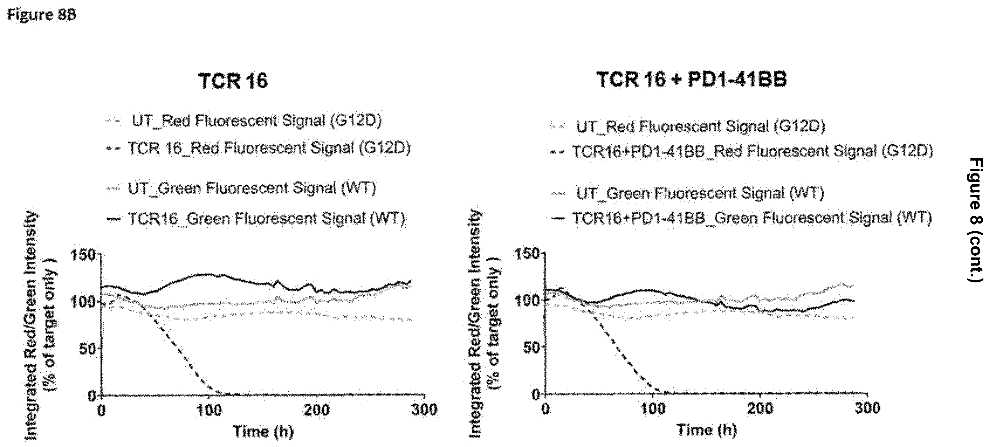

**Figure 8****. TCR-gated control of PD1-41BB CSP activation in TCR + PD1-41BB-transduced T cells.** TCR16- and TCR 16 + PD1-41BB-transduced CD8⁺ T cells (TCR 16 and TCR16+PD1-411B) were tested in a co-culture assay with K562_A11 cells transduced with PDL1 (K562_A11_PDL1) transfected with either ivtRNA encoding a fragment of mKRAS G12D gene (~402 bp) or ivtRNA encoding a fragment of KRAS G12 wild type gene (~402 bp). K562_A11_PDL1 cells electroporated with water (mock) as well as untransduced CD8⁺ T cells (UT) were included as negative controls. After 20 h of co-culture, an ELISA was performed to evaluate IFN-γ secretion by T cells. Shown is the mean value of triplicates with standard deviations (values are cut at 4000 pg/ml, upper limit of quantification). Data of one representative experiment is shown (**Figure 8A**). Two tumor cell lines (one mKRAS G12D-positive and one KRAS wild type) expressing high levels of transgenic PDL1 molecules were additionally transduced to express either a red fluorescent protein (HPAF-II cells, mKRAS G12D-positive) or a green fluorescent protein (BxPC-3, KRAS wild type) for tumor cell tracking by a live-cell imaging system. Red (G12D) and green (WT) fluorescent tumor cell spheroids were combined in the same wells and co-cultured with either TCR 16- or TCR16 + PD1-41 BB-transduced CD8⁺ T cells. Untransduced CD8⁺ T cells (UT) served as internal negative controls. The co-culture plates were monitored over time by live-cell imaging system. Cytotoxicity against mKRAS G12D-positive and KRAS wild type tumor cell spheroids was detected by the reduction over time of red fluorescence signal and green fluorescence signal, respectively. Shown are the red and green fluorescence integrated intensity (Red Calibrated Unit (RCU) x x µm²/image) normalized to only-target control values at every point of measurement as mean of three replicates which was calculated using the IncuCyte^{®} software. Data of one representative experiment are shown (**Figure 8B**).

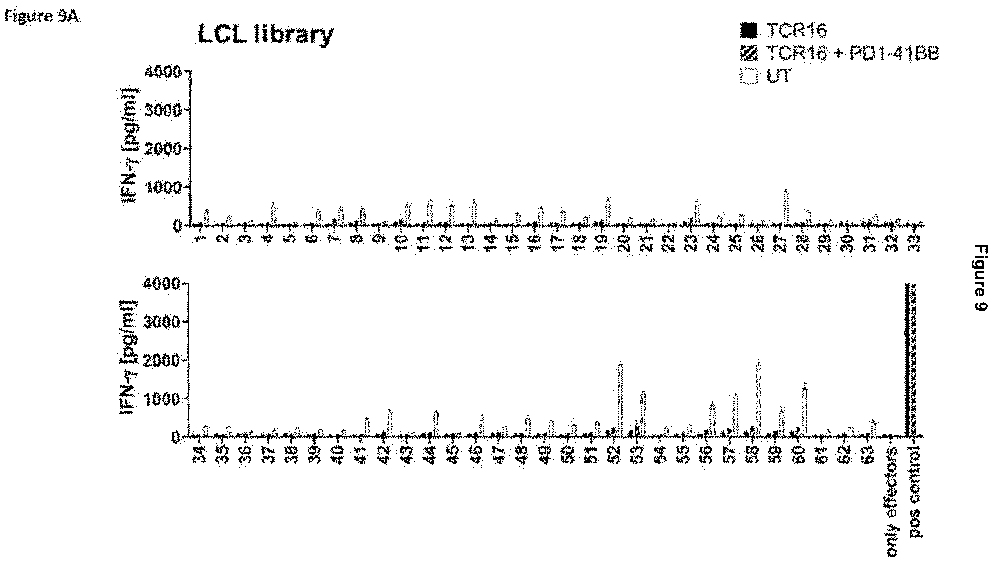

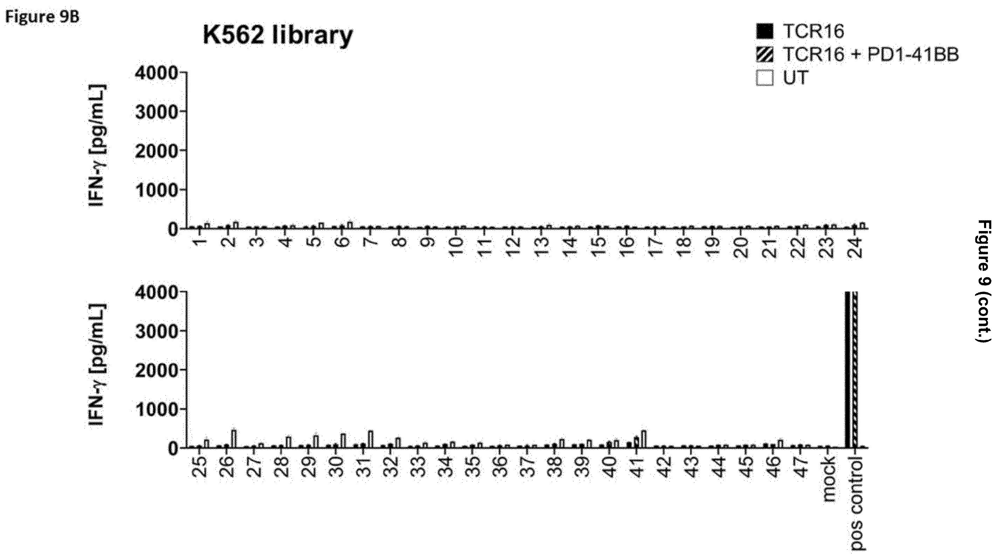

**Figure 9****. No signs of target peptide-independent cross-recognition of globally frequent HLA allotypes.** TCR 16-transduced T cells (TCR 16), TCR 16 + PD1-41BB-transduced CD8⁺ T cells (TCR 16 + PD1-41 BB) and untransduced CD8⁺ T cells (UT) were co-cultured with two cellular libraries. The first library comprised 63 lymphoblastoid cell lines (LCLs, LCL library, **Figure 9A**) and the second library comprised 47 K562 cell samples transduced with individual HLA molecules (K562 library, **Figure 9B**). K562_A11 cells loaded with mKRAS₇₋₁₆ G12D peptide (10⁻⁵ M) served as internal positive controls (pos control). Untransduced K562 cells were included as negative control. IFN-γ secretion was evaluated by ELISA 20 h after setting up the co-culture. Shown is the mean value of duplicates with standard deviations (values are cut at 4000 pg/ml, upper limit of quantification). Data of one representative experiment are shown.

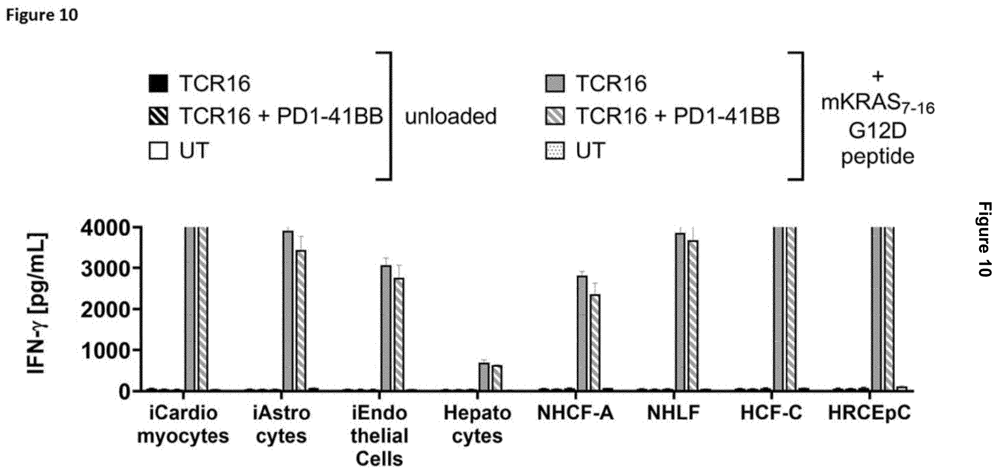

**Figure 10****. No signs of off-target toxicity were identified using a panel of healthy cell types.** HLA-A*11:01-positive primary healthy cells and induced pluripotent stem cell (iPS)-derived cell lines representing essential organs, namely induced pluripotent stem cell-derived iCell Cardiomyocytes (iCardiomyocytes), induced pluripotent stem cell-derived astrocytes (iAstrocytes), induced pluripotent stem cell-derived endothelial cells (iEndothelial Cells), primary normal human hepatocytes (Hepatocytes), Human Cardiac Fibroblasts-Atrial (NHCF-A), Normal Human Lung Fibroblasts (NHLF), Human Cardiac Fibroblasts (HCF-cC, Human Renal Cortical Epithelial Cells (HRCEpC), were tested for recognition by TCR16- and TCR 16 + PD1-41BB-transduced CD8⁺ T cells (TCR 16 and TCR 16 + PD1-41BB). Untransduced CD8⁺ T cells (UT) served as internal controls. Target cells loaded with mKRAS₇₋₁₆G12D peptide (10⁻⁵ M) were included as internal positive controls. IFN-γ secretion was evaluated by ELISA 20 h after setting up the co-culture. Shown is the mean value of triplicates with standard deviations (values are cut at 4000 pg/ml, upper limit of quantification). Data of one representative experiment are shown.

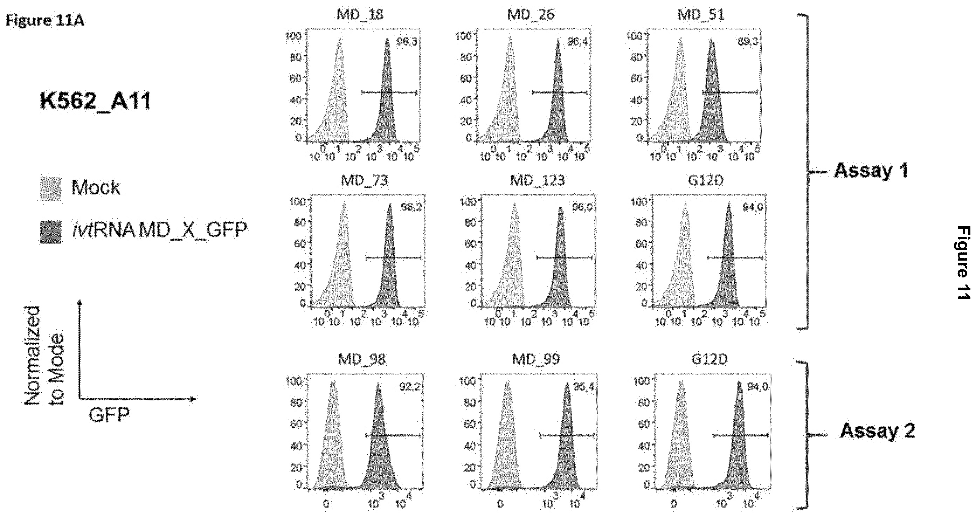

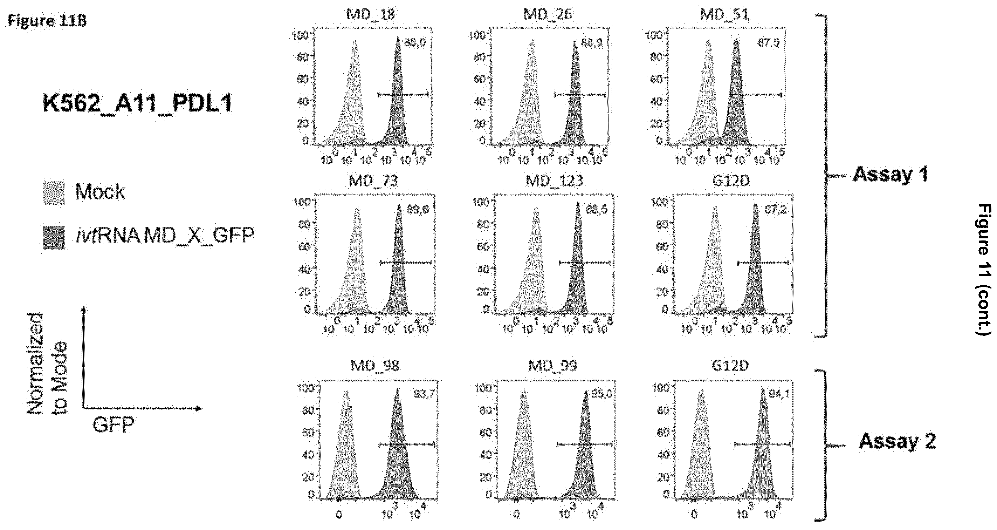

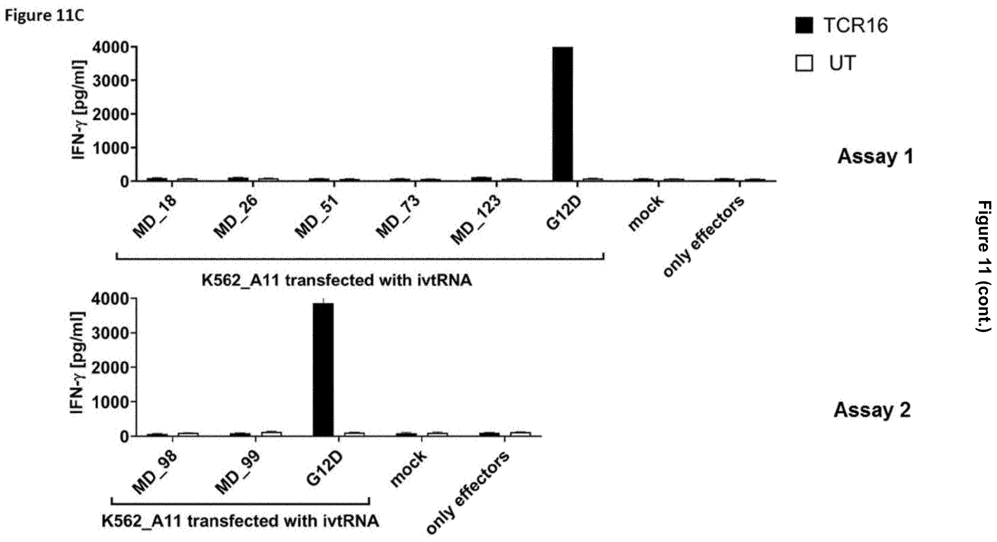

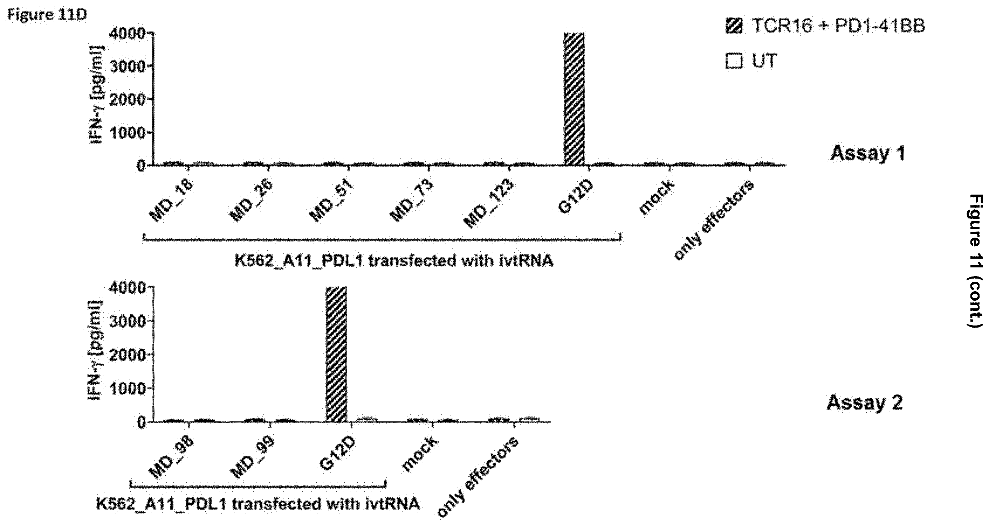

**Figure 11****. No signs of off-target toxicity against a library of self-peptides with a high sequence similarity to the target peptide independently of PD1-41BB CSP co-expression.** Mismatched peptides, which were identified as recognized by TCR16 and TCR16+PD1-41BB in a screening assay performed with peptide loading of target cells at high concentrations (10⁻⁵ M and 10⁻⁶ M), were tested for their potential to induce IFN-γ release when translated from *ivt*RNA and endogenously processed and presented by target cells. Midi-gene fragments were designed spanning the respective peptide-coding region and linked to GFP reporter gene. Co-culture experiments were established by using TCR 16- and TCR16 + PD1-41BB-transduced CD8⁺ T cells (TCR 16 and TCR 16 + PD1-41 BB) with mismatched peptide midi-gene (MD) fragment *ivt*RNA-transfected target cells (constructs were tested in two separate assays, assay 1: MD_18, MD_26, MD_51, MD_73, MD_123 and assay 2: MD_98, MD_99). K562_A11 were used as target cells in co-culture with TCR16-transduced T cells, while K562_A11_PDL1 were used as target cells in co-culture with TCR16+PD1-41BB-transduced T cells. Target cells transfected with mKRAS G12D midi-gene fragment *ivt*RNA (fragment spanning mKRAS₇₋₁₆G12D peptide-coding region and linked to GFP) were included as positive control. Water electroporation of target cells served as negative control (mock). The transfection efficiency of target cells was evaluated by flow cytometry analyzing GFP signals (**Figure 11A** **and** **11B**). IFN-γ secretion was evaluated by ELISA 20 h after setting up the co-culture. Shown is the mean value of triplicates with standard deviations (values are cut at 4000 pg/ml, upper limit of quantification). Data of one representative experiment are shown (**Figure 11C** **and** **D**).

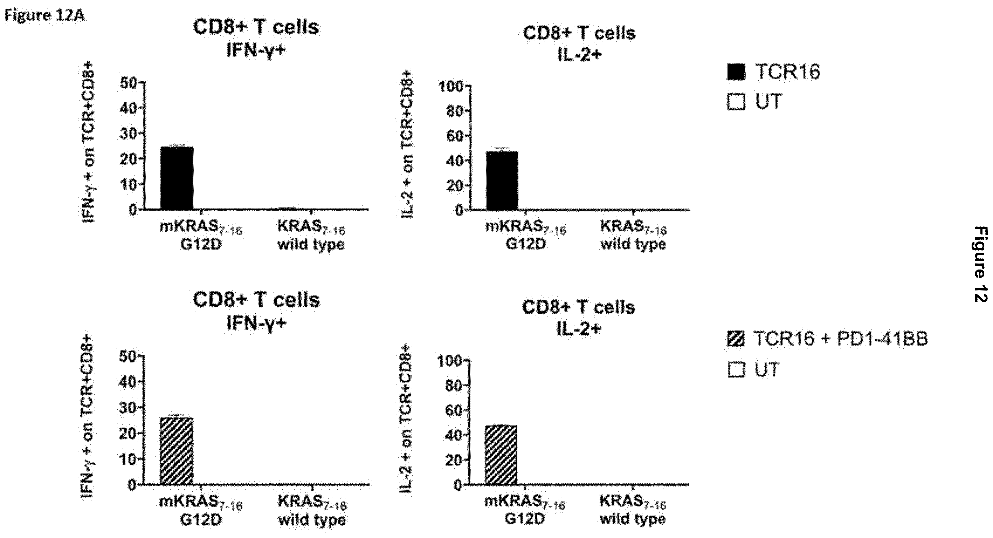

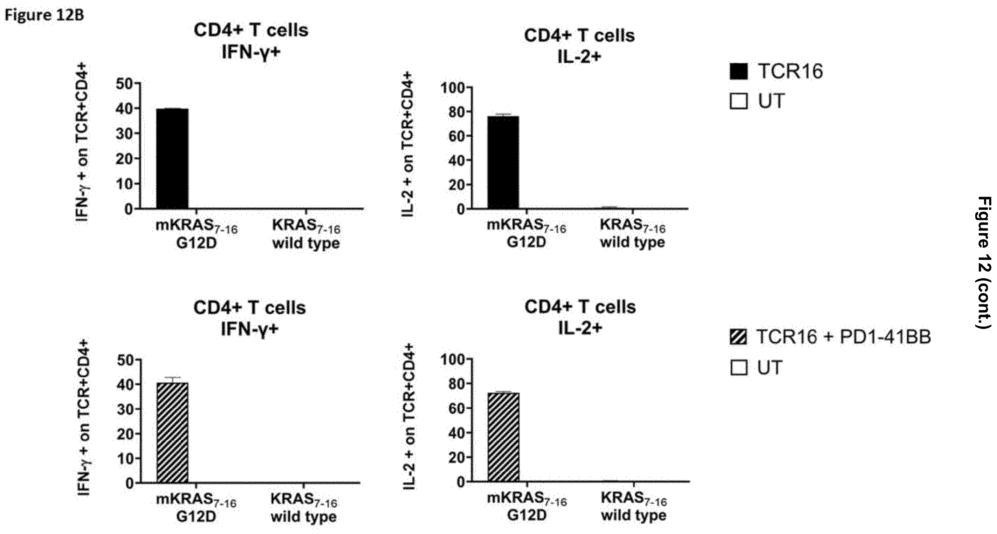

**Figure 12****. Signs of TCR functionality in CD4⁺ T cells.** TCR 16 either alone (TCR 16) or in combination with PD1-41BB CSP (TCR 16 + PD1-41BB) was transduced in CD3⁺ T cells derived from healthy donors (mixed population containing both CD4⁺ and CD8⁺ T cells) and utilized as effector samples in a co-culture experiment. Corresponding untransduced CD3⁺ T cells (UT) served as internal controls. Co-culture experiments were established using target cells loaded with either KRAS₇₋₁₆ wild type or mKRAS₇₋₁₆ G12D peptide at high concentrations (10⁻⁵ M). After overnight incubation in the presence of Brefeldin A, co-culture samples were assessed for CD3, CD8, CD4, Cb1 surface expression as well as for IFN-γ and IL-2 intracellular accumulation by flow cytometry. Populations were analyzed to detect IFN-γ-positive and IL-2-positive cells contained in CD8⁺ Cb1⁺ (**Figure 12A**) and in CD4⁺ Cb1⁺ T cells (**Figure 12B**). Data of one representative experiment are shown.

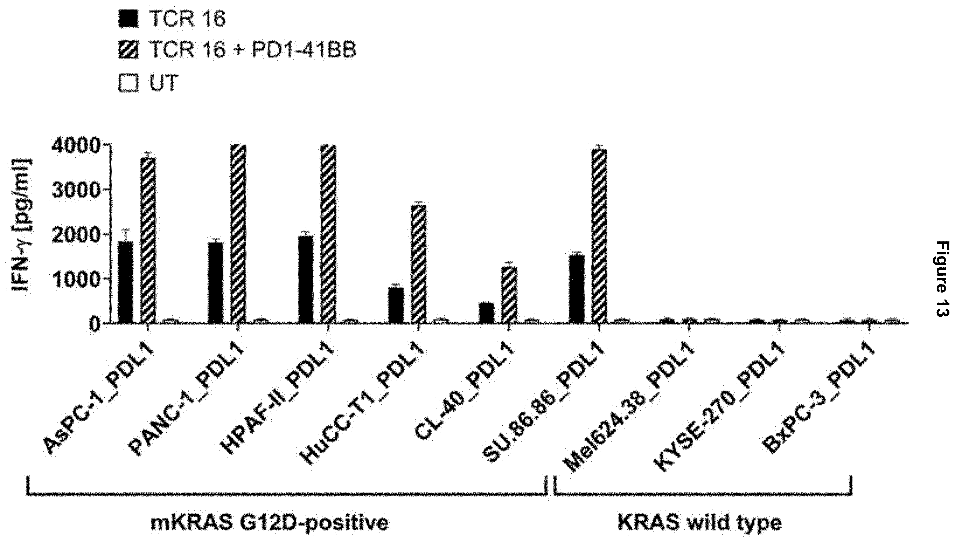

**Figure 13****. Co-expression of PD1-41BB CSP enhanced the specific release of IFN-γ in response to mKRAS G12D-positive tumor cells overexpressing PDL1.** CD8⁺ T cells expressing only TCR 16 (TCR 16) or TCR 16 in combination with PD1-41BB CSP (TCR 16 + PD1-41BB) were co-cultured with a panel of tumor cells expressing either mKRAS G12D (AsPC-1, PANC-1, HPAF-II, HuCC-T1, CL-40, SU.86.86) or KRAS wild type (Mel624.38, KYSE-270, BxPC-3). To obtain robust and stable PDL1 molecule expression, all selected tumor cell lines were transduced with PDL1. Untransduced CD8⁺ T cells (UT) served as negative control. IFN-γ secretion was assessed 20 h after setting up the co-culture. Shown is the mean value of triplicates with standard deviations (values are cut at 4000 pg/ml, upper limit of quantification). Data of one representative experiment are shown.

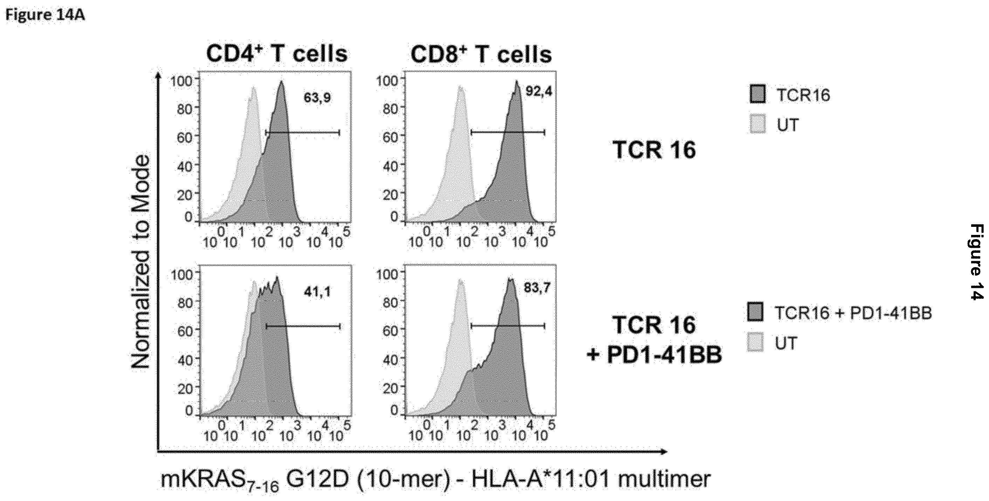

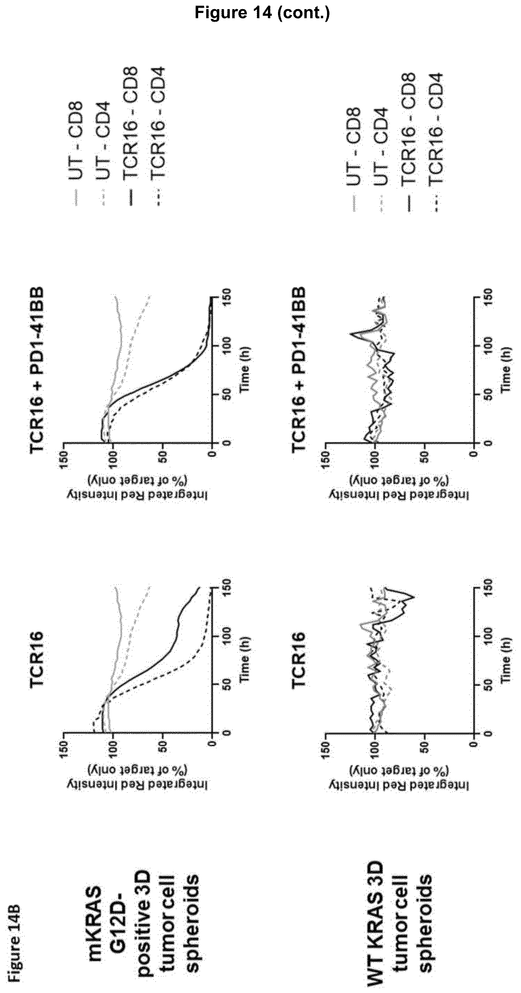

Figure 14. **Cytotoxic response against tumor cells is mediated by TCR-transduced CD4⁺ T cells independently of PD1-41BB CSP co-expression.** TCR 16 alone or in combination with PD1-41BB CSP was expressed in both CD8⁺ T cells and CD4⁺ T cells. CD8⁺ and CD4⁺ TCR-expressing T cells were then enriched by fluorescence-activated cell sorting (FACS). After enrichment, T cell samples were stained with mKRAS₇₋₁₆G12D (10-mer) HLA-A*11:01 multimer and analyzed by flow cytometry. Populations shown are pre-gated on live single cells. One representative experiment is shown (**Figure 14A**). Enriched T cells samples were co-cultured with either mKRAS G12D-positive (HPAF-II) or KRAS wild type (BxPC-3) 3D tumor cell spheroids which were labelled with a red fluorescent protein to allow tumor cell tracking over time. Cytotoxicity mediated by T cells was determined by the reduction of red fluorescence signal over time using a live-cell imaging system. Shown is the red fluorescence integrated intensity (Red Calibrated Unit (RCU) × µm²/image) at every point of measurement as mean of three replicates which was calculated using the IncuCyte^{®} software. One representative experiment is shown (**Figure 14B**).

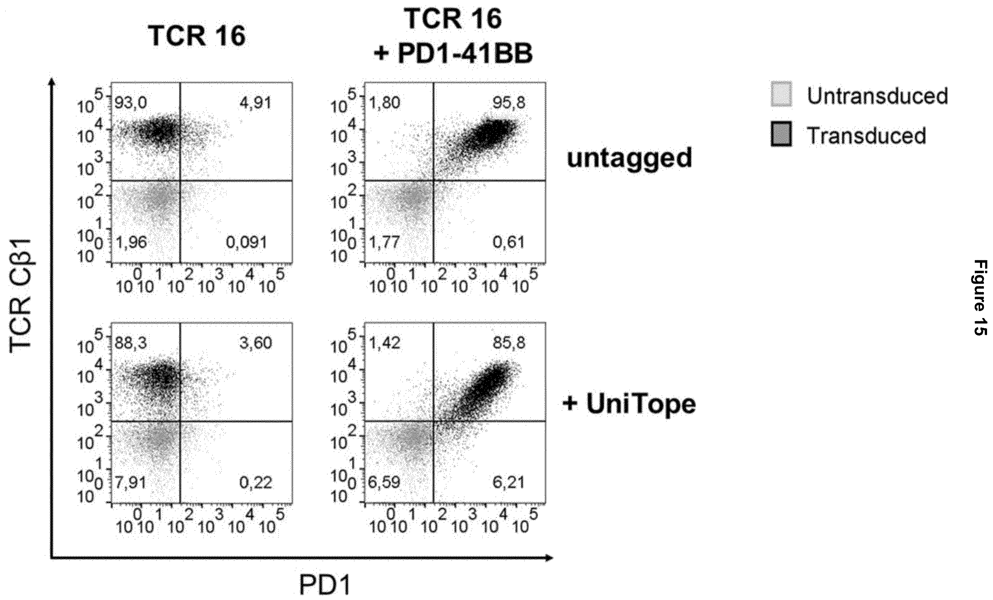

**Figure 15****. Transgenic TCR and PD1-41BB CSP expression is not affected by UniTope presence.** Jurkat biosensor cells were transduced with retroviral particles containing the transgene encoding either untagged TCR 16 (± PD1-41BB CSP) or UniTope tagged TCR 16 (± PD1-41BB CSP). Untransduced Jurkat biosensor cells were prepared in the same manner and served as controls. Transduction efficiency was assessed by staining transduced Jurkat biosensor cells with anti-PD1 and anti-Cβ1 antibodies followed by flow cytometry analysis. Populations shown are pre-gated on live, single Jurkat biosensor cells. Data of one representative experiment are shown.

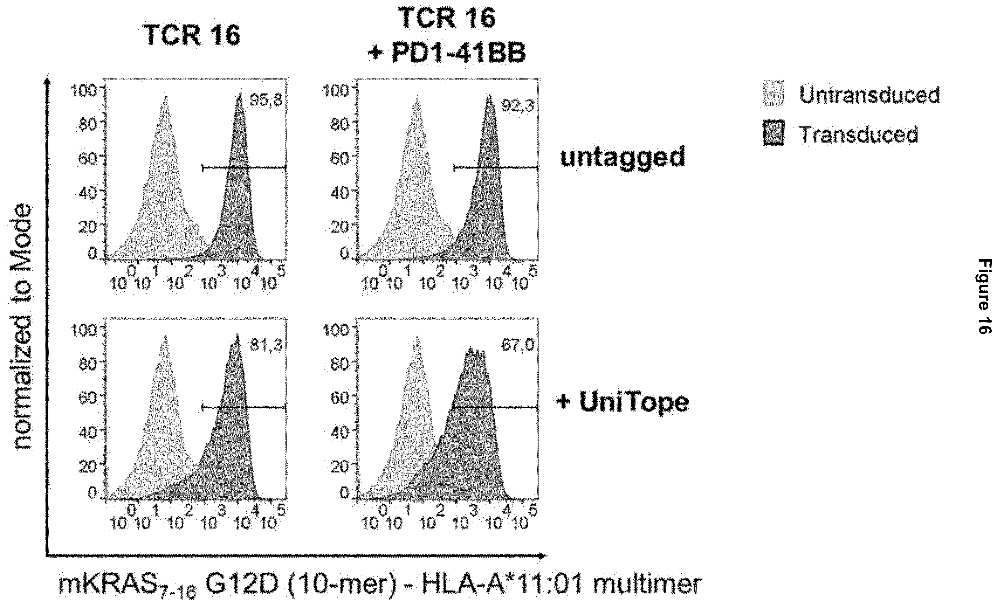

**Figure 16****. mKRAS7-16 G12D (10-mer) multimer binding is not affected by uniTope presence.** After transduction and expansion, untagged TCR 16-transduced (± PD1-41BB CSP) and UniTope tagged TCR 16-transduced (± PD1-41BB CSP) Jurkat biosensor cells were stained with mKRAS₇₋₁₆G12D (10-mer) HLA-A*11:01 multimer and analyzed by flow cytometry. Untransduced Jurkat biosensor cells were stained and analyzed in parallel as internal controls. Populations shown are pre-gated on live, single cells. Data of one representative experiment are shown.

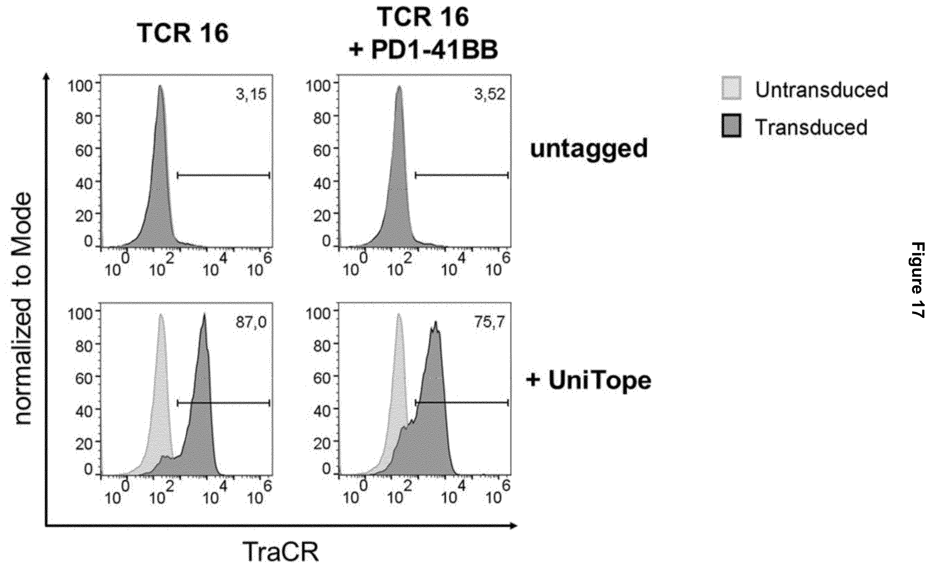

**Figure 17****. Specific and reliable detection of UniTope tagged TCR-transduced Jurkat biosensor cells.** After transduction and expansion, untagged TCR 16-transduced (± PD1-41BB CSP) and UniTope tagged TCR 16-transduced (± PD1-41BB CSP) Jurkat biosensor cells were stained with the UniTope specific TraCR antibody and analyzed by flow cytometry. Untransduced Jurkat biosensor cells were stained and analyzed in parallel as internal controls. Populations shown are pre-gated on live single cells. Data of one representative experiment are shown.

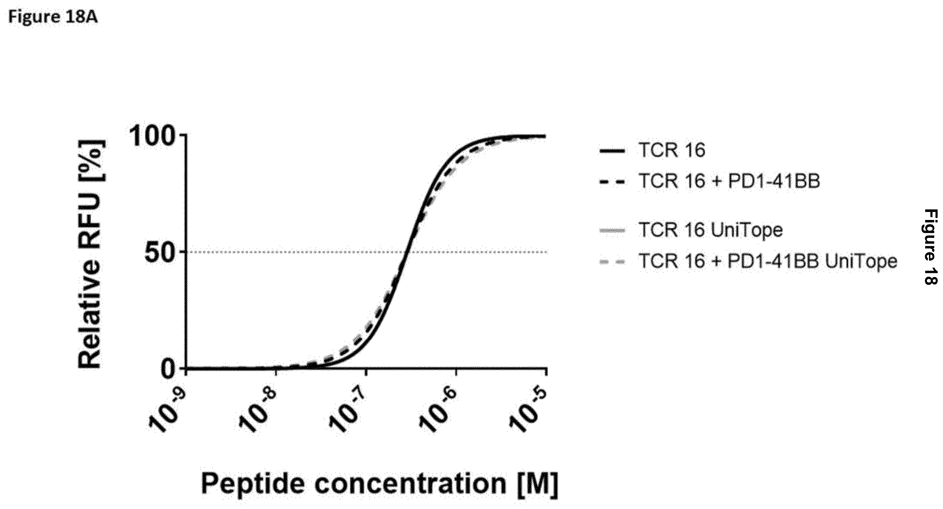

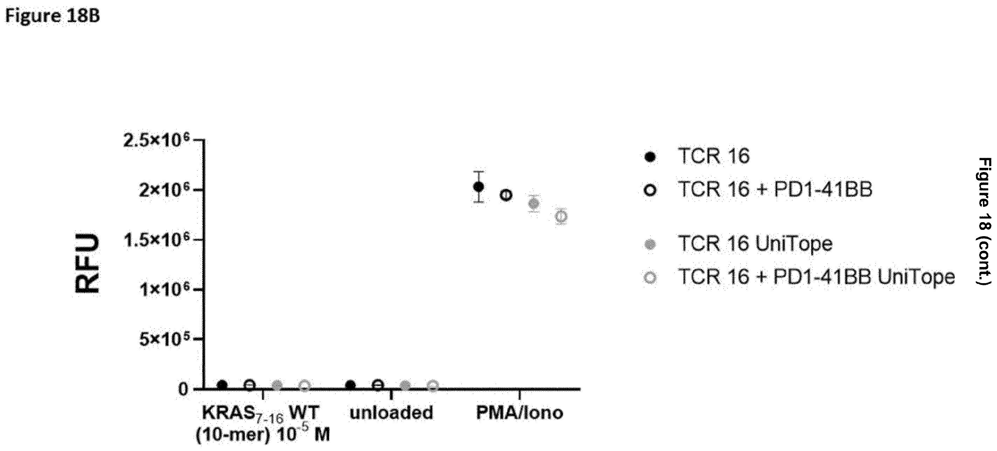

**Figure 18****. Specificity and sensitivity for mKRAS₇₋₁₆ G12D (10-mer) peptide are not affected by uniTope presence.** Untagged and UniTope tagged TCR 16-transduced Jurkat biosensor cells and TCR 16 + PD1-41BB-transduced Jurkat biosensor cells were tested in a co-culture experiment using K562_A11 cells loaded with titrated amounts of mKRAS₇₋₁₆G12D (10-mer) peptide (10⁻⁵ M to 10⁻⁹ M) to assess peptide sensitivity after UniTope inclusion. After 20 h of co-culture, eGFP expression was analyzed and maximal eGFP expression per transduced cell sample was set to 100%. Based on this, the relative eGFP expression was calculated. A nonlinear regression analysis was used for untagged and UniTope tagged TCR 16 (± PD1-41BB expression) transduced Jurkat biosensor cells in response to mKRAS₇₋₁₆ G12D (10-mer) peptide. Data of one representative experiment are shown (**Figure 18A**). Unloaded target cells and K562_A11 cells loaded with 10⁻⁵ M KRAS₇₋₁₆ wild type (10-mer) served as negative control, while transduced Jurkat biosensor cells stimulated with PMA/lono were used as positive control. Data of one representative experiment are shown (**Figure 18B**).

The TCR of the invention can also be referred to in the Figures as "TCR16" or "TCR16 + PD1-41BB" when used in combination with PD1-41BB.

### DETAILED DESCRIPTION OF THE INVENTION

### TCR and CDRs:

The term "TCR" as used herein includes native TCRs as well as TCR variants, fragments and constructs as defined herein. The term thus includes heterodimers comprising TCR alpha and beta chains as well as multimers and single chain constructs; optionally comprising further domains and/or moieties.

In its native form, the TCR exists as a complex of several proteins on the surface of T cells. The T cell receptor is composed of two (separate) protein chains, which are produced from the independent T cell receptor alpha and beta (TCR α and TCR β) genes and are called alpha (α-) and beta (β-) chains. Each chain of the TCR possesses one N-terminal immunoglobulin-like (Ig)-variable (V) domain/region, one Ig-constant-like (C) domain/region, a transmembrane/cell membrane-spanning region anchoring the chain in the plasma membrane, and a short cytoplasmic tail at the C-terminal end.

Antigen specificity is conferred by the variable regions of the alpha and beta chain. Both variable regions of the TCR alpha chain and beta chain comprise three hypervariable or complementarity determining regions (CDR1alpha/beta, CDR2alpha/beta and CDR3 alpha/beta) surrounded by framework (FR) regions. CDR3 is the prime determinant of antigen recognition and specificity (i.e. the ability to recognize and interact with a specific antigen), whereas CDR1 and CDR2 mainly interact with the MHC molecule presenting the antigenic peptide.

The TCR of the present invention is characterized by comprising i) an α chain CDR3 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 16; and/or ii) a β chain CDR3 comprising an amino acid sequence having at least 80 %, such as at least 81, 82, 83, 84, 85, 86, 87, 88, 89, 90, 91, 92, 93, 94, 95, 96, 97, 98, 99% or even 100% identity to SEQ ID NO: 4. It is also comprised herein a TCR comprising an α chain CDR3 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 16. It is also comprised herein a TCR comprising an α chain CDR3 comprising an amino acid sequence having at least 80 %, such as at least 81, 82, 83, 84, 85, 86, 87, 88, 89, 90, 91, 92, 93, 94, 95, 96, 97, 98, 99% or even 100% identity to SEQ ID NO: 4. The present invention also comprise the TCR as defined herein being characterized as comprising i) an α chain CDR3 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 16 and ii) a β chain CDR3 comprising an amino acid sequence having at least 80 %, such as at least 81, 82, 83, 84, 85, 86, 87, 88, 89, 90, 91, 92, 93, 94, 95, 96, 97, 98, 99% or even 100% identity to SEQ ID NO: 4.

It is envisaged that the CDR3 of the present invention can in principle be combined with any of the CDR1 depicted in SEQ ID NO: 14 and/or 2 and/or with any of the CDR2 depicted in SEQ ID NO: 15 and/or 3, preferably provided that the TCR retains its ability to recognize its epitope as defined elsewhere herein, to a similar, the same or even a higher extent as the TCR evaluated in the Examples.

The TCR of the invention may further be provided in "isolated" or "substantially pure" form. "isolated" or "substantially pure" when used herein means that the TCR have been identified separated and/or recovered from a component of its production environment, such that the "isolated" TCR is free or substantially free of other contaminant components from its production environment that might interfere with its therapeutic or diagnostic use. Contaminant components may include enzymes, hormones, and other proteinaceous or non-proteinaceous solutes. "isolated" TCR will thus be prepared by at least one purification step removing or substantially removing these contaminant components. The aforementioned definition is equally applicable to "isolated" polynucleotides/nucleic acids, *mutatis mutandis.*

The TCR of the present invention as defined herein may further be characterized as comprising at least one (e.g. 1, 2, 3, or 4) of the following: i) an α chain CDR 1 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 14; ii) an α chain CDR2 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 15; iii) a β chain CDR1 comprising an amino acid sequence having at least 80 %, such as at least 81, 82, 83, 84, 85, 86, 87, 88, 89, 90, 91, 92, 93, 94, 95, 96, 97, 98, 99% or even 100% identity to SEQ ID NO: 2; and/or iv) a β chain CDR2 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 3.

Thus, the present invention may also comprise the TCR as defined above being further characterized as comprising an α chain CDR 1 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 14. Thus, the present invention may also comprise the TCR as defined above being further characterized as comprising an α chain CDR 2 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 15. Thus, the present invention may also comprise the TCR as defined above being further characterized as comprising a β chain CDR1 comprising an amino acid sequence having at least 80 %, such as at least 81, 82, 83, 84, 85, 86, 87, 88, 89, 90, 91, 92, 93, 94, 95, 96, 97, 98, 99% or even 100% identity to SEQ ID NO: 2. Thus, the present invention may also comprise the TCR as defined above being further characterized as comprising a β chain CDR2 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 3.

The present invention may also comprise the TCR as defined herein further being characterized as comprising i) an α chain CDR 1 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 14; ii) an α chain CDR2 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 15; and iii) an α chain CDR3 comprising an amino acid sequence having at least 80 %, such as at least 81, 82, 83, 84, 85, 86, 87, 88, 89, 90, 91, 92, 93, 94, 95, 96, 97, 98, 99% or even 100% identity to SEQ ID NO: 16. The present invention may also comprise the TCR as defined herein further being characterized as comprising i) a β chain CDR1 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 2; ii) a β chain CDR2 comprising an amino acid sequence having at least 80 %, such as at least 81, 82, 83, 84, 85, 86, 87, 88, 89, 90, 91, 92, 93, 94, 95, 96, 97, 98, 99% or even 100% identity to SEQ ID NO: 3; and iii) a β chain CDR3 comprising an amino acid sequence having at least 80%, such as at least 81, 82, 83, 84, 85, 86, 87, 88, 89, 90, 91, 92, 93, 94, 95, 96, 97, 98, 99% or even 100% identity to SEQ ID NO: 4.

The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative or highly conservative amino acid substitutions as defined herein, preferably corresponding to position 1, 2, 3, 4, 5 and/or 6 of SEQ ID NO: 14; position(s) 1, 2, 3, 4, 5, 6, 7 and/or 8 of SEQ ID NO: 15; position(s) 1, 2, 3, 4, 5, 6, 7, 8, 9, 10, 11, 12 and/or 13 of SEQ ID NO: 16; position(s) 1, 2, 3, 4 and/or 5 of SEQ ID NO: 2; position(s) 1, 2, 3, 4, 5 and/or 6 of SEQ ID NO: 3; and/or position(s) 1, 2, 3, 4, 5, 6, 7, 8, 9, 10, 11, 12, 13 and/or 14 of SEQ ID NO: 4.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises conservative or highly conservative amino acid substitutions as defined herein corresponding to position 1, 2, 3, 4, 5 and/or 6 of SEQ ID NO: 14.

The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 1 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 2 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 3 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 4 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 5 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 6 of SEQ ID NO: 14.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 1 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 2 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 3 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 4 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 5 of SEQ ID NO: 14. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 6 of SEQ ID NO: 14.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative or conservative amino acid substitutions as defined herein corresponding to position 1, 2, 3, 4, 5, 6, 7, and/or 8 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 1 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 2 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 3 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 4 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 5 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 6 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 7 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 8 of SEQ ID NO: 15.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 1 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 2 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 3 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 4 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 5 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 6 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 7 of SEQ ID NO: 15. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 8 of SEQ ID NO: 15.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative or conservative amino acid substitutions as defined herein corresponding to position 1, 2, 3, 4, 5, 6, 7, 8, 9, 10, 11, 12, and/or 13 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 1 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 2 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 3 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 4 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 5 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 6 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 7 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 8 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 9 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 10 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 11 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 12 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 13 of SEQ ID NO: 16.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 1 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 2 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 3 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 4 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 5 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 6 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 7 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 8 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 9 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 10 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 11 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 12 of SEQ ID NO: 16. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 13 of SEQ ID NO: 16.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative or conservative amino acid substitutions as defined herein corresponding to position 1, 2, 3, 4, and/or 5 of SEQ ID NO: 2. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 1 of SEQ ID NO: 2. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 2 of SEQ ID NO: 2. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 3 of SEQ ID NO: 2. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 4 of SEQ ID NO: 2. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 5 of SEQ ID NO: 2.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 1 of SEQ ID NO: 2. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 2 of SEQ ID NO: 2. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 3 of SEQ ID NO: 2. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 4 of SEQ ID NO: 2. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 5 of SEQ ID NO: 2.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative or conservative amino acid substitutions as defined herein corresponding to position 1, 2, 3, 4, 5 and/or6 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 1 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 2 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 3 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 4 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 5 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 6 of SEQ ID NO: 3.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 1 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 2 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 3 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 4 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 5 of SEQ ID NO: 3. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 6 of SEQ ID NO: 3.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative or conservative amino acid substitutions as defined herein corresponding to position 1, 2, 3, 4, 5, 6, 7, 8, 9, 10, 11, 12, 13, and/or 14 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 1 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 2 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 3 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 4 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 5 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 6 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 7 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 8 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 9 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 10 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 11 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 12 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 13 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises conservative amino acid substitutions as defined herein corresponding to position 14 of SEQ ID NO: 4.

The present invention may also comprise the TCR as defined herein and further being characterized, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 1 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 2 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 3 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 4 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 5 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 6 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 7 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 8 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 9 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 10 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 11 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 12 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 13 of SEQ ID NO: 4. The present invention may also comprise the TCR as defined herein, wherein the at least 80%, such as at least 81, 82, 83, 84, 85, 86, 87, 88, 89, 90, 91, 92, 93, 94, 95, 96, 97, 98, 99% or even 100% identity comprises highly conservative amino acid substitutions as defined herein corresponding to position 14 of SEQ ID NO: 4.

In a preferred embodiment, the present invention comprises the TCR as defined herein, further comprising i) an α chain CDR 1 comprising the amino acid sequence of SEQ ID NO: 14; ii) an α chain CDR2 comprising the amino acid sequence of SEQ ID NO: 15; iii) an α chain CDR3 comprising the amino acid sequence of SEQ ID NO: 16; iv) a β chain CDR1 comprising the amino acid sequence of SEQ ID NO: 2; v) a β chain CDR2 comprising the amino acid sequence of SEQ ID NO: 3; and vi) a β chain CDR3 comprising the amino acid sequence of SEQ ID NO: 4 (see Table 2).

### Complete variable region:

The present invention may further provide a TCR comprising a TCR α chain variable region and/or a TCR β chain variable region. Said α and β chain variable region sequences are also shown in Table 2 herein. The present invention may further comprise the TCR as defined herein comprising i) an amino acid sequence having at least 80%, such as at least 81, 82, 83, 84, 85, 86, 87, 88, 89, 90, 91, 92, 93, 94, 95, 96, 97, 98, 99 or even 100% identity to SEQ ID NO: 20; and/or ii) an amino acid sequence having at least 80%, such as at least 81, 82, 83, 84, 85, 86, 87, 88, 89, 90, 91, 92, 93, 94, 95, 96, 97, 98, 99 or even 100% identity to SEQ ID NO: 8. Thus, the present invention may further comprise the TCR as defined herein comprising an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 20. The present invention may further comprise the TCR as defined herein comprising an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 8. Thus, the present invention may further comprise the TCR as defined herein comprising an amino acid sequence having at least 85 % identity to SEQ ID NO: 20. The present invention may further comprise the TCR as defined herein comprising an amino acid sequence having at least 85 % identity to SEQ ID NO: 8. Thus, the present invention may further comprise the TCR as defined herein comprising an amino acid sequence having at least 90 % identity to SEQ ID NO: 20. The present invention may further comprise the TCR as defined herein comprising an amino acid sequence having at least 90 % identity to SEQ ID NO: 8. Thus, the present invention may further comprise the TCR as defined herein comprising an amino acid sequence having at least 95 % identity to SEQ ID NO: 20. The present invention may further comprise the TCR as defined herein comprising an amino acid sequence having at least 95 % identity to SEQ ID NO: 8. Thus, the present invention may further comprise the TCR as defined herein comprising an amino acid sequence having at least 98 % identity to SEQ ID NO: 20. The present invention may further comprise the TCR as defined herein comprising an amino acid sequence having at least 98 % identity to SEQ ID NO: 8.

In a more preferred embodiment, the present invention comprises the TCR as defined herein, further comprising i) an α chain variable region comprising the amino acid sequence of SEQ ID NO: 20, and/or ii) a β chain variable region comprising the amino acid sequence of SEQ ID NO: 8.

### Constant region:

The TCR may further comprise a constant (C) region in its α and/or β chain. The constant region can be a human constant region or derived from another species, yielding a "chimeric" TCR. For instance, human alpha and/or beta chains can be replaced by their murine counterparts ("murinization") which has been found to enhance surface expression of human TCRs by supporting preferential pairing of the TCR α and β chains, and a more stable association with the CD3 co-receptor. Thus, the present invention may comprise the TCR as defined herein further comprising a (minimally) murinized constant region in its α and/or β chain. The present invention may further comprise the TCR as defined herein comprising i) an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 22; and/or ii) an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 10. Thus, the present invention may further comprise the TCR as defined herein comprising an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 22. The present invention may further comprise the TCR as defined herein comprising an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 10. Thus, the present invention may further comprise the TCR as defined herein comprising an amino acid sequence having at least 85 % identity to SEQ ID NO: 22. The present invention may further comprise the TCR as defined herein comprising an amino acid sequence having at least 85 % identity to SEQ ID NO: 10. Thus, the present invention may further comprise the TCR as defined herein comprising an amino acid sequence having at least 90 % identity to SEQ ID NO: 22. The present invention may further comprise the TCR as defined herein comprising an amino acid sequence having at least 90 % identity to SEQ ID NO: 10. Thus, the present invention may further comprise the TCR as defined herein comprising an amino acid sequence having at least 95 % identity to SEQ ID NO: 22. The present invention may further comprise the TCR as defined herein comprising an amino acid sequence having at least 95 % identity to SEQ ID NO: 10. Thus, the present invention may further comprise the TCR as defined herein comprising an amino acid sequence having at least 98 % identity to SEQ ID NO: 22. The present invention may further comprise the TCR as defined herein comprising an amino acid sequence having at least 98 % identity to SEQ ID NO: 10.

In a more preferred embodiment, the present invention comprises the TCR as defined herein, further comprising i) an α chain constant region comprising the amino acid sequence of SEQ ID NO: 22, and/or ii) a β chain constant region comprising the amino acid sequence of SEQ ID NO: 10 (see **Table 2** herein).

In a further preferred embodiment, the present invention comprises the TCR as defined herein, further comprising, in said constant region, at least one epitope tag, wherein the insert position of the epitope tag is located in the alpha constant region (TRAC) and/or the beta constant region (TRBC), preferably in the TRBC.

Suitable epitope tags are generally known in the art and aid in identification, tracking, purification and/or isolation of the respective molecule (tags). Such epitope tags are typically short stretches of amino acids that allow for binding of a specific antibody and therefore enable identification and tracking of the binding and movement of soluble TCRs or host cells within the patient's body or cultivated (host) cells. Detection of the epitope tag, and hence, the tagged TCR, can be achieved using a number of different techniques. Tags can further be employed for stimulation and expansion of host cells carrying the respective TCR by cultivating the cells in the presence of binding molecules (antibodies) specific for said tag.

In another preferred embodiment, the TCR according to the present invention comprises a suitable epitope tag (e.g., SEQ ID NOs: 41, 42, 43 or 44) within: (i) the α chain constant region comprising the amino acid sequence of SEQ ID NO: 22, and/or within ii) the β chain constant region comprising the amino acid sequence of SEQ ID NO: 10.

In another embodiment of the invention, the epitope tag as defined herein may be inserted by means of linker sequences, preferably short glycine-rich linker sequences, more preferably glycine-rich linker sequences consisting of 2 to 6, preferably 3 to 5 amino acids, more preferably comprising or consisting of the amino acid sequence GSG.

### Alpha and beta chains:

The present invention may further comprise the TCR as defined herein comprising i) an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 24; and/or ii) an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 12. Thus, the present invention may further comprise the TCR as defined herein comprising an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 24. The present invention may further comprise the TCR as defined herein comprising an amino acid sequence having at least 80 %, such as at least 81, 82, 83, 84, 85, 86, 87, 88, 89, 90, 91, 92, 93, 94, 95, 96, 97, 98, 99 or even 100% identity to SEQ ID NO: 12. Thus, the present invention may further comprise the TCR as defined herein comprising an amino acid sequence having at least 85 % identity to SEQ ID NO: 24. The present invention may further comprise the TCR as defined herein comprising an amino acid sequence having at least 85 % identity to SEQ ID NO: 12. Thus, the present invention may further comprise the TCR as defined herein comprising an amino acid sequence having at least 90 % identity to SEQ ID NO: 24. The present invention may further comprise the TCR as defined herein comprising an amino acid sequence having at least 90 % identity to SEQ ID NO: 12. Thus, the present invention may further comprise the TCR as defined herein comprising an amino acid sequence having at least 95 % identity to SEQ ID NO: 24. The present invention may further comprise the TCR as defined herein comprising an amino acid sequence having at least 95 % identity to SEQ ID NO: 12. Thus, the present invention may further comprise the TCR as defined herein comprising an amino acid sequence having at least 98 % identity to SEQ ID NO: 24. The present invention may further comprise the TCR as defined herein comprising an amino acid sequence having at least 98 % identity to SEQ ID NO: 12.

In a most preferred embodiment, the present invention comprises the TCR as defined herein, further comprising i) an α chain comprising the amino acid sequence of SEQ ID NO: 24; and/or ii) a β chain comprising the amino acid sequence of SEQ ID NO: 12 (see **Table 2**).

As used herein the term "sequence identity" indicates the extent to which two (nucleotide or amino acid) sequences have identical residues at the same positions in an alignment, and is often expressed as a percentage. Preferably, identity is determined over the entire length of the sequences being compared. Thus, two copies of exactly the same sequence have 100% identity, but sequences that are less highly conserved and have deletions, additions, or replacements, may have a lower degree of identity. Those skilled in the art will recognize that several algorithms are available for determining sequence identity using standard parameters, for example Blast (Altschul, et al. (1997) Nucleic Acids Res. 25:3389-3402), Blast2 (Altschul, et al. (1990) J. Mol. Biol. 215:403-410), Smith-Waterman (Smith, et al. (1981) J. Mol. Biol. 147:195-197) and ClustalW.

### Epitope:

KRAS, also referred to as GTPase KRas, V-Ki-Ras2 Kirsten rat sarcoma viral oncogene, or KRAS2, is a member of the small GTPase superfamily. There are two transcript variants of KRAS: KRAS variant A and KRAS variant B. Hereinafter, references to "KRAS" (mutated or unmutated) refer to both variant A and variant B, unless specified otherwise. Without being bound to a particular theory or mechanism, it is believed that, when mutated, KRAS may be involved in signal transduction early in the oncogenesis of many human cancers. A single amino acid substitution may activate the mutation. When activated, mutated KRAS binds to guanosine-5 '-triphosphate (GTP) and converts GTP to guanosine 5 '-diphosphate (GDP). The mutated KRAS protein product may be constitutively activated. Mutated KRAS protein may be expressed in any of a variety of human cancers such as, for example, pancreatic (e.g., pancreatic carcinoma), colorectal, lung (e.g., lung adenocarcinoma), endometrial, ovarian (e.g., epithelial ovarian cancer), and prostate cancers.

The TCR of the present invention may further have antigenic specificity to an epitope presented in the context of a MHC class I molecule, the epitope comprising or consisting of the amino acid sequence of SEQ ID NO: 1 (VVVGA**D**GVGK). Such epitope is mutated and refers to a mutated KRAS peptide, which generally corresponds to positions 7-16 of the unmutated, wild type (WT) KRAS protein amino acid sequence of SEQ ID NO: 37 with the exception that for SEQ ID NO: 1, the glycine at position 12 of SEQ ID NO: 37 (which refers to position 6 in SEQ ID NO: 1) is substituted with Asp (D) - aspartic acid, respectively. The mutated KRAS amino acid sequence SEQ ID NO: 1 is also referred to herein as "mKRAS ₇₋₁₅ G12**D** - 10mer." The TCR of the present invention may not have antigenic specificity to an epitope presented in the context of a MHC class I molecule, the epitope comprising or consisting of the amino acid sequence of SEQ ID NO: 34. In preferred embodiments the TCR of the present invention does not recognize KRAS (WT), KRAS G12C, KRAS G12V, or KRAS G12R.

In a further embodiment of the invention, the TCR may further have antigenic specificity to an epitope presented in the context of a MHC class I molecule, the epitope comprising or consisting of a fragment of the amino acid sequence of SEQ ID NO: 1, or a variant of the amino acid sequence of SEQ ID NO: 1 comprising one or more (e.g. one or two) conservative amino acid substitutions (preferably corresponding to position 1, 2, 3, 4, 5, 6, 7, 8, 9 and/or 10 of SEQ ID NO: 1).

A "fragment thereof" as used herein, refers to a part of the mutated KRAS peptide (according to SEQ ID NO: 1) having one or more amino acids absent from the amino and/or carboxyl terminus of the mutated KRAS peptide comprising or consisting of the amino acid sequence of SEQ ID NO: 1. The fragment as defined herein may also comprise the mutated epitope comprising the amino acid sequence of VVGADGVGK (SEQ ID NO: 35). Such mutated epitope refers to a mutated KRAS peptide which generally corresponds to positions 8-16 of the unmutated, wild type (WT) KRAS protein amino acid sequence of SEQ ID NO: 37 with the exception that for SEQ ID NO: 35, the glycine at position 12 of SEQ ID NO: 37 (which refers to position 5 in SEQ ID NO: 35) is substituted with Asp (D) - aspartic acid, respectively. The mutated KRAS amino acid sequence SEQ ID NO: 35 is also referred to herein as "mKRAS G12D - 9mer." The TCR of the present invention may not have antigenic specificity to an epitope presented in the context of a MHC class I molecule, the epitope comprising or consisting of the amino acid sequence of SEQ ID NO: 36. In preferred embodiments the TCR of the present invention does not recognize KRAS (WT), KRAS G12C, KRAS G12V, or KRAS G12R.

The term "variant" may refer to a polypeptide having specific activity as described herein comprising an alteration, i.e., a substitution, insertion, and/or deletion, at one or more (e.g., several, e.g., 2, 3, 4, 5, 6, 7, 8, 9 or 10, etc.) positions. A "variant thereof" as used herein, may particularly comprise the mutated KRAS peptide as mentioned above with one or more positions being substituted. A substitution means replacement of the amino acid occupying a position with a different amino acid; a deletion means removal of the amino acid occupying a position; and an insertion means adding an amino acid adjacent to and immediately following the amino acid occupying a position.

As used herein, "silent" mutations mean base substitutions within a nucleic acid sequence which do not change the amino acid sequence encoded by the nucleic acid sequence. "Conservative or equivalent" substitutions (or mutations) mean substitutions as listed as "Exemplary Substitutions" in Table 1 below. "Highly conservative" substitutions as used herein mean substitutions as shown under the heading "Preferred Substitutions" in **Table 1** below.

**Table 1: Amino acid substitutions.**

| Original | Exemplary Substitutions | Preferred Substitutions |
|---|---|---|
| Ala (A) | val, leu; ile | val |
| Arg (R) | lys; gln; asn | lys |
| Asn (N) | gln; his; asp, lys; arg | gln |
| Asp (D) | glu; asn | glu |
| Cys (C) | ser; ala | ser |
| Gln (Q) | asn; glu | asn |
| Glu (E) | asp; gln | asp |
| Gly (G) | ala | ala |
| His (H) | asn; gln; lys; arg | arg |
| Ile (I) | leu; val; met; ala; phe; | leu |
| Leu (L) | norleucine; ile; val; met; ala; | ile |
| Lys (K) | arg; gln; asn | arg |
| Met (M) | leu; phe; ile | leu |
| Phe (F) | leu; val; ile; ala; tyr | tyr |
| Pro (P) | ala | ala |
| Ser (S) | thr | thr |
| Thr (T) | ser | set |
| Trp (W) | tyr; phe | tyr |
| Tyr (Y) | trp; phe; thr; set | phe |
| Val (V) | ile; leu; met; phe; ala; | leu |

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. For ease of reference, polypeptide variants (or mutants) of the invention may be referred to by the use of the following nomenclature: original amino acid(s): position(s): substituted amino acid(s). According to this nomenclature, for instance the substitution of G (glycine) for V (valine) in position 12 can be shown as "G12V".

The term "position" when used in accordance with the disclosure means the position of either an amino acid within an amino acid sequence depicted herein or the position of a nucleotide within a nucleic acid sequence depicted herein. The term "corresponding" as used herein also includes that a position is not only determined by the number of the preceding nucleotides/amino acids, but is rather to be viewed in the context of the circumjacent portion of the sequence. Accordingly, the position of a given amino acid or nucleotide in accordance with the disclosure may vary due to deletion or addition of amino acids or nucleotides elsewhere in the sequence. Thus, when a position is referred to as a "corresponding position" in accordance with the disclosure it is understood that nucleotides/amino acids may differ in terms of the specified numeral but may still have similar neighboring nucleotides/amino acids. In order to determine whether an amino acid residue (or nucleotide) in a given sequence corresponds to a certain position in the amino acid sequence of a "parent" amino acid/nucleotide sequence, the skilled person can use means and methods well-known in the art, e.g., sequence alignments, either manually or by using computer programs such as exemplified herein.

In a further embodiment of the invention, the TCR may have further antigenic specificity to an epitope presented in the context of a MHC class I molecule, the epitope comprising or consisting of the amino acid sequence of SEQ ID NO: 35.

In a further embodiment of the invention, the TCR may have further antigenic specificity to an epitope presented in the context of a MHC class I molecule, the epitope comprising or consisting of the amino acid sequence of SEQ ID NO: 1 comprising one conservative amino acid substitution, preferably corresponding to position 1, 2, 3, 4, 5, 6, 7, 8, 9 and/or 10 of SEQ ID NO: 1. In a further embodiment of the invention, the TCR may have further antigenic specificity to an epitope presented in the context of a MHC class I molecule, the epitope comprising or consisting of the amino acid sequence of SEQ ID NO: 1 comprising one highly conservative amino acid substitution, preferably corresponding to position 1, 2, 3, 4, 5, 6, 7, 8, 9 and/or 10 of SEQ ID NO: 1. In a further embodiment of the invention, the TCR may have further antigenic specificity to an epitope presented in the context of a MHC class I molecule, the epitope comprising or consisting of the amino acid sequence of SEQ ID NO: 1 comprising one conservative amino acid substitution corresponding to position 1, 2, 3, 4, 5, 7, 8, 9 and/or 10 of SEQ ID NO: 1. In a further embodiment of the invention, the TCR may have further antigenic specificity to an epitope presented in the context of a MHC class I molecule, the epitope comprising or consisting of the amino acid sequence of SEQ ID NO: 1 comprising one highly conservative amino acid substitution corresponding to position 1, 2, 3, 4, 5, 7, 8, 9 and/or 10 of SEQ ID NO: 1.

In a further embodiment of the invention, the TCR may further have antigenic specificity to an epitope presented in the context of a MHC class I molecule, the epitope comprising or consisting of the amino acid sequence of SEQ ID NO: 1 comprising two conservative amino acid substitutions, preferably corresponding to position 1, 2, 3, 4, 5, 6, 7, 8, 9 and/or 10 of SEQ ID NO: 1. In a further embodiment of the invention, the TCR may further have antigenic specificity to an epitope presented in the context of a MHC class I molecule, the epitope comprising or consisting of the amino acid sequence of SEQ ID NO: 1 comprising two highly conservative amino acid substitutions, preferably corresponding to position 1, 2, 3, 4, 5, 6, 7, 8, 9 and/or 10 of SEQ ID NO: 1. In a further embodiment of the invention, the TCR may have further antigenic specificity to an epitope presented in the context of a MHC class I molecule, the epitope comprising or consisting of the amino acid sequence of SEQ ID NO: 1 comprising two conservative amino acid substitutions corresponding to position 1, 2, 3, 4, 5, 7, 8, 9 and/or 10 of SEQ ID NO: 1. In a further embodiment of the invention, the TCR may have further antigenic specificity to an epitope presented in the context of a MHC class I molecule, the epitope comprising or consisting of the amino acid sequence of SEQ ID NO: 1 comprising two highly conservative amino acid substitutions corresponding to position 1, 2, 3, 4, 5, 7, 8, 9 and/or 10 of SEQ ID NO: 1.

The term "antigenic specificity," as used herein, means that the TCR can specifically bind to and immunologically recognize mutated target, e.g., mutated KRAS peptide as defined herein, with high avidity. Effector host cells expressing the TCR as described herein are envisaged to bind to their antigenic target (i.e. the epitope(s) as defined herein) with a high functional avidity. The term "functional avidity" refers to the capability of TCR expressing cells (in particular T-cells expressing native TCRs as described herein) to respond *in vitro* to a given concentration of a ligand and is thought to correlate with the *in vivo* effector capacity of TCR expressing cells. By definition, TCR expressing cells with high functional avidity respond in *in vitro* tests to very low antigen doses, while such cells of lower functional avidity require higher amounts of antigen before they mount an immune response similar to that of high-avidity TCR expressing cells. The functional avidity can be therefore considered as a quantitative determinant of the activation threshold of a TCR expressing cell. It is determined by exposing such cells *in vitro* to different amounts of cognate antigen. TCR expressing cells with high functional avidity respond to low antigen doses. For example, a TCR expressing cell will typically be considered to bind with "high" functional avidity to its antigenic target (having antigenic specificity for the mutated epitope(s)) if a) it secretes at least about 200 pg/mL or more (e.g., 200 pg/mL or more, 300 pg/mL or more, 400 pg/mL or more, 500 pg/mL or more, 600 pg/mL or more, 700 pg/mL or more, 1000 pg/mL or more, 5,000 pg/mL or more, 7,000 pg/mL or more, 10,000 pg/mL or more, or 20,000 pg/mL or more, or a range defined by any two of the foregoing values) of interferon gamma (IFN-gamma) upon co-culture with antigen-negative HLA-A*11 expressing target cells loaded with a low concentration of the mutated target peptide ranging from about 10⁻⁵ to about 10⁻¹¹M (i.e., about0.05 ng/mL to about 5 ng/mL, 0.05 ng/mL, 0.1 ng/mL, 0.5 ng/mL, 1 ng/mL, or 5 ng/mL or a range defined by any two of the foregoing values), or (b) antigen-negative HLA-A11⁺ target cells into which a nucleotide sequence encoding the mutated target has been introduced such that the target cell expresses the mutated target. Cells expressing the TCR of the invention may also secrete IFN-γ upon co-culture with antigen-negative HLA-A11⁺ target cells pulsed with higher concentrations of mutated target peptide.

Other methods to determine specific binding of the TCR of the invention may include the 51Cr-release assay described by Gertner-Dardenne et al. J Immunol 188(9): 4701-4708, CD107a/b mobilization described by Leisegang et al., Clin. Cancer Res 2010. 16: 2333-2343 and peptide:MHC multimer binding analyses described by Wilde et al., J Immunol 2012; 189:598-605.

Alternatively or additionally, a TCR may be considered to have "antigenic specificity" for a mutated target if T cells expressing the TCR secrete at least twice as much IFN-γ upon co-culture with (a) antigen-negative HLA-A11⁺ target cells pulsed with a low concentration of mutated target peptide or (b) antigen-negative HLA-A11⁺ target cells into which a nucleotide sequence encoding the mutated target has been introduced such that the target cell expresses the mutated target as compared to the amount of IFN-γ expressed by a negative control. The negative control may be, for example, (i) T cells expressing the TCR, co-cultured with (a) antigen-negative HLA-A11⁺ target cells pulsed with the same concentration of an irrelevant peptide (e.g., some other peptide with a different sequence from the mutated target peptide) or (b) antigen-negative HLA-A11⁺ target cells into which a nucleotide sequence encoding an irrelevant peptide has been introduced such that the target cell expresses the irrelevant peptide, or (ii) untransduced T cells (e.g., derived from PBMC, which do not express the TCR) co-cultured with (a) antigen-negative HLA-A11⁺ target cells pulsed with the same concentration of mutated target peptide or (b) antigen-negative HLA-A11⁺ target cells into which a nucleotide sequence encoding the mutated target has been introduced such that the target cell expresses the mutated target. IFN-γ secretion may be measured by methods known in the art such as, for example, enzyme-linked immunosorbent assay (ELISA).

Alternatively or additionally, a TCR may be considered to have "antigenic specificity" for a mutated target if at least twice as many of the numbers of T cells expressing the TCR secrete IFN-γ upon co-culture with (a) antigen-negative HLA-A11⁺ target cells pulsed with a low concentration of mutated target peptide or (b) antigen-negative HLA-A11⁺ target cells into which a nucleotide sequence encoding the mutated target has been introduced such that the target cell expresses the mutated target as compared to the numbers of negative control T cells that secrete IFN-γ. The concentration of peptide and the negative control may be as described herein. The numbers of cells secreting IFN-γ may be measured by methods known in the art such as, for example, ELISPOT.

Preferably, the TCR of the present invention specifically binds to the abovementioned epitopes. The term "specific(ally) binding" generally indicates that a TCR binds via its antigen binding site more readily to its intended epitope than to a random, unrelated non-target antigen. Particularly the term "specifically binds" indicates that the binding specificity of the TCR will be at least about 5-fold, preferably 10-fold, more preferably 25-fold, even more preferably 50-fold, and most preferably 100-fold or more, greater for its epitope than its binding specificity for a non-target antigen.

Each embodiment concerning the antigenic specificity for the abovementioned epitope(s) as defined herein may also be applicable to the TCR of the invention being able to recognize said mutated epitope(s) in the context of a MHC class II molecule.

The TCR of the invention is able to recognize said mutated epitope (antigenic target), e.g., mutated KRAS, in a MHC class I and/or class II-dependent manner, meaning the epitope(s) is/are presented in the context of a MHC class I and/or class II molecule. "MHC class I-dependent manner," as used herein, means that the TCR elicits an immune response upon binding to a mutated target, e.g., mutated KRAS peptide as defined herein, within the context of an MHC class I molecule. The MHC class I molecule can be any MHC class I molecule known in the art. Such MHC class I molecule covers any HLA-A, HLA-B and/or HLA-C molecules. "MHC class II-dependent manner," as used herein, means that the TCR elicits an immune response upon binding to a mutated target, e.g., mutated KRAS peptide as defined herein, within the context of an MHC class II molecule.

In a preferred embodiment of the invention, the TCR as defined herein may further have antigenic specificity for the mutated epitope as defined above presented in the context of an HLA-A molecule. In a more preferred embodiment of the invention, the TCR as defined herein may further have antigenic specificity for the mutated epitope as defined above presented in the context of: an HLA-A molecule, preferably HLA-A*11 molecule; an HLA-A*11:01 molecule; and/or at least any one of an HLA-A*11 molecule, preferably HLA-A*11:01, HLA-A* 11 :02, HLA-A* 11:03 and/or HLA-A*11 :12.

As used herein, the term "epitope" may refer to a part of an antigen (antigenic target) that is recognized by the immune system, specifically by antibodies, B cells, or T cells. The epitope is the specific piece of the antigen to which the TCR binds to (particularly with the CDR3 as the prime determinant of antigen recognition and specificity) and is defined herein as mentioned elsewhere above.

As used herein, the term "human leukocyte antigen" or "HLA" may refer to any of various (polymorphic) proteins that are encoded by genes of the major histocompatibility complex in humans and are found on the surface of many cell types (such as white blood cells). HLA class I alleles include HLA-A, HLA-B and HLA-C alleles (http://hla.alleles.org/genes/index.html). The human major histocompatibility complex (MHC) is divided into 3 regions on chromosome 6p21.3: class II (centromeric), class III, and class I (telomeric), with extended class I and class II regions on either side.

As used herein, the term "major histocompatibility complex" or "MHC" may refer to a group of genes in mammals that code for cell-surface polymorphic glycoprotein molecules which display antigenic peptide fragments for T cell recognition and aid in the ability of the immune system to determine self from nonself. The MHC encodes highly polymorphic proteins, many of which are associated with the immune system. The products of classical polymorphic class I genes, human leukocyte antigen-A (HLA-A), HLA-B, and HLA-C, interact with T-cell receptor (TCR) molecules, as well as killer immunoglobulin-like receptors (KIRs) expressed on natural killer cells and some T cells.

As used herein, the term "allele" may refer to any of the alternative forms of a gene (e.g., human HLA gene) that may occur at a given locus. Each HLA allele name has a unique number corresponding to up to four sets of digits separated by colons. The length of the allele designation is dependent on the sequence of the allele and that of its nearest relative.

HLA-A alleles may be clustered into superfamilies on the basis of structural motifs and peptide binding specificity. These superfamilies reflect the broad characteristics of anchor motifs present in the peptides that they bind and present. Typically, an HLA allele name comprises the following elements: "HLA" prefix, hyphen ("-") used to separate gene name from HLA prefix, gene name (e.g., "A", "B", "C"), separator "*", field 1 depicting allele group (e.g., 03, 11, 01, 26, 24, 32, 02, 07, 15, 58, 40, 35, 08, 04, 07, 16, 03, 07 or 06 etc.), field separator ":", field 2 depicting specific HLA protein (e.g., 01 or 02 etc.). Exemplary human class I HLA genes of the present invention include HLA-A (Class I α-chain), HLA-B (Class I α-chain) and HLA-C (Class I α-chain). Further human class I HLA alleles of the present invention include HLA-E, HLA-F, HLA-G alleles as well as pseudogenes HLA-H, HLA-J, HLA-K, HLA-L, HLA-N, HLA-P, HLA-S, HLA-T, HLA-U, HLA-V, HLA-W and HLA-Y (http://hla.alleles.org/alleles/class1.html). Exemplary alleles of the present invention include any human class I and class II HLA alleles, e.g., selected from the group consisting of class I HLA-A, HLA-B and HLA-C alleles. Such alleles may be selected from the following exemplary human HLA allele groups: HLA-A*03, HLA-A*11, HLA-A*01, HLA-A*26, HLA-A*24, HLA-A*32 and HLA-A*02; HLA-B*07, HLA-B*15, HLA-B*58, HLA-B*40, HLA-B*35 and HLA-B*08; HLA-C*04, HLA-C*07, HLA-C*16, HLA-C*03, HLA-C*07 and HLA-C*06 alleles. Exemplary human HLA alleles of the present invention include but are not limited to: HLA-A*03:01, HLA-A*30:01, HLA-A*31:01, HLA-A*33:01, HLA-A*68:01, HLA-A*11:01, HLA-A*11:02, HLA-A*11:03, HLA-A*11:04 alleles. According to http://hla.alleles.org/alleles/index.html there are currently 32330 HLA and related alleles described by the HLA nomenclature and included in the IPD-IMGT/HLA Database Release 3.46 (2021-10) Build 2d19adf (https://www.ebi.ac.uk/ipd/imgt/hla/). Any human class I HLA allele (e.g., HLA-A, HLA-B or HLA-C allele) may be within the meaning of the present invention. Any human class II HLA allele may be within the meaning of the present invention. Exemplary class II HLA alleles of the present invention further include but are not limited to alleles of HLA-DRA, HLA-DRB1, HLA-DRB2-9, HLA-DQA1, HLA-DQA2, HLA-DQB1, HLA-DPA1, HLA-DPB1, HLA-DMA, HLA-DMB, HLA-DOA, HLA-DOB proteins (e.g., http://hla.alleles.org/alleles/class2.html). Further human class I and class II HLA alleles may also be queried at the IPD-IMGT/HLA Database as described above using "Allele Query Tool".

Within the context of the present invention, the HLA-A3 superfamily is of high importance within the HLA-A allotype. HLA-A*11 forms a part of the HLA-A3 superfamily which further comprises, but is not limited to, HLA-A*03:01, HLA-A*30:01, HLA-A*31:01, HLA-A*33:01 and HLA-A*68:01, characterized by recognition of peptides with small or aliphatic residues in position 2 and basic residues at their C-terminus. All currently classified HLA-A*11 serotypes fall into the A3 superfamily (Sidney et al., 2008, BMC Immunology; 9(1)). The overwhelming majority of HLA-A*11 serotypes are the HLA-A*11:01, *11:02, *11:03, or 11:04 subtypes, the novel TCR is capable of binding to the abovementioned epitopes presented by an HLA-A*11:01, an HLA-A**11:02, and/or an HLA-A**11:03 molecule within the HLA-A*11 (Habel et al., 2022, PLoS Pathog; 18(3)).

As used herein, the term "functionally expressed" may refer to HLA allele/s that is/are expressed (e.g., on cell surface) and functioning as HLA protein/s, e.g., presenting peptides derived from endogenous protein (e.g., from inside the cell). The term "functionally expressed" may in particularly refer to HLA allele/s that is/are expressed (e.g., on cell surface, e.g., of the mammal of the present invention) and functioning as HLA protein/s, e.g., presenting peptides derived from endogenous protein (e.g., from inside the cell, e.g., of the mammal of the present invention) in that the corresponding MHC I polypeptides are expressed on the surface of cells, e.g., of the mammal of the present invention, and present MHC antigens to which the non-human mammal providing an antigen-specific CD8⁺ T cell response and, optionally, providing an antigen-specific CD4⁺ T cell response.

The present invention may further comprise the TCR as defined herein, wherein the TCR has said antigenic specificity for said epitope(s) as defined herein in the absence of the CD8 co-receptor. The CD8 co-receptor is known to directly bind to HLA class I molecules and to be critical for the development of CD8⁺ T cells. CD8 co-receptor stabilizes the binding of a TCR to the HLA:peptide complex and facilitates early events of the TCR signaling cascade. The present inventors showed that the TCR of the invention as defined elsewhere herein restricted to MHC class I molecules can induce T cell activation upon antigen encounter without the need of CD8-costimulation.

### Chimeric co-stimulatory receptor(s):

As used herein the term "Chimeric co-stimulatory receptor(s)", may refer to a chimeric co-stimulatory molecule(s) or chimeric receptor(s), which may be synthetic and/or engineered cell-surface receptor(s) designed to enhance the activation and/or function of immune cell(s), particularly T-cells, preferably in the context of cancer immunotherapy. These receptors are chimeric in nature because they combine components, fragments and/or portions from different receptors (e.g., naturally occurring, recombinant and/or synthetic) to create a novel receptor with specific properties (e.g., therapeutic properties). Preferably, the structure of a chimeric co-stimulatory receptor of the present invention comprises three main components: **(1) Extracellular Domain:** this portion may be responsible for recognizing and/or binding to a specific target molecule, e.g., on cancer cell(s) and/or other cells of interest. It can be derived from various receptors (e.g., PD-1), such as antibodies and/or ligands, that are capable of binding to the target. For example, the extracellular domain (preferably comprising or consisting of SEQ ID NO: 28) might be derived from a PD-1 (Programmed Cell Death Protein 1), which is a cell surface receptor protein that plays a role in the regulation of the immune system. It is primarily found on the surface of certain immune cells, particularly T cells, B cells, and some natural killer (NK) cells. Further example, the extracellular domain might be derived from a monoclonal antibody targeting a cancer-specific antigen. Further example, the extracellular domain might be derived from CD40L; **(2) Transmembrane Domain (TMD):** this region can be derived from a natural cell surface receptor and/or can anchor the chimeric receptor in the cell membrane; Preferably, TMD of the present invention might be derived from a PD-1 and further preferably comprising or consisting of SEQ ID NO: 30; **(3) Intracellular Domain:** the intracellular domain is where the co-stimulatory signalling activity is conferred. Preferably, the intracellular domain of the present invention might be derived from 4-1BB protein and further preferably comprising or consisting of SEQ ID NO: 32. The intracellular domain of the present invention can be derived from a co-stimulatory receptor, such as CD28 and/or 4-1BB (CD137), which can provide activation signals to T cells. By incorporating a co-stimulatory signalling domain, the chimeric receptor of the present invention can be designed to provide strong activation signals to the immune cell upon binding its target. In some aspects, the present invention relates to fusion proteins comprising (a) an extracellular domain containing a polypeptide derived from PD-1 or CD40L at its N-terminus; (b) a transmembrane domain; and (c) an intracellular domain containing a polypeptide derived from 4-1BB or CD28 at its C-terminus. Also, fusion proteins with CD28 at the N-terminus and CD40L at the C-terminus are envisaged. In further aspects, the present invention relates to a fusion protein comprising: (a) an extracellular domain (ECD) containing a polypeptide derived from PD-1 or CD40L at its N-terminus; (b) a transmembrane domain (TMD); and (c) an intracellular domain (ICD) containing a polypeptide derived from 4-1BB or CD28 at its C-terminus. Preferably, in accordance with the present invention, if the extracellular domain (ECD) contains a polypeptide derived from PD-1 at its N-terminus, the intracellular domain (ICD) contains a polypeptide derived from 4-1BB at its C-terminus and vice versa. Likewise, if the extracellular domain contains a polypeptide derived from CD40L at its N-terminus, the intracellular domain contains a polypeptide derived from CD28 at its C-terminus and vice versa. For fusions proteins with an ECD derived from CD40L and an ICD derived from CD28, it is also possible that the ICD is located N-terminally of the TMD, while the TMD is located at the very C-terminus of the fusion protein. Furthermore, the present invention relates to a fusion (i.e., chimeric) protein comprising the extracellular domain (ECD) of CD40L and the intracellular signaling domain (ICD) of CD28. In the context of the present invention the term "fusion" and "chimeric" can be used interchangeably.

In context with the present invention, unless otherwise specified herein, when a chimeric co-stimulatory receptor-expressing T cell encounters a cell expressing the target molecule (e.g., a cancer cell), the receptor's extracellular domain binds to the target, and the intracellular domain delivers co-stimulatory signals to the T cell. This activation can enhance the immune cell's ability to recognize, attack, and/or eliminate the target cells, particularly in the context of cancer immunotherapy.

### PD1-41BB:

As described herein references can be made to UniProtKB Accession Numbers (http://www.uniprot.org/, e.g., as available in UniProt release 2023_03 published 28 Jun 2023). As described herein the term "PD1-41BB" may refer to a fusion polypeptide comprising a PD-1-derived polypeptide (e.g., wherein said PD-1 is also known as Programmed Cell Death Protein 1, e.g., having UniProt Accession Number: Q15116) and a 4-1BB-derived polypeptide (e.g., wherein said 4-1BB is also known as CD137 or TNFRS9, which can be used interchangeably, e.g., having UniProt Accession Number: Q07011). In this context, the term "derived from" may particularly mean that the polypeptide contained in the extracellular domain comprises at least a part of PD-1 protein (e.g., human PD-1), preferably the extracellular domain of PD-1, respectively. The chimeric co-stimulatory receptor comprising an extracellular domain derived from PD-1 has binding activity for PD-L1, PD-L2 or other inhibitory ligands of PD-1. As used herein, the term "derived from" PD-1 also allows that up to 0, 1, 2, 3, 4, 5, 6, 7, 8, 9, 10 or more amino acids are substituted, deleted, and/or inserted compared to a native (or parent) sequence of PD-1 (e.g. human PD-1) or part thereof (e.g., extracellular domain). In this context, the term "derived from" may particularly mean that the polypeptide contained in the intracellular domain comprises at least a part of 4-1BB protein (e.g., human 4-1BB), preferably the intracellular domain of 4-1BB, respectively. The chimeric co-stimulatory receptor comprising an intracellular domain derived from 4-1BB is capable of increasing the proliferation rate of a T cell expressing said chimeric co- stimulatory receptor upon stimulation with PD-L1, PD-L2 or another inhibitory ligand of PD-1 and/or is capable of increasing the effector function (such as increased IFN-y release and/or increased cytotoxicity) of a T cell expressing said chimeric co-stimulatory receptor compared to a corresponding T cell not expressing the chimeric co-stimulatory receptor. As used herein, the term "derived from" 4-1BB also allows that up to 0, 1, 2, 3, 4, 5, 6, 7, 8, 9, 10 or more amino acids are substituted, deleted, and/or inserted compared to a native (or parent) sequence of 4-1BB (human or murine, preferably human 4-1BB) or part thereof (e.g., intracellular domain).

A PD-1-4-1BB fusion polypeptide as described herein, also referred to as PD1-41BB fusion, can refer to a chimeric protein resulting from a fusion of the PD-1 and 4-1BB proteins and/or fragment(s) and/or portion(s) thereof. For example, such a fusion protein can be associated and/or used with/for cancer immunotherapy and/or is designed to harness the immune system to target cancer cells more effectively. In the context of the present invention, unless otherwise specified herein, the PD-1 portion of the fusion protein (e.g., the extracellular domain, e.g., SEQ ID NO: 28 and/or transmembrane domain, e.g., SEQ ID NO: 30) may allow the fusion polypeptide of the present invention to bind to PD-L1 and/or PD-L2 ligands on the surface of cancer cells and/or antigen-presenting cells. PD-1 binding to its ligands normally sends inhibitory signals to T cells, preventing them from attacking these cells. However, in the context of a PD-1-4-1BB fusion of the present invention, this interaction may be modified. In the context of the present invention, unless otherwise specified herein, the 4-1BB portion of the fusion protein (e.g., the intracellular domain, e.g., SEQ ID NO: 32) may provide a co-stimulatory signal to T cells when engaged. This co-stimulatory signal may help activate and/or enhance the immune response against the cancer cells, overcoming the inhibitory effects of the PD-1 portion. Accordingly, the fusion protein of the present invention may combine the checkpoint-blocking capability of PD-1 with the immune-activating potential of 4-1BB. Preferably, a PD-1-4-1BB fusion of the present invention is capable of enhancing the anti-tumor immune response, e.g., by simultaneously blocking the inhibitory signals mediated by PD-1 and providing a strong co-stimulatory signal through 4-1BB. This approach is advantageous for use in immunotherapy (e.g., for cancer treatment), as it may overcome some of the resistance mechanisms that cancer cells often use to evade the immune system.

Generally, in context with the present invention, unless otherwise specified herein, fusion proteins comprising or consisting of the extracellular domain (ECD) derived from PD-1, a transmembrane domain (TMD, e.g., derived from PD-1), and the intracellular domain (ICD) derived from 4-1BB are also referred to herein as "PD-1:4-1BB" or "PD-1:BB". Likewise, fusion proteins comprising or consisting of the extracellular domain (ECD) derived from PD-1, a transmembrane domain (TMD), and the intracellular domain (ICD) derived from CD28 are also referred to herein as "PD-1:CD28". In context with the present invention, unless otherwise specified herein, CD28 (e.g., having UniProt Accession Number: P10747) may refer to another cell surface receptor found on T cells, and it plays a co-stimulatory role in the activation of T cells. CD28 signalling helps promote T cell activation and proliferation when engaged by its ligands, such as B7-1 (CD80) and B7-2 (CD86), on antigen-presenting cells. Likewise, fusion proteins comprising or consisting of the extracellular domain (ECD) derived from CD40L (e.g., having UniProt Accession Number: P29965), a transmembrane domain (TMD), and the intracellular domain (ICD) derived from CD28 are also referred to herein as "CD40L:CD28".

In context with the present invention, unless otherwise specified herein, PD-1 (e.g., having UniProt Accession Number: Q15116) is a cell surface receptor primarily found on the surface of T cells. It can play an important role in regulating the immune response by inhibiting T cell activation. For example, when PD-1 binds to its ligands PD-L1 or PD-L2, which can be found on other cells, it sends inhibitory signals to the T cell, essentially putting the brakes on the immune response. This mechanism helps prevent the immune system from attacking healthy cells and tissues, but it can also be exploited by cancer cells to evade immune surveillance.

In context with the present invention, unless otherwise specified herein, 4-1BB (also known as CD137 or TNFRS9, which can be used interchangeably, e.g., having UniProt Accession Number: Q07011) is another cell surface receptor, but it has a different role than PD-1. For example, 4-1BB is typically found on activated T cells and natural killer (NK) cells. When 4-1BB is engaged by its ligand, it provides a co-stimulatory signal that enhances the activation and proliferation of these immune cells. This can lead to a more robust immune response against pathogens or cancer cells.

In context with the present invention, the term "extracellular domain", also known as the "extracellular region" or "extracellular portion", may refer to a structural component of protein(s), which can be found at the outer surface of cells and/or within the extracellular matrix. This region of a protein is preferably located outside the cell membrane and/or the confines of a cellular compartment, e.g., where it may play a role in various biological functions, including cell signalling, recognition, and/or interaction with other molecules. Preferably, the extracellular domain of the present invention is a polypeptide derived from the PD-1 (e.g., human PD-1, e.g., comprising or consisting of SEQ ID NO: 28 or a variant thereof).

In context with the present invention, the term "transmembrane domain", which can also be abbreviated as "TMD", may refer to a structural component of integral membrane proteins. Integral membrane proteins are a class of proteins that are embedded within the lipid bilayer of a biological membrane, such as the cell membrane (e.g., plasma membrane) and/or the membranes of organelles like the endoplasmic reticulum and/or mitochondria. Preferably, TMD of the present invention is a polypeptide derived from the PD-1 (e.g., human PD-1, e.g., comprising or consisting of SEQ ID NO: 30 or a variant thereof).

In context with the present invention, the term "intracellular domain", which can also be referred to as "cytoplasmic domain", may refer to a structural component of integral membrane proteins. It may refer to the portion of the protein that is located on the cytoplasmic side of a biological membrane, such as the cell membrane (e.g., plasma membrane) and/or the membranes of organelles like the endoplasmic reticulum, Golgi apparatus, and/or mitochondria. Preferably, the intracellular domain of the present invention is a polypeptide derived from 4-1BB (e.g., human 4-1BB, e.g., comprising or consisting of SEQ ID NO: 32 or a variant thereof).

In context with the present invention, the term "polypeptide complex", which can be used interchangeably with "protein complex" may refer to a group of two or more individual protein or polypeptide molecules (e.g., a TCR and a chimeric co-stimulatory receptor as defined herein) that are bound together, preferably physically and functionally, to perform a specific biological function and/or task within a cell (e.g., T-cell) or organism.

In context with the present invention, the term "combination" may refer to the coexistence and/or interaction (e.g., functional interaction) of a group of two or more individual protein or polypeptide molecules (e.g., a TCR and a chimeric co-stimulatory receptor as defined herein) within a cellular context (e.g., on/in a cell, preferably a T-cell; or population of cells as define herein, e.g., population of T cells) or biological system.

In some aspects, the present invention provides/relates to a T-cell receptor (TCR) polypeptide complex and/or a combination comprising: a TCR; and a chimeric co-stimulatory receptor, wherein the TCR comprises: i) an α chain Complementarity Determining Region 3 (CDR3) comprising an amino acid sequence having at least 80 % identity to SEQ ID NO: 16; and/or ii) a β chain Complementarity Determining Region 3 (CDR3) comprising an amino acid sequence having at least 80 % identity to SEQ ID NO: 4.

The present invention may further encompass the TCR as defined herein, wherein the TCR is selected from the group consisting of a native TCR, a TCR variant, a TCR fragment, or a TCR construct.

### TCR variants:

As noted previously, the term "TCR" encompasses TCR variants, which include TCR sequence variants, fragments and constructs. All TCR variants are envisaged to be functional variants of the TCR of the invention. The term "functional variant" as used herein refers to a TCR, polypeptide, or protein having substantial or significant sequence identity or similarity to a parent TCR, its variable regions or its antigen-binding regions and shares its biological activity, i.e. its ability to specifically bind to the antigenic target for which the parent TCR of the invention has antigenic specificity to a similar, the same or even a higher extent as the TCR disclosed herein.

The term "TCR variants" includes "sequence variants" of the TCR disclosed herein, i.e. variants substantially comprising the amino acid sequence of the TCR as described above (also referred to as the "parent" TCR) but containing at least one amino acid modification (i.e. a substitution, deletion, or insertion) as compared to the "parent" / "native" TCR amino acid sequence, provided that the variant preferably retains the antigenic specificity of the inventive "patent" TCR as shown in the Examples. TCR sequence variants of the invention are typically prepared by introducing appropriate nucleotide changes into the nucleic acids encoding the "parent" TCR, or by peptide synthesis. Generally, the aforementioned amino acid modifications may be introduced into, or present in, the variable region or the constant region of the TCR, and may serve to modulate properties like binding strength and specificity, post-translational processing (e.g. glycosylation), thermodynamic stability, solubility, surface expression or TCR assembly.

As set out previously, amino acid modifications include, for example, deletions from, and/or insertions into, and/or substitutions of, residues within the amino acid sequences of the parent TCR. Exemplary insertional variants of a TCR of the invention include fusion products of said TCR and an enzyme or another functional polypeptide. Exemplary substitutional variants of a TCR of the invention are those including amino acid substitutions in variable regions or CDRs of the alpha and/or beta chain, the framework region or the constant region.

The addition of a disulfide bond in the constant region has been reported to foster correct pairing of the TCR alpha and beta chains (Kuball J et al. Blood. 2007 Mar 15; 109(6):2331-8.). Thus, the addition of one or more cysteine bonds in the constant region is also envisaged herein.

Murinization of TCRs (i.e. exchanging the human constant regions in the alpha and beta chain by their murine counterparts) is a technique that is commonly applied in order to improve cell surface expression of TCRs in host cells. Without wishing to be bound by specific theory, it is thought that murinized TCRs associate more effectively with CD3 co-receptors; and/or that preferentially pair with each other and are less prone to form mixed TCRs on human T cells engineered ex *vivo* to express the TCRs of desired antigenic specificity, but still retaining and expressing their "original" TCRs.

Recently nine amino acids responsible for the improved expression of murinized TCRs have been identified (Sommermeyer and Uckert, J Immunol. 2010 Jun 1; 184(11):6223-31) and it is envisaged to substitute one or all of the amino acid residues in the TCRs alpha and/or beta chain constant region for their murine counterpart residues. This technique is also referred to as "minimal murinization" and offers the advantage of enhancing cell surface expression while, at the same time, reducing the number of "foreign" amino acid residues in the amino acid sequence and, thereby, the risk of immunogenicity.

In general, TCR sequence variants are envisaged to comprise at least one of the CDR1, CDR2, CDR3, alpha chain variable regions, beta chain variable regions, alpha chains and/or beta chains as disclosed herein, or comprising or consisting of an amino acid sequence that is at least about 80%, about 85%, about 90%, about 95% , about 96%, about 97%, about 98%, about 99%, or identical to the amino acid sequences disclosed herein, provided that said variants exhibit comparable, the same or improved binding characteristics as compared to the TCR evaluated in the Examples.

### TCR constructs and fragments:

The term "TCR" as used herein further comprises TCR constructs. The term "construct" includes proteins or polypeptides comprising at least one antigen binding domain of the TCR of the invention, but do not necessarily share the basic structure of a native TCR (i.e. variable regions incorporated into a TCR alpha chain and a TCR beta chain forming a heterodimer). TCR constructs and fragments are typically obtained by routine methods of genetic engineering and are often artificially constructed to comprise additional functional protein or polypeptide domains. In accordance with the foregoing, TCR constructs and fragments of the invention are envisaged to comprise at least one CDR3alpha and/or at least one CDR3beta as disclosed elsewhere herein. Further envisaged herein are constructs and fragments comprising at least one CDR1alpha, CDR2alpha, CDR1beta, CDR2beta, alpha chain variable region, beta chain variable region, alpha chain and/or beta chain, or combinations thereof, optionally in combination with further protein domains or moieties as exemplified herein. The TCR constructs and fragments provided herein may further be envisaged to be capable of specifically binding to the same antigenic target as the TCR described above and evaluated in the Examples.

The present invention may also comprise the TCR construct as defined herein comprising at least one TCR alpha-chain(s) and at least one TCR beta-chain(s) covalently linked to each other to form TCR heterodimers or multimers.

The term "TCR construct" encompasses heterodimers and multimers in which at least one TCR alpha chain variable region or TCR alpha-chain and at least one TCR beta-chain variable region are covalently linked to each other. In its simplest form a multivalent TCR construct according to the invention comprises a multimer of two or three or four or more TCRs associated (e. g. covalently or otherwise linked) with one another, preferably via a linker molecule.

Suitable linker molecules include, but are not limited to, multivalent attachment molecules such as avidin, streptavidin, neutravidin and extravidin, each of which has four binding sites for biotin. Thus, biotinylated TCRs can be formed into multimers having a plurality of TCR binding sites. The number of TCRs in the multimer will depend upon the quantity of TCR in relation to the quantity of linker molecule used to make the multimers, and also on the presence or absence of any other biotinylated molecules. Exemplary multimers are dimeric, trimeric, tetrameric or pentameric or higher-order multimer TCR constructs. Multimers of the invention may also comprise further functional entities such as labels or drugs or (solid) carriers.

The term "TCR construct" also encompasses TCR molecules which are linked via a suitable linker to a spheric body, preferably a uniform bead, more preferably a polystyrene bead, most preferably a bio-compatible polystyrene bead. Such TCR constructs can also be comprised of the TCR as defined herein and a bead having a pre-defined fluorescence dye incorporated into the bead.

### TCR fusion proteins:

The present invention may also comprise the TCR as defined herein, further comprising one or more fusion component(s) selected from the group consisting of a Fc receptor; a Fc domain, including IgA, IgD, IgG, IgE, and IgM; a cytokine, including IL-2 or IL-15; a toxin; an antibody or an antigen-binding fragment thereof, including anti-CD3, anti-CD28, anti-CD5, anti-CD 16 or an anti- CD56 antibody or an antigen-binding fragment thereof; or a CD247 (CD3-zeta), CD28, CD137, or a CD134 domain, or combinations thereof, optionally further comprising at least one linker. These are called a "fusion protein".

The term "TCR construct" may also relate to such fusion proteins or polypeptides comprising at least one TCR alpha chain, TCR alpha chain variable region or CDR3alpha and/or at least one TCR beta chain, TCR beta chain variable region or CDR3beta; and further one or more fusion component(s). Useful components include Fc receptors; Fc domains (derived from IgA, IgD, IgG, IgE, and IgM); cytokines (such as IL-2 or IL-15); toxins; antibodies or antigen-binding fragments thereof (such as anti-CD3, anti-CD28, anti-CD5, anti-CD 16 or anti- CD56 antibodies or antigen-binding fragments thereof); CD247 (CD3-zeta), CD28, CD137, CD134 domains; or any combinations thereof.

Exemplary antibody fragments that can be used as fusion components include fragments of full-length antibodies, such as (s)dAb, Fv, Fd, Fab, Fab', F(ab')2 or "r IgG" ("half antibody"); modified antibody fragments such as scFv, di-scFv or bi(s)-scFv, scFv-Fc, scFv-zipper, scFab, Fab2, Fab3, diabodies, single chain diabodies, tandem diabodies (Tandab's), tandem di-scFv, tandem tri-scFv, minibodies, multibodies such as triabodies or tetrabodies, and single domain antibodies such as nanobodies or single variable domain antibodies comprising only one variable domain, which might be VHH, VH or VL.

TCR constructs of the invention may be fused to one or more antibody or antibody fragments, yielding monovalent, bivalent and polyvalent/multivalent constructs and thus monospecific constructs, specifically binding to only one target antigen as well as bispecific and polyspecific/multispecific constructs, which specifically bind more than one target antigens, e.g. two, three or more, through distinct antigen binding sites.

Optionally, a linker may be introduced between the one or more of the domains or regions of the TCR construct of the invention, i.e. between the TCR alpha chain CDR3, TCR alpha chain variable region, and/or a TCR alpha chain, the TCR beta chain CDR3, TCR beta chain variable region, and/or a TCR beta chain, and/or the one or more fusion component(s) described herein. Linkers are known in the art and have been reviewed, *inter alia,* by Chen et al. Adv Drug Deliv Rev. 2013 Oct 15; 65(10): 1357-1369. In general, linkers include flexible, cleavable and rigid linkers and will be selected depending on the type of construct and intended use/application. For example, for therapeutic application, non-immunogenic, flexible linkers are often preferred in order to ensure a certain degree of flexibility or interaction between the domains while reducing the risk of adverse immunogenic reactions. Such linkers are generally composed of small, non-polar (e.g. Gly) or polar (e.g. Ser or Thr) amino acids and include "GS" linkers consisting of stretches of Gly and Ser residues.

Particularly useful TCR constructs envisaged in accordance with the invention are those comprising at least one TCR alpha chain, TCR alpha chain variable region or CDR3 alpha as defined herein, at least one TCR beta chain, TCR beta chain variable region or CDR3 beta as defined herein, optionally linked to each other and fused, optionally via a liker, to at least one antibody or an antibody fragment (such as a single chain antibody fragment (scFv)) directed against an antigen or epitope on the surface of lymphocytes, such as a T cell. Useful antigenic targets recognized by the antibody or antibody fragment (e.g. scFv) include CD3, CD28, CD5, CD16 and CD56. Said construct can in general have any structure as long the "TCR portion" (i.e. TCR alpha and beta chain or variable regions or CDR3s thereof) retains its ability to recognize the antigenic target defined herein, and the "antibody portion" binds to the desired surface antigen or epitope, thereby recruiting and targeting the respective lymphocyte to the target cell. Such constructs may advantageously serve as "adapters" joining an antigen presenting cell displaying the antigenic target (such as a tumor cell) and a lymphocyte (such as a cytotoxic T cell or NK cell) together. Accordingly, a TCR construct of the invention may comprise at least one TCR antigen binding domain as described herein (for instance a TCR variable alpha and variable beta chain fused to each other) linked to a scFv (or other binding domain) of the desired binding specificity, e.g. CD3 or CD56. The scFv (or other binding domain) binds to T cells such as via the CD3 receptor or to CD56 for NK cell activation, and the other to a tumor cell via the antigenic target as defined herein specifically expressed on the tumor cell. Also envisaged herein are tribodies comprising at least one TCR antigen binding domain as described herein, an scFv (or other binding domain) and a further domain e.g. for targeting the construct to a site of action within the body (e.g. an Fc domain).

### Labels:

The present invention may also comprise the TCR as defined herein, further comprising at least one label.

The TCR of the invention can be labelled. Useful labels are known in the art and can be coupled to the TCR or TCR variant using routine methods, optionally via linkers of various lengths. The term "label" or "labelling group" refers to any detectable label. In general, labels fall into a variety of classes, depending on the assay in which they are to be detected - the following examples include, but are not limited to: isotopic labels, which may be radioactive or heavy isotopes, such as radioisotopes or radionuclides (e.g., ³H, ¹⁴C, ¹⁵N, ³⁵S, ⁸⁹Zr, ⁹⁰Y, ⁹⁹Tc, ¹¹¹In, ¹²⁵I ¹³¹I); magnetic labels (e.g., magnetic particles); redox active moieties; optical dyes (including, but not limited to, chromophores, phosphors and fluorophores) such as fluorescent groups (e.g., FITC, rhodamine, lanthanide phosphors), chemiluminescent groups, and fluorophores which can be either "small molecule" fluorophores or proteinaceous fluorophores; enzymatic groups (e.g. horseradish peroxidase, β-galactosidase, luciferase, alkaline phosphatase; biotinylated groups; or predetermined polypeptide epitopes recognized by a secondary reporter (e.g., leucine zipper pair sequences, binding sites for secondary antibodies, metal binding domains, epitope tags, etc.). Labelling is particularly envisaged when the TCR, TCR variants or especially soluble TCR constructs (such as those comprising at least one TCR alpha and/or TCR beta chain as described herein) are intended for diagnostic use.

### Functional moieties:

The TCR of the invention can be modified by attaching further functional moieties, e.g. for reducing immunogenicity, increasing hydrodynamic size (size in solution) solubility and/or stability (e.g. by enhanced protection to proteolytic degradation) and/or extending serum half-life.

Exemplary functional moieties for use in accordance with the invention include peptides or protein domains binding to other proteins in the human body (such as serum albumin, the immunoglobulin Fc region or the neonatal Fc receptor (FcRn), polypeptide chains of varying length (e.g., XTEN technology or PASylation^{®}), non-proteinaceous polymers, including, but not limited to, various polyols such as polyethylene glycol (PEGylation), polypropylene glycol, polyoxyalkylenes, or copolymers of polyethylene glycol and polypropylene glycol, or of carbohydrates, such as hydroxyethyl starch (e.g., HESylation^{®}) or polysialic acid (e.g., PolyXen^{®} technology).

Other useful functional moieties include "suicide" or "safety switches" that can be used to shut off effector host cells carrying the TCR in a patient's body. An example is the inducible Caspase 9 (iCasp9) "safety switch" described by Gargett and Brown Front Pharmacol. 2014; 5: 235. Briefly, effector host cells are modified by well-known methods to express a Caspase 9 domain whose dimerization depends on a small molecule dimerizer drug such as AP1903/CIP, and results in rapid induction of apoptosis in the modified effector cells. The system is for instance described in EP2173869 (A2). Examples for other "suicide" "safety switches" are known in the art, e.g. Herpes Simplex Virus thymidine kinase (HSV-TK), expression of CD20 and subsequent depletion using anti-CD20 antibody or myc tags (Kieback et al, Proc Natl Acad Sci USA. 2008 Jan 15;105(2):623-8).

### Glycosylation:

TCR as defined herein with an altered glycosylation pattern may also be envisaged herein. As is known in the art, glycosylation patterns can depend on the amino acid sequence (e.g., the presence or absence of particular glycosylation amino acid residues, discussed below) and/or the host cell or organism in which the protein is produced. Glycosylation of polypeptides is typically either N-linked or O-linked. N-linked refers to the attachment of the carbohydrate moiety to the side chain of an asparagine residue. Addition of N-linked glycosylation sites to the binding molecule is conveniently accomplished by altering the amino acid sequence such that it contains one or more tri-peptide sequences selected from asparagine-X-serine and asparagine-X-threonine (where X is any amino acid except proline). O-linked glycosylation sites may be introduced by the addition of or substitution by, one or more serine or threonine residues to the starting sequence.

Another means of glycosylation of TCRs is by chemical or enzymatic coupling of glycosides to the protein. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. Similarly, deglycosylation (i.e., removal of carbohydrate moieties present on the binding molecule) may be accomplished chemically, e.g. by exposing the TCR to trifluoromethanesulfonic acid, or enzymatically by employing endo- and exo-glycosidases.

### Drug conjugates:

It is also conceivable to add a drug such as a small molecule compound to the TCR of the invention. Linkage can be achieved via covalent bonds, or non-covalent interactions such as through electrostatic forces. Various linkers, known in the art, can be employed in order to form the drug conjugates.

### Tags:

The TCR of the invention can be modified to introduce additional domains which aid in identification, tracking, purification and/or isolation of the respective molecule (tags). Non-limiting examples of such tags comprise peptide motives known as Myc-tag, HAT-tag, HA-tag, TAP-tag, GST-tag, chitin binding domain (CBD-tag), maltose binding protein (MBP-tag), Flag-tag, Strep-tag and variants thereof (e.g. Strep II-tag), His-tag, CD20, Her2/neu tags, myc-tag, FLAG-tag, T7-tag, HA(hemagglutinin)-tag, or GFP-tags or epitope tag/s with or without linker (e.g., SEQ ID NOs: 41, 42, 43 or 44).

Epitope tags are useful examples of tags that can be incorporated into the TCR of the invention. Epitope tags are short stretches of amino acids that allow for binding of a specific antibody and therefore enable identification and tracking of the binding and movement of soluble TCRs or host cells within the patient's body or cultured (host) cells. Detection of the epitope tag, and hence, the tagged TCR, can be achieved using a number of different techniques. Examples of such techniques include: immunohistochemistry, immunoprecipitation, flow cytometry, immunofluorescence microscopy, ELISA, immunoblotting ("Western"), and affinity chromatography. The epitope tags can for instance have a length of 4 to 15 amino acids, in particular 6 to 12, most preferred 6 to 9 amino acids. It is also possible to include more than one epitope tag in the TCR of the invention.

Tags can further be employed for stimulation and expansion of host cells carrying the TCR by cultivating the cells in the presence of binding molecules (antibodies) specific for said tag.

### Soluble forms:

The TCR of the present invention can be provided in soluble form. Soluble TCRs are useful as diagnostic tools, and carriers or "adapters" that specifically target therapeutic agents or effector cells to, for instance, a cancer cell expressing the antigenic target recognized by the soluble TCR. Soluble TCRs (sTCRs) will typically be fragments or constructs comprising TCR alpha and/or beta chains, or variable regions or CDRs thereof and optionally stabilized via disulfide bonds or covalently linked via a suitable linker molecule, e.g. as described above in the context of TCR constructs of the invention. They will typically not comprise e.g. a transmembrane region. In some circumstances amino acid modifications in the polypeptide sequence may be introduced in order to enhance solubility of the molecules, and/or correct folding and pairing of the alpha and beta chains (if desired), in particular when produced in a recombinant host that does not provide for the aforementioned features. For instance, when using E. coli as production host cells, folding and pairing of the TCR alpha and beta chains is typically accomplished *in vitro.* TCR according to the invention may therefore for instance comprise additional cysteine residues, as described elsewhere herein.

Besides additional cysteine bridges, other useful modifications include, for instance, the addition of leucine zippers and/or ribosomal skipping sequences, e.g. sequence 2A from picorna virus as described in Walseng et al. (2015), PLoS ONE 10(4): e0119559 to increase folding, expression and/or pairing of the TCR alpha and/or beta chains.

### Nucleic acid:

The present invention further comprises a nucleic acid comprising a nucleotide sequence encoding the TCR as defined elsewhere herein.

The term "polynucleotide" or "nucleic acid" as used herein comprises a sequence of polyribonucleotides and polydeoxribonucleotides, e.g. modified or unmodified RNA or DNA, each in single-stranded and/or double-stranded form linear or circular, or mixtures thereof, including hybrid molecules. The nucleic acids according to this invention thus comprise DNA (such as dsDNA, ssDNA, cDNA), RNA (such as dsRNA, ssRNA, mRNA, ivtRNA), combinations thereof or derivatives (such as PNA) thereof.

A polynucleotide may comprise a conventional phosphodiester bond or a nonconventional bond (e.g., an amide bond, such as found in peptide nucleic acids (PNA)). The polynucleotides of the invention may also contain one or more modified bases, such as, for example, tritylated bases and unusual bases such as inosine. Other modifications, including chemical, enzymatic, or metabolic modifications, are also conceivable, as long as a binding molecule of the invention can be expressed from the polynucleotide. The polynucleotide may be provided in isolated form as defined elsewhere herein. A polynucleotide may include regulatory sequences such as transcription control elements (including promoters, enhancers, operators, repressors, and transcription termination signals), ribosome binding site, introns, or the like.

The nucleic acid can comprise any nucleotide sequence which encodes the TCR of the invention. In particular, the present invention may comprise a nucleic acid comprising or consisting of a nucleotide sequence encoding the TCR of the invention of at least about 80%, about 81%, about 82%, about 83%, about 84%, about 85%, about 90%, about 91%, about 92%, about 93%, about 94%, about 95%, about 96%, about 97%, about 98%, about 99%, or 100% identity to the nucleotide sequence of SEQ ID NO: 9 and/or SEQ ID NO: 21 (see **Table 2**). Thus, the present invention may comprise a nucleic acid comprising or consisting of a nucleotide sequence encoding the TCR of the invention of at least about 80%, about 81%, about 82%, about 83%, about 84%, about 85%, about 90%, about 91%, about 92%, about 93%, about 94%, about 95%, about 96%, about 97%, about 98%, about 99%, or 100% identity to the nucleotide sequence of SEQ ID NO: 9. Thus, the present invention may comprise a nucleic acid comprising or consisting of a nucleotide sequence encoding the TCR of the invention of at least about 80%, about 81%, about 82%, about 83%, about 84%, about 85%, about 90%, about 91%, about 92%, about 93%, about 94%, about 95%, about 96%, about 97%, about 98%, about 99%, or 100% identity to the nucleotide sequence of SEQ ID NO: 21.

Additionally, the present invention may also comprise a nucleic acid comprising or consisting of a nucleotide sequence encoding the TCR of the invention of at least about 80%, about 81%, about 82%, about 83%, about 84%, about 85%, about 90%, about 91%, about 92%, about 93%, about 94%, about 95%, about 96%, about 97%, about 98%, about 99%, or 100% identity to the nucleotide sequence of SEQ ID NO: 13 and/or SEQ ID NO: 25 (see **Table 2**). Thus, the present invention may comprise a nucleic acid comprising or consisting of a nucleotide sequence encoding the TCR of the invention of at least about 80%, about 81%, about 82%, about 83%, about 84%, about 85%, about 90%, about 91%, about 92%, about 93%, about 94%, about 95%, about 96%, about 97%, about 98%, about 99%, or 100% identity to the nucleotide sequence of SEQ ID NO: 13. Thus, the present invention may comprise a nucleic acid comprising or consisting of a nucleotide sequence encoding the TCR of the invention of at least about 80%, about 81%, about 82%, about 83%, about 84%, about 85%, about 90%, about 91%, about 92%, about 93%, about 94%, about 95%, about 96%, about 97%, about 98%, about 99%, or 100% identity to the nucleotide sequence of SEQ ID NO: 25.

The nucleic acid described above may or may not comprise additional or altered nucleotide sequences encoding e.g., altered amino acid residues, a signal peptide to direct secretion of the encoded TCR, constant regions or other heterologous polypeptides as described herein. Such nucleic acids may thus encode fusion polypeptides, fragments, variants and other derivatives of the binding molecules described herein. The nucleotide sequences of the present invention may be codon-optimized for optimal expression in the desired host cell, e.g. a human lymphocyte; or for expression in bacterial, yeast or insect cells that are particularly envisaged for the expression of the soluble TCR of the invention. Codon-optimization refers to the exchange in a sequence of interest of codons that are generally rare in highly expressed genes of a given species by codons that are generally frequent in highly expressed genes of such species, such codons encoding the same amino acids as the codons that are being exchanged. Selection of optimum codons thus depends on codon usage of the host genome and the presence of several desirable and undesirable sequence motifs.

The term "polypeptide" is equally used herein with the term "protein". Proteins (including fragments thereof, preferably biologically active fragments, and peptides, usually having less than 30 amino acids) comprise one or more amino acids coupled to each other via a covalent peptide bond (resulting in a chain of amino acids). The term "polypeptide(s)" as used herein describes a group of molecules, which, for example, consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a heteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is affected e.g. by post-translational modifications like glycosylation, acetylation, phosphorylation and the like. Such modifications are well known in the art.

### Vector:

Further provided herein is a vector, comprising one or more of the nucleic acids as described herein. A "vector" is a nucleic acid molecule used as a vehicle to transfer (foreign) genetic material into a host cell where it can for instance be replicated and/or expressed.

The term "vector" encompasses, without limitation plasmids, viral vectors (including retroviral vectors, lentiviral vectors, adenoviral vectors, vaccinia virus vectors, polyoma virus vectors, and adenovirus-associated vectors (AAV)), phages, phagemids, cosmids and artificial chromosomes (including BACs and YACs). The vector itself is generally a nucleotide sequence, commonly a DNA sequence that comprises an insert (transgene) and a larger sequence that serves as the "backbone" of the vector. Engineered vectors typically comprise an origin for autonomous replication in the host cells (if stable expression of the polynucleotide is desired), selection markers, and restriction enzyme cleavage sites (e.g. a multiple cloning site, MCS). Vector may additionally comprise promoters, genetic markers, reporter genes, targeting sequences, and/or protein purification tags. As known to those skilled in the art, large numbers of suitable vectors are known to those of skill in the art and many are commercially available. Examples of suitable vectors are provided in J. Sambrook et al., Molecular Cloning: A Laboratory Manual (4th edition), Cold Spring Harbor Laboratory, Cold Spring Harbor Laboratory Press, New York (2012).

The vector of the present invention can be a targeting vector. Targeting vectors can be used to integrate a polynucleotide into the host cell's chromosome by methods known in the art, such as described by J. Sambrook et al., Molecular Cloning: A Laboratory Manual (4th edition), Cold Spring Harbor Laboratory, Cold Spring Harbor Laboratory Press, New York (2012). Briefly, suitable means include homologous recombination or use of a hybrid recombinase that specifically targets sequences at the integration sites. Targeting vectors are typically circular and linearized before used for homologous recombination. As an alternative, the foreign polynucleotides may be DNA fragments joined by fusion PCR or synthetically constructed DNA fragments which are then recombined into the host cell. It is also possible to use heterologous recombination which results in random or non-targeted integration.

The vector of the present invention can also be an expression vector. "Expression vectors" or "expression constructs" can be used for the transcription of heterologous polynucleotide sequences, for instance those encoding the TCR of the invention, and translation of their mRNA in a suitable host cell.

Besides an origin of replication, selection markers, and restriction enzyme cleavage sites, expression vectors typically include one or more regulatory sequences operably linked to the heterologous polynucleotide to be expressed.

The term "regulatory sequence" refers to a nucleotide sequence necessary for the expression of an operably linked coding sequence of a (heterologous) polynucleotide in a particular host organism or host cell and thus include transcriptional and translational regulatory sequences. Typically, regulatory sequences required for expression of heterologous polynucleotide sequences in prokaryotes include a promoter(s), optionally operator sequence(s), and ribosome binding site(s). In eukaryotes, promoters, polyadenylation signals, enhancers and optionally splice signals are typically required. Moreover, specific initiation and secretory signals also may be introduced into the vector in order to allow for secretion of the polypeptide of interest into the culture medium.

Exemplary regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. As set out before, the expression vectors may also include origins of replication and selectable markers.

As mentioned previously, vectors of the invention may further comprise one or more selection markers. Suitable selection markers for use with eukaryotic host cells include, without limitation, the herpes simplex virus thymidine kinase (tk), hypoxanthine-guanine phosphoribosyltransferase (hgprt), and adenine phosphoribosyltransferase (aprt) genes. Other genes include dhfr (methotrexate resistance), gpt (mycophenolic acid resistance) neo (G-418 resistance) and hygro (hygromycin resistance). Vector amplification can be used to increase expression levels. In general, the selection marker gene can either be directly linked to the polynucleotide sequences to be expressed or introduced into the same host cell by co-transformation.

In view of the above, the present invention thus further provides one or more of the nucleotide sequences described herein inserted into (i.e. comprised by) a vector. Specifically, the invention provides (replicable) vectors comprising a nucleotide sequence encoding the TCR of the invention, or an alpha or beta chain thereof, or an alpha or beta variable region, or any CDR alpha or CDR beta operably linked to a promoter.

The skilled person will readily be able to select a suitable expression vector based on, e.g., the host cell intended for TCR expression. Examples for suitable expression vectors are viral vectors, such as retroviral vectors e.g. MP71 vectors or retroviral SIN vectors; and lentiviral vectors or lentiviral SIN vectors. Viral vectors comprising polynucleotides encoding the TCR of the invention are for instance capable of infecting lymphocytes, which are envisaged to subsequently express the heterologous TCR. Another example for a suitable expression vector is the Sleeping Beauty (SB) transposon transposase DNA plasmid system, SB DNA plasmid. The nucleic acids and/or in particular expression vectors of the invention can also be transferred into cells by transient RNA transfection.

Currently used viral vectors for native TCR expression typically link the TCR-alpha and TCR-beta chain genes in one vector with either an internal ribosomal entry site (IRES) sequence or the 2A peptide sequence derived from a porcine tsechovirus, resulting in the expression a single messenger RNA (mRNA) molecule under the control of the viral promoter within the transduced cell.

### A cell population comprising cells:

The term "A cell population comprising cells" as used herein may refer to a group and/or collection of individual cells of the same or similar type that share certain characteristics and/or properties within a specific biological context. Cell populations of the present invention can be defined based on various criteria, such as cell type (e.g., T cells, preferably CD8⁺ T cell(s)), developmental stage, location within a tissue or organ, functional characteristics (e.g., immune cells, preferably CD8⁺ T cell(s)) and/or genetic markers (e.g., expressing a TCR and a chimeric co-stimulatory receptor as defined herein). Preferably, a cell population comprising cells of the present invention is a population of immune cells (e.g., comprising T cells, preferably CD8⁺ T cell(s)), preferably a population of T cells (e.g., comprising T cells, preferably CD8⁺ T cell(s)). In particular, the cell population of the present invention may comprise a significant percentage of cells, such as 0.1 %, 0.2 %, 0.3 %, 0.4 %, 0.5 %, 1 %, 1.5 %,2 %, 3 %, 4 %, 4.5 % cells.

A cell population of the present invention as defined herein may comprise cells which secrete at least two (e.g. two, three, four or more) proteins. Alternatively, the cell as defined herein may secrete at least two (e.g. two, three, four or more) proteins. Preferably, such cell population as defined herein comprises cells which secrete at least two proteins which are selected from the group consisting of effector proteins (effector PSI; e.g. Gzm-B, IFN-γ, MIP-1α, TNF-α, TNF-β), stimulatory cytokines (stimulatory PSI; e.g. GM-CSF, IL-5, IL-9) and chemo-attractive cytokines (chemoattractive PSI; e.g. IP-10, MIP-1β). Further preferably, such cell as defined herein secretes at least two proteins which are selected from the group consisting of effector proteins (effector PSI; e.g. Gzm-B, IFN-γ, MIP-1α, TNF-α, TNF-β), stimulatory cytokines (stimulatory PSI; e.g. GM-CSF, IL-5, IL-9) and chemo-attractive cytokines (chemoattractive PSI; e.g. IP-10, MIP-1β).

### Cell:

The term "cell" as used herein may refer to a basic structural and functional unit of life in all living organisms. Preferred cells of the present invention are immune cells, preferably T cells. A cell (e.g. T cell) or a cell population (e.g. T-cell population) as defined herein co-expressing the TCR as defined herein and the chimeric costimulatory receptor such as PD1-41BB CSR as further defined herein thus shows a higher polyfunctionality (secretion of 2 or more proteins) compared to a cell or a cell population as defined herein expressing only the TCR of the invention, but not the chimeric costimulatory receptor (see **Figure 12**). T cells (or T lymphocytes) as defined herein are a type of white blood cells. There are several types of T cells including: helper T cells (CD4+ T cells), cytotoxic T cells (CD8⁺ T cells) and regulatory T cells (Tregs). A T cell(s) of the invention may be selected from the group consisting of: helper T cell(s) (CD4+ T cell(s)), cytotoxic T cell(s) (CD8⁺ T cell(s)) and regulatory T cell(s) (Tregs), preferably CD8⁺ T cell(s). Preferably, such cell as defined herein secretes at least two proteins which are selected from the group consisting of effector proteins (effector PSI), stimulatory cytokines (stimulatory PSI), chemo-attractive cytokines (chemoattractive PSI).

### Host cell:

The present invention further comprises a host cell comprising the TCR, the nucleic acid or the vector as described herein.

A variety of host cells can be used in accordance with the invention. As used herein, the term "host cell" encompasses cells which can be or has/have been recipients of polynucleotides or vectors described herein and/or express (and optionally secreting) the TCR of the present invention. The terms "cell" and "cell culture" are used interchangeably to denote the source of a TCR unless it is clearly specified otherwise. The term "host cell" also includes "host cell lines".

In general, the term "host cell" includes prokaryotic or eukaryotic cells, and also includes without limitation bacteria, yeast cells, fungi cells, plant cells, and animal cells such as insect cells and mammalian cells, e.g., murine, rat, macaque or human cells. The term "host cell" may also encompass any progeny of a parent cell that is not identical to the parent cell due to mutations that occur during replication.

In view of the above, the invention thus provides, *inter alia,* host cells comprising a polynucleotide or a vector, e.g. an expression vector comprising a nucleotide sequence encoding the TCR or a TCR construct as described herein.

Polynucleotides and/or vectors of the invention can be introduced into the host cells using routine methods known in the art, e.g. by transfection, transformation, or the like.

In view of the above, the present invention thus further provides host cells comprising at least one TCR, nucleic acid and/or vector as described herein.

For expression of the TCR of the invention, a host cell may be chosen that modulates the expression of the inserted nucleotide sequences, and/or modifies and processes the gene product (i.e. RNA and/or protein) as desired. Such modifications (e.g., glycosylation) and processing (e.g., cleavage) of gene products may be important for the function of the TCR. Different host cells have characteristic and specific mechanisms for the post-translational processing and modification of gene products. Appropriate cell lines or host systems can be chosen to ensure the correct modification and processing of the product. To this end, eukaryotic host cells that possess the cellular machinery for proper processing of the primary transcript, glycosylation, and phosphorylation of the gene product may be used.

It may be envisaged herein to provide (a) host cells for expressing and obtaining the TCR of the invention, in particular in soluble form ("production host cells") and (b) host cells expressing the TCR of the invention and having effector function ("effector host cells").

Such "effector host cells" are particularly useful for therapeutic applications and are envisaged for administration to a subject in need thereof. Preferred "effector host cells" include lymphocytes such as cytotoxic T lymphocytes (CTLs), CD8⁺ T cells, CD4⁺ T cells, natural killer (NK) cells, natural killer T (NKT) cells, gamma/delta-T-cells. Thus, the present invention may also comprise the host cell as defined herein, which is selected from lymphocytes including but not limited to cytotoxic T lymphocytes (CTLs), CD8⁺ T cells, CD4⁺ T cells, natural killer (NK) cells, natural killer T (NKT) cells, or gamma/ delta-T-cells.

Said effector host cells can be modified using routine methods to comprise a nucleotide sequence encoding the TCR of the invention, and are envisaged to express the TCR described herein, in particular on the cell surface. For the purposes of the present invention, "modified host cells expressing the TCR of the invention" generally refers to (effector or production) host cells treated or altered to express the TCR according to the present invention, for instance by RNA transfection. Other methods of modification or transfection or transduction may also be envisaged. The term "modified host cell" thus includes "transfected", "transduced" and "genetically engineered" host cells preferably expressing the TCR of the present invention.

Preferably, such "(modified) effector host cells" (in particular "(modified) effector lymphocytes") are capable of mediating effector functions through intracellular signal transduction upon binding of the TCR to its specific epitope. Such effector functions include for instance the release of perforin (which creates holes in the target cell membrane), granzymes (which are proteases that act intracellularly to trigger apoptosis), the expression of Fas ligand (which activates apoptosis in a Fas-bearing target cell) and the release of cytokines, preferably Th1/Tc1 cytokines such as IFN-γ, IL-2 and TNF-α. Thus, an effector host cell engineered to express the TCR of the invention that is capable recognizing and binding to its epitope in the subject to be treated is envisaged to carry out the above-mentioned effector functions, thereby killing the target (e.g. cancer) cells. Cytolysis of target cells can be assessed e.g. with the CTL fluorescent killing assay (CTL, USA) detecting the disappearance of fluorescently labeled target cells during co-culture with TCR-transfected recipient T cells.

In view of the above, effector host cells preferably express a functional TCR, i.e. that typically comprises a TCR alpha and beta chain described herein; and also the signal transducing subunits CD3 gamma, delta, epsilon and zeta (CD3 complex). Moreover, expression of co-receptors CD4 or CD8 may also be desired. Generally, lymphocytes harboring the required genes involved in antigen binding, receptor activation and downstream signalling (e.g. Lck, FYN, CD45, and/or Zap70), T cells are particularly suitable as effector host cells. However, effector host cells expressing the TCR of the invention as a "binding domain" without the CD3 signal transducing subunit and/or aforementioned downstream signalling molecules (i.e. being capable of recognizing the antigenic target described herein, but without effecting functions mediated by CD3 and/or the aforementioned downstream signalling molecules) are also envisaged herein. Such effector cells are envisaged to be capable of recognizing the epitope described herein, and optionally of effecting other functions not associated with CD3 signalling and/or signalling of the aforementioned downstream signalling molecules. Examples include NK or NKT cells expressing the TCR and being capable of e.g. releasing cytotoxic granules upon recognition of their antigenic target.

Effector host cells in particular lymphocytes such as T cells can be autologous host cells that are obtained from the subject to be treated and transformed or transduced to express the TCR of the invention. Techniques for obtaining and isolating the cells from the patient are known in the art.

"Production host cells" used for the expression of the TCR of the invention are preferably capable of expressing high amounts of recombinant protein. Exemplary mammalian host cells that can be used for as "production host cells" include Chinese Hamster Ovary (CHO cells) including DHFR minus CHO cells such as DG44 and DUXBI 1, NSO, COS (a derivative of CVI with SV40 T antigen), HEK293 (human kidney), and SP2 (mouse myeloma) cells. Other exemplary host cell lines include, but are not limited to, HELA (human cervical carcinoma), CVI (monkey kidney line), VERY, BHK (baby hamster kidney), MOCK, 293, WI38, R1610 (Chinese hamster fibroblast) BALBC/3T3 (mouse fibroblast), HAK (hamster kidney line), P3x63-Ag3.653 (mouse myeloma), BFA-lcIBPT (bovine endothelial cells), and RAJI (human lymphocyte). Host cell lines are typically available from commercial services, the American Tissue Culture Collection (ATCC) or from published literature.

Non-mammalian cells such as bacterial, yeast, insect or plant cells are also readily available and can also be used as "production host cells" as described above. Exemplary bacterial host cells include enterobacteriaceae, such Escherichia coli, Salmonella; Bacillaceae, such as Bacillus subtilis; Pneumococcus; Streptococcus, and Haemophilus influenza. Other host cells include yeast cells, such as Saccharomyces cerevisiae, and Pichia pastoris. Insect cells include, without limitation, Spodoptera frugiperda cells.

In accordance with the foregoing, conceivable expressions systems (i.e. host cells comprising an expression vector as described above) include microorganisms such as bacteria (e.g., E. coli, B. subtilis) transformed with recombinant bacteriophage DNA, plasmid DNA or cosmid DNA expression vectors; yeast (e.g., Saccharomyces , Pichia) transformed with recombinant yeast expression vectors; insect cell systems infected with recombinant virus expression vectors (e.g., baculovirus); plant cell systems infected with recombinant virus expression vectors (e.g., cauliflower mosaic virus, CaMV; tobacco mosaic virus, TMV) or transformed with recombinant plasmid expression vectors (e.g., Ti plasmid). Mammalian expression systems harboring recombinant expression constructs containing promoters derived from the genome of mammalian cells (e.g., metallothionein promoter) or from mammalian viruses (e.g., the adenovirus late promoter; the vaccinia virus 7.5K promoter, the cytomegalovirus (CMV) major immediate-early promoter (MIEP) promoter) are often preferred. Suitable mammalian host cells can be selected from known cell lines (e.g., COS, CHO, BLK, 293, 3T3 cells), however it is also conceivable to use lymphocytes such as cytotoxic T lymphocytes (CTLs), CD8⁺ T cells, CD4⁺ T cells, natural killer (NK) cells, natural killer T (NKT) cells, gamma/ delta-T-cells.

### Method for obtaining the TCR:

In accordance with the foregoing, the present invention also comprises a method for producing or obtaining the TCR as described herein comprising the steps of (i) incubating the host cell (i.e., a production host cell) under conditions causing expression of said TCR and (ii) purifying said TCR.

The host cells harboring the expression vector are grown under conditions appropriate to the production of the TCR defined herein, in particular alpha chains and/or beta chains as described elsewhere herein, and assayed for alpha and/or beta chain protein synthesis. For the expression of double-chained TCRs, vectors encoding both the alpha and beta chains may be co- expressed in the host cell for expression of the entire molecule.

Once a TCR of the invention has been expressed, it may be purified by any purification method known in the art, for example, by chromatography (e.g., ion exchange chromatography (e.g. hydroxylapatite chromatography), affinity chromatography, particularly Protein A, Protein G or lectin affinity chromatography, sizing column chromatography), centrifugation, differential solubility, hydrophobic interaction chromatography, or by any other standard technique for the purification of proteins. The skilled person will readily be able to select a suitable purification method based on the individual characteristics of the TCR to be recovered.

Any definitions regarding the TCR, the nucleic acid, the vector, or the host cell can be applied for the production method as defined herein, where necessary.

### Pharmaceutical or diagnostic composition:

The present invention further comprises a pharmaceutical composition comprising one or more active agents, namely the TCR, the nucleic acid, the vector and/or the host cell as described herein, and, optionally, one or more pharmaceutically excipient(s). Accordingly, the use of said TCR, nucleic acid, vector and/or host cell for the manufacture of a pharmaceutical composition or medicament is also envisaged herein.

The term "pharmaceutical composition" particularly refers to a composition suitable for administering to a subject, preferably to a mammal, most preferably to a human. However, compositions suitable for administration to non-human mammal are generally also encompassed by the term.

The pharmaceutical composition and its components (i.e. active agents and optionally excipients) are preferably pharmaceutically acceptable, i.e. capable of eliciting the desired therapeutic effect without causing any undesirable local or systemic effects in the recipient. Pharmaceutically acceptable compositions of the invention may for instance be sterile. Specifically, the term "pharmaceutically acceptable" may mean approved by a regulatory agency or other generally recognized pharmacopoeia for use in animals, and more particularly in humans.

The active agent described in the foregoing (for instance the host cell or the TCR) is preferably present in the pharmaceutical composition in a therapeutically effective amount. By "therapeutically effective amount" is meant an amount of the active agent that elicits the desired therapeutic effect. Therapeutic efficacy and toxicity can be determined by standard procedures, e.g. in cell culture or in test animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, ED50/LD50. Pharmaceutical compositions that exhibit large therapeutic indices are preferred.

The exact dosage of the TCR, nucleic acid, vector and/or host cell will be ascertainable by one skilled in the art using known techniques. Suitable dosages provide sufficient amounts of the active agent of the invention and are preferably therapeutically effective, i.e. elicit the desired therapeutic effect.

As is known in the art, adjustments for purpose of the treatment (e.g. remission maintenance vs. acute flare of disease), route, time and frequency of administration, time and frequency of administration formulation, age, body weight, general health, sex, diet, severity of the disease state, drug combination(s), reaction sensitivities, and tolerance/response to therapy may be necessary. Suitable dosage ranges, for instance for the soluble TCR as described herein, can be determined using data obtained from cell culture assays and animal studies and may include the ED50. Typically, dosage amounts may vary from 0.1 to 100000 micrograms, up to a total dose of about 2 g, depending upon the route of administration. Exemplary dosages of the active agent of the invention may be in the range from about 0.01 mg/kg to about 10 mg/kg, from about 0.1 mg/kg to about 10 mg/kg, from about 1 mg/kg to about 10 mg/kg, from about 1 mg/kg to about 5 mg/kg, from about 0.01 mg/kg to about 1 mg/kg, or from about 0.1 mg/kg to about 1 mg/kg. Guidance as to particular dosages and methods of delivery is provided in the literature. It is recognized that treatment may require a single administration of a therapeutically effective dose or multiple administrations of a therapeutically effective dose of the active agent of the invention.

As set out previously, the pharmaceutical composition may optionally comprise one or more excipients and/or additional active agents.

The term "excipient" includes fillers, binders, disintegrants, coatings, sorbents, antiadherents, glidants, preservatives, antioxidants, flavoring, coloring, sweeting agents, solvents, co-solvents, buffering agents, chelating agents, viscosity imparting agents, surface active agents, diluents, humectants, carriers, diluents, preservatives, emulsifiers, stabilizers and tonicity modifiers. It is within the knowledge of the skilled person to select suitable excipients for preparing the desired pharmaceutical composition of the invention. Exemplary carriers for use in the pharmaceutical composition of the invention include saline, buffered saline, dextrose, and water. Typically, choice of suitable excipients will *inter alia* depend on the active agent used, the disease to be treated, and the desired formulation of the pharmaceutical composition.

The present invention further provides pharmaceutical compositions comprising one or more of the active agents specified above (for instance a host cell or the TCR of the invention), and one or more additional active agents that are suitable for treatment and/or prophylaxis of the disease to be treated. Preferred examples of additional active agents suitable for combinations include known anti-cancer drugs such as cis-platin, maytansine derivatives, rachelmycin, calicheamicin, docetaxel, etoposide, gemcitabine, ifosfamide, irinotecan, melphalan, mitoxantrone, sorfimer sodiumphotofrin II, temozolmide, topotecan, trimetreate glucuronate, auristatin E vincristine and doxorubicin; and peptide cytotoxins such as ricin, diphtheria toxin, pseudomonas bacterial exotoxin A, DNAase and RNAase; radionuclides such as iodine 131, rhenium 186, indium 111, yttrium 90, bismuth 210 and 213, actinium 225 and astatine 213; prodrugs, such as antibody directed enzyme pro-drugs; immuno-stimulants, such as IL-2, chemokines such as IL-8, plateletfactor4, melanoma growth stimulatory protein, etc., antibodies or fragments thereof such as anti-CD3 antibodies or fragments thereof, complement activators, xenogeneic protein domains, allogeneic protein domains, viral/bacterial protein domains and viral/bacterial peptides.

A variety of routes are applicable for administration of the pharmaceutical composition according to the present invention. Typically, administration will be accomplished parenterally. Methods of parenteral delivery include topical, intra-arterial, intramuscular, subcutaneous, intramedullary, intrathecal, intraventricular, intravenous, intraperitoneal, intrauterine, intravaginal, sublingual or intranasal administration.

The pharmaceutical composition of the invention can be formulated in various forms, depending *inter alia* on the active agent used (e.g., the TCR), e.g. in solid, liquid, gaseous or lyophilized form and may be, *inter alia,* in the form of an ointment, a cream, transdermal patches, a gel, powder, a tablet, solution, an aerosol, granules, pills, suspensions, emulsions, capsules, syrups, liquids, elixirs, extracts, tincture or fluid extracts or in a form which is particularly suitable for the desired method of administration. Processes known per se for producing medicaments are indicated in 22nd edition of Remington's Pharmaceutical Sciences (Ed. Maack Publishing Co, Easton, Pa., 2012) and may include, for instance conventional mixing, dissolving, granulating, dragee-making, levigating, emulsifying, encapsulating, entrapping or lyophilizing processes. A pharmaceutical composition comprising, for instance, host cells or the TCR as described herein will typically be provided in a liquid form, and preferably comprise a pharmaceutically acceptable buffer.

After a pharmaceutical composition of the invention has been prepared it can be placed in an appropriate container and labeled for treatment of an indicated condition. Such labeling would for instance include amount, frequency and method of administration.

The present invention further comprises a diagnostic composition comprising one or more diagnostic agents, namely the TCR, the nucleic acid, the vector and/or the host cell as described herein, and optionally, one or more diagnostically acceptable excipient(s). Typically, said diagnostic agent will comprise means for detecting its binding to its epitope, for instance a label as described in the context of the TCR of the invention. As regards the host cell, it is for instance conceivable to use modified host cells comprising a dye or a contrast agent that is released (instead of cytotoxic granules) upon antigen recognition.

The term "diagnostic composition" when used herein refers to a composition comprising one or more diagnostic agents of the present invention as defined herein, which can be applied for use in diagnosis *in vitro.*

Said diagnostically acceptable excipient includes also any excipient that does not itself elicit an adverse reaction, which would be harmful when used in *in vitro* and *in vivo* diagnosis. Suitable excipients are typically large, slowly metabolized macromolecules such as proteins, polysaccharides, polylactic acids, polyglycolic acids, polymeric amino acids, amino acid copolymers and lipid aggregates such as, e.g. oil droplets or liposomes. The excipient used in combination with the one or more diagnostic agents of the present invention may be water-based and forms an aqueous solution. An oil-based excipient solution containing the one or more diagnostic agents of the present invention is an alternative to the aqueous carrier solution. Either aqueous or oil-based solutions further contain thickening agents to provide the composition with the viscosity of a liniment, cream, ointment, gel, or the like. Suitable thickening agents are well known to those skilled in the art.

Diagnostically acceptable excipients according to the present invention include, by the way of illustration and not limitation, diluent, disintegrants, binding agents, adhesives, wetting agents, polymers, lubricants, gliands, substances added to mask or counteract a disagreeable texture, taste or odor, flavors, dyes, fragrances, and substances added to improve appearance of the composition. The diagnostic composition can additionally include preservatives, solubilizers, stabilizers, wetting agents, emulsifiers, sweeteners, colorants, flavorings, buffers, coating agents, surfactants or antioxidants.

Any definitions regarding the TCR, the nucleic acid, the vector, or the host cell can be applied for the compositions as defined herein, where necessary.

### Treatment:

Additionally, the present invention also comprises the TCR, nucleic acid, vector, host cell and/or the pharmaceutical composition as described herein for use as a medicament and/or for use in therapy.

The TCR, nucleic acid, vector, host cell and/or pharmaceutical composition can in general be employed for treatment detection, diagnosis, prognosis, prevention and/or treatment of diseases or disorders. The term "treatment" in all its grammatical forms includes therapeutic or prophylactic treatment of a subject in need thereof. A "therapeutic or prophylactic treatment" comprises prophylactic treatments aimed at the complete prevention of clinical and/or pathological manifestations or therapeutic treatment aimed at amelioration or remission of clinical and/or pathological manifestations. The term "treatment" thus also includes the amelioration or prevention of diseases.

The terms "subject" or "patient" are used interchangeably herein to refer to any subject, particularly a mammal, for whom therapy is desired. Mammalian subjects generally include humans, non-human primates, dogs, cats, guinea pigs, rabbits, rats, mice, horses, cattle, cows, and the like. However, it will readily be understood that the TCR, nucleic acid, vector, host cell and/or pharmaceutical composition provided herein are especially envisaged for treatment of human subjects.

For therapy, the TCR of the invention, the nucleic acid, the vector (such as viral vectors), the host cell and/or the pharmaceutical composition of the invention can be administered directly to the subject in need thereof. Thus, the present invention also comprises the TCR, the nucleic acid, the vector, the host cell, and/or the pharmaceutical composition as defined herein for use in a method of detecting, diagnosing, prognosing, preventing and/or treating cancer. The invention may also comprise the TCR, the nucleic acid, the vector, the host cell and/or the pharmaceutical composition for the use as defined herein, wherein prevention and/or treatment of cancer comprises administering at least any one of the following: the TCR, the nucleic acid, the vector, the host cell, or the pharmaceutical composition to a subject in need thereof. Said method can also comprise the steps of (a') providing one or more of the TCR, the nucleic acid, the vector, the host cell, and/or the pharmaceutical composition of the present invention; and (b') administering one or more of the abovementioned to the subject in need thereof. Optionally, the method can comprise a further step of cancer therapy, e.g. radiation, or administration of one or more anti-cancer agents.

### Ex vivo treatment:

The present invention may also comprise the TCR, the nucleic acid, the vector, the host cell and/or the pharmaceutical composition for the use as defined herein, wherein prevention and/or treatment of cancer comprises (a) introducing at least any one of the following: the TCR, the nucleic acid, the vector, the host cell or the pharmaceutical composition into lymphocytes comprised by a sample obtained from a subject as defined herein, thereby obtaining modified lymphocytes, and (b) administering the modified lymphocytes of step (a) to a subject in need thereof.

The treatment according to the invention may also comprise the steps of (a') providing a sample of a subject, said sample comprising lymphocytes; (b') providing one or more of the TCR, nucleic acid, vector, host cell and/or pharmaceutical composition of the invention; (c') introducing of one or more of the abovementioned of step (b') into the lymphocytes of step (a') and, thereby, obtaining modified lymphocytes; (d') administering the modified lymphocytes of step (c') to a subject in need thereof.

The lymphocytes provided in step (a') are particularly envisaged to be "effector host cells" as described in the foregoing and are advantageously selected from T cells, NK cells and/or NKT cells, especially CD8⁺ T cells; and can be obtained in a previous step (a") from a sample -in particular a blood sample- of the subject by routine methods known in the art. It is however also conceivable to use other lymphocytes that are preferably capable of expressing the TCR of the present invention and exert the desired biological effector functions as described herein. Moreover, said lymphocytes will typically be selected for compatibility with the subject's immune system, i.e. they will preferably not elicit an immunogenic response. For instance, it is conceivable to use a "Universal Recipient Cells", i.e. universally compatible lymphocytes exerting the desired biological effector functions that can be grown and expanded *in vitro.* Use of such cells will thus obviate the need for obtaining and providing the subject's own lymphocytes in step (a').

The *ex vivo* introduction of optional step (c') can be carried out by introducing a nucleic acid or vector described herein via electroporation into the lymphocytes, or by infecting the lymphocytes with a viral vector, such as a lentiviral or retroviral vector as described previously in the context of the effector host cell. Other conceivable methods include the use of by transfection reagents, such as liposomes, or transient RNA transfection. The transfer of antigen-specific TCR genes into (primary) T cells by e.g. (retro-)viral vectors or transient RNA transfection represents a promising tool for generating tumor-associated antigen-specific T cells that can subsequently be re-introduced into the donor, where they specifically target and destroy tumor cells expressing said antigen, such as the epitope as defined herein.

Any definitions regarding the TCR, the nucleic acid, the vector, the host cell or the pharmaceutical composition can be applied for the treatments as defined herein, where necessary.

### Use:

The present invention envisages use of the diagnostic agents described in the foregoing for detecting, diagnosing and/or prognosing cancer in a subject which can be accomplished *in vivo* or *in vitro.*

Thus, the invention provides a diagnostic composition for use in detecting, diagnosing and/or prognosing cancer in a subject *in vivo*, said composition comprising, as a diagnostic agent, the TCR, the nucleic acid, the vector, the host cell and/or the pharmaceutical composition of the invention. The method will typically comprise (a) administering said diagnostic agent to the subject and (b) detecting binding of said diagnostic agent to its antigenic target.

Moreover, the invention provides a method of detecting, diagnosing and/or prognosing cancer in a subject *in vitro.* In detail, the present invention comprises a method of detecting the presence of a cancer in a subject *in vitro,* comprising (a) contacting a sample obtained from a subject and comprising one or more cells with the TCR, the nucleic acid, the vector, the host cell, and/or the pharmaceutical composition, thereby forming a complex, and (b) detecting the complex, wherein detection of the complex is indicative of the presence of the cancer in the subject. In a preferred embodiment of such method the TCR as defined elsewhere herein comprises a tag as defined herein.

The present invention may also encompass a method of detecting the presence of a cancer in a subject *in vitro,* comprising the steps of (a') providing a sample of a subject, said sample comprising one or more cells; (b') contacting said sample with the TCR, the nucleic acid, the vector, the host cell, and/or the pharmaceutical composition of the invention; thereby forming a complex, and (c') detecting the complex. Said complex is envisaged to be indicative for binding of the diagnostic agent as defined herein to its antigenic target and is of the presence of a (cancer) cell expressing said antigenic target.

In both methods binding of the diagnostic agent to its antigenic target (epitope as defined herein) is detectable by using routine methods known in the art and will *inter alia* depend on the specific diagnostic agent used. Suitable labels that can be coupled to the diagnostic agent of the invention are exemplified in the section relating to labeled TCR constructs.

The present invention also comprises the use of the TCR, the nucleic acid, and/or the vector as described elsewhere herein for generating modified lymphocytes as defined herein. Means and methods for introducing, e.g. a nucleic acid and a vector into the lymphocytes have been described elsewhere herein.

Any definitions regarding the TCR, the nucleic acid, the vector, the host cell or the pharmaceutical composition can be applied for the uses, where necessary.

### Kit:

The present invention also comprises a kit comprising the TCR, the nucleic acid, the vector, the host cell, and/or the pharmaceutical or diagnostic composition as defined herein. Thus, when a kit comprises the TCR per se, said TCR may be provided in a vial or a container. The same applies for the other components, such as the nucleic acid, the vector, the host cell, and/or the pharmaceutical or diagnostic composition.

Further it may be associated with a notice in the form prescribed by a governmental agency regulating the manufacture, use or sale of pharmaceuticals or biological products, reflecting approval by the agency of the manufacture, use or sale of the product for human administration or diagnostics.

Said kit may comprise the component as described above, preferably in a vial or container, in dried form, such as a lyophilized, air-dried, or spray-dried form (in form of a powder), for later reconstitution into a liquid form or other form prior to use. Further, said kit may also comprise the component, preferably in a vial or container, in a frozen state, being thawed prior to use.

According to the present invention, the kit comprising the component may further comprise a diagnostically or pharmaceutically acceptable excipient as defined elsewhere herein. In some embodiments, said excipient may also be comprised in one or more containers or vials in said kit, meaning said kit additionally comprising either one vial or container comprising said excipient as a mixture or said kit additionally comprising for each component such as the excipient separate vials or containers.

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments described throughout the specification should not be construed to limit the present invention to only the explicitly described embodiments. This description should be understood to support and encompass embodiments which combine the explicitly described embodiments with any number of the disclosed and/or preferred elements. Furthermore, any permutations and combinations of all elements described herein should be considered disclosed by the description of the present application unless the context indicates otherwise.

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. When used herein the term "comprising" can be substituted with the term "containing" or "including" or sometimes when used herein with the term "having". When used herein "consisting of" excludes any element, step, or ingredient not specified.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Unless otherwise indicated, the term "at least" preceding a series of elements is to be understood to refer to every element in the series. The term "at least one" refers to one, two, three or more such as four, five, six, seven, eight, nine, ten and more. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

When used herein "consisting of" excludes any element, step, or ingredient not specified in the claim element. When used herein, "consisting essentially of" does not exclude materials or steps that do not materially affect the basic and novel characteristics of the claim.

The term "including" means "including but not limited to". "including" and "including but not limited to" are used interchangeably.

The term "about" means plus or minus 20%, preferably plus or minus 10%, more preferably plus or minus 5%, most preferably plus or minus 1%.

Throughout the description and claims of this specification, the singular encompasses the plural unless the context otherwise requires. In particular, where the indefinite article is used, the specification is to be understood as contemplating plurality as well as singularity, unless the context requires otherwise.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

The present invention further relates to the following items:
1. A T-cell receptor (TCR) (e.g., an isolated and/or recombinant TCR) comprising:
   i) an α chain Complementarity Determining Region 3 (CDR3) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 16 (CAVSRSGNTPLVF); and/or
   ii) a β chain Complementarity Determining Region 3 (CDR3) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 4 (CASSGAGETGELFF).
2. The TCR of item 1, wherein the TCR further comprises at least one (e.g., 1, 2, 3 or 4, preferably 4) of the following:
   i) an α chain Complementarity Determining Region 1 (CDR 1) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 14 (SSVSVY);
   ii) an α chain Complementarity Determining Region 2 (CDR2) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 15 (YLSGSTLV);
   iii) a β chain CDR1 comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 2 (WNHNN); and/or
   iv) a β chain CDR2 comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 3 (SYGVHD).
3. The TCR of any one of the preceding items, having antigenic specificity to an epitope presented in the context of a MHC class I molecule, the epitope comprising the amino acid sequence of VVVGADGVGK (SEQ ID NO: 1), or a fragment thereof (e.g., SEQ ID NO: 35, VVGADGVGK) or a variant thereof comprising one or more (e.g. one or two) conservative amino acid substitutions (preferably corresponding to position 1, 2, 3, 4, 5, 6, 7, 8, 9 and/or 10 of SEQ ID NO: 1).
4. The TCR of any one of the preceding items, wherein the TCR has antigenic specificity for said epitope presented in the context of an HLA-A molecule.
5. The TCR of any one of the preceding items, wherein the TCR has antigenic specificity for said epitope presented in the context of (e.g., as shown in the Examples herein):
   i) an HLA-A molecule, preferably HLA-A*11 molecule;
   ii) an HLA-A*11:01 molecule; and/or
   iii) at least any one of an HLA-A*11 molecule, preferably HLA-A*11:01, HLA-A*11:02, HLA-A*11:03 and/or HLA-A*11:12.
6. The TCR of any one of the preceding items, wherein the at least 80% (e.g. at least 81%, 82%, 83%, 84%, 85%, 86%, 87%, 88%, 89%, 90%, 91%, 92%, 93%, 94%, 95%, 96%, 97%, 98%, 99% or 100%) identity comprises conservative and/or highly conservative amino acid substitutions, preferably corresponding to position(s) 1, 2, 3, 4, 5 and/or 6 of SEQ ID NO: 14; position(s) 1, 2, 3, 4, 5, 6, 7 and/or 8 of SEQ ID NO: 15; position(s) 1, 2, 3, 4, 5, 6, 7, 8, 9, 10, 11, 12 and/or 13 of SEQ ID NO: 16; position(s) 1, 2, 3, 4 and/or 5 of SEQ ID NO: 2; position(s) 1, 2, 3, 4, 5 and/or 6 of SEQ ID NO: 3; and/or position(s) 1, 2, 3, 4, 5, 6, 7, 8, 9, 10, 11, 12, 13 and/or 14 of SEQ ID NO: 4.
7. The TCR of any one of the preceding items, comprising (or consisting of) at least one (e.g., 1, 2, 3, 4, 5, or 6, preferably 6) of the following:
   i) an α chain CDR 1 comprising the amino acid sequence of SEQ ID NO: 14;
   ii) an α chain CDR2 comprising the amino acid sequence of SEQ ID NO: 15;
   iii) an α chain CDR3 comprising the amino acid sequence of SEQ ID NO: 16;
   iv) a β chain CDR1 comprising the amino acid sequence of SEQ ID NO: 2;
   v) a β chain CDR2 comprising the amino acid sequence of SEQ ID NO: 3; and/or
   vi) a β chain CDR3 comprising the amino acid sequence of SEQ ID NO: 4.
8. The TCR of any one of the preceding items, comprising:
   i) an α chain variable region comprising the amino acid sequence of SEQ ID NO: 20, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 20; and/or
   ii) a β chain variable region comprising the amino acid sequence of SEQ ID NO: 8, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 8.
9. The TCR of any one of the preceding items, further comprising:
   i) an α chain constant region comprising the amino acid sequence of SEQ ID NO: 22, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 22; and/or
   ii) a β chain constant region comprising the amino acid sequence of SEQ ID NO: 10, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 10.
10. The TCR of any one of the preceding items, comprising:
   i) an α chain comprising the amino acid sequence of SEQ ID NO: 24, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 24; and/or
   ii) a β chain comprising the amino acid sequence of SEQ ID NO: 12, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 12.
11. The TCR of any one of the preceding items, wherein the TCR has said antigenic specificity for said epitope in the absence of the CD8 co-receptor.
12. The TCR of any one of the preceding items, wherein the TCR is selected from the group consisting of a native TCR, a TCR variant, a TCR fragment, and a TCR construct.
13. The TCR of any one of the preceding items, wherein the TCR construct comprises at least one TCR alpha-chain(s) and at least one TCR beta-chain(s) covalently linked to each other to form TCR heterodimers or multimers.
14. The TCR of any one of the preceding items, further comprising one or more fusion component(s) selected from the group consisting of: any suitable segment or fragment of a protein or polypeptide, preferably a peptide comprising at least six or at least nine amino acids, e.g., one or more of any one of SEQ ID NOs: 41-44) recognized by the immune system, e.g., by T cells via their TCRs and/or by antibodies, further preferably said segment or fragment comprises an uniTope 9-mer or uniTope 6-mer of one or more of any one of SEQ ID NOs: 41-44 (e.g., according to one or more of any one of SEQ ID NOs: 38, 45 or 46); a Fc receptor; a Fc domain, including IgA, IgD, IgG, IgE, and IgM; a cytokine, including IL-2 or IL-15; a toxin; an antibody or an antigen-binding fragment thereof, including anti-CD3, anti-CD28, anti-CD5, anti-CD 16 or an anti- CD56 antibody or an antigen-binding fragment thereof; and a CD247 (CD3-zeta), CD28, CD137, or a CD134 domain or combinations thereof, optionally further comprising at least one linker.
15. The TCR of any one of the preceding items, further comprising at least one label or at least one epitope-tag, preferably said at least one label or at least one epitope-tag (e.g., wherein said epitope-tag is not immunogenic and/or detectable, for example, by an antibody) comprising one or more of any one of: SEQ ID NOs: 41-44, optionally according to one or more of any one of: SEQ ID NOs: 38, 45 or 46.
16. The TCR of any one of the preceding items, which is soluble.
17. A nucleic acid comprising a nucleotide sequence encoding the TCR of any one of the preceding items (or fragment thereof, e.g., as depicted in Table 2).
18. The nucleic acid of any one of the preceding items, comprising the nucleotide sequence of
   i) SEQ ID NO: 9 and/or SEQ ID NO: 21;
   ii) SEQ ID NO: 11 and/or SEQ ID NO: 23; and/or
   iii) SEQ ID NO: 13 and/or SEQ ID NO: 25.
19. A vector comprising the nucleic acid of any one of the preceding items, preferably said vector is an expression vector or viral vector.
20. A host cell (e.g., isolated and/or recombinant host cell) comprising the TCR of any one of the preceding items, the nucleic acid of any one of the preceding items, or the vector of any one of the preceding items, preferably said host cell is selected from the group consisting of: a prokaryotic host cell (e.g., bacterial), fungal host cell, mammalian host cell, non-human mammalian host cell.
21. The host cell of any one of the preceding items, which is selected from lymphocytes including but not limited to cytotoxic T lymphocytes (CTLs), CD8⁺ T cells, CD4⁺ T cells, natural killer (NK) cells, natural killer T (NKT) cells, and gamma/ delta-T-cells.
22. A method for obtaining the TCR of any one of the preceding items, comprising
   i) incubating the host cell of any one of the preceding items under conditions causing expression of said TCR; and
   ii) purifying said TCR.
23. A composition, preferably a pharmaceutical and/or diagnostic composition, comprising one or more of the following:
   i) the TCR of any one of the preceding items;
   ii) the nucleic acid of any one of the preceding items;
   iii) the vector of any one of the preceding items; and/or
   iv) the host cell of any one of the preceding items; and,
   v) optionally, pharmaceutically excipient(s).
24. The TCR of any one of the preceding items, the nucleic acid of any one of the preceding items, the vector of any one of the preceding items, the host cell of any one of the preceding items and/or the pharmaceutical composition of any one of the preceding items, for use as a medicament and/or for use in therapy.
25. The TCR of any one of any one of the preceding items, the nucleic acid of any one of the preceding items, the vector of any one of the preceding items, the host cell of any one of the preceding items and/or the pharmaceutical composition of any one of the preceding items, for use in a method of detecting, diagnosing, prognosing, preventing and/or treating cancer.
26. The TCR, the nucleic acid, the vector, the host cell and/or the pharmaceutical composition for the use of any one of the preceding items, wherein prevention and/or treatment of cancer comprises:
   administering at least any one of the following (i) to (v):
      i) the TCR of any one of the preceding items;
      ii) the nucleic acid of any one of the preceding items;
      iii) the vector of any one of the preceding items;
      iv) the host cell of any one of the preceding items; and/or
      v) the pharmaceutical composition of any one of the preceding items;
   to a subject in need thereof.
27. The TCR, the nucleic acid, the vector, the host cell and/or the pharmaceutical composition for the use of any one of the preceding items, wherein prevention and/or treatment of cancer comprises:
   (a) introducing at least any one of the following (i) to (v):
      i) the TCR of any one of the preceding items;
      ii) the nucleic acid of any one of the preceding items;
      iii) the vector of any one of the preceding items;
      iv) the host cell of any one of the preceding items; and/or
      v) the pharmaceutical composition of any one of the preceding items;
      into lymphocytes comprised by a sample obtained from a subject, thereby obtaining modified lymphocytes, and
   (b) administering the modified lymphocytes of step (a) to a subject in need thereof.
28. A method of detecting the presence of a cancer in a subject *in vitro,* comprising:
   (a) contacting a sample obtained from a subject and comprising one or more cells with
      i) the TCR of any one of the preceding items;
      ii) the nucleic acid of any one of the preceding items;
      iii) the vector of any one of the preceding items;
      iv) the host cell of any one of the preceding items, and/or
      v) the pharmaceutical composition of any one of the preceding items;
      thereby forming a complex, and
   (b) detecting the complex, wherein detection of the complex is indicative of the presence of the cancer in the subject.
29. Use of the TCR of any one of the preceding items, the nucleic acid of any one of the preceding items and/or the vector of any one of the preceding items for generating modified lymphocytes.
30. A kit comprising the TCR of any one of the preceding items, the nucleic acid of any one of the preceding items, the vector of any one of the preceding items, the host cell of any one of the preceding items, and/or the pharmaceutical or diagnostic composition of any one of the preceding items.
31. The TCR, the nucleic acid, the vector, the host cell, the pharmaceutical and/or diagnostic composition, method, use or kit according to any one of the preceding items, carried out as shown in Examples (e.g., Figures and/or Table depicted herein) and/or herein below.
32. A T-cell receptor (TCR) polypeptide complex and/or a combination comprising:
   a) a TCR, preferably a TCR of any one of the preceding items; and
   b) a chimeric co-stimulatory receptor, preferably said chimeric co-stimulatory receptor is a fusion polypeptide comprising one or more (e.g., two) PD-1-derived polypeptide (e.g., wherein said PD-1 is also known as Programmed Cell Death Protein 1, e.g., having UniProtAccession Number: Q15116) and 4-1BB-derived polypeptide (e.g., wherein said 4-1BB is also known as CD137 or TNFRS9, which can be used interchangeably, e.g., having UniProt Accession Number: Q07011); further preferably said chimeric co-stimulatory receptor is a fusion polypeptide comprising a CD40L-derived polypeptide and a CD28-derived polypeptide;
   wherein the TCR comprises:
   i) an α chain Complementarity Determining Region 3 (CDR3) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 16; and/or
   ii) a β chain Complementarity Determining Region 3 (CDR3) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 4.
33. A cell population (preferably a T-cell population) comprising cells (e.g., isolated and/or recombinant cells, e.g., T-cells) or a cell (e.g., isolated and/or single and/or recombinant cell, e.g., T-cell) expressing:
   a) a TCR, preferably a TCR of any one of the preceding items; and
   b) a chimeric co-stimulatory receptor, preferably said chimeric co-stimulatory receptor is a fusion polypeptide comprising one or more (e.g., two) PD-1-derived polypeptide (e.g., wherein said PD-1 is also known as Programmed Cell Death Protein 1, e.g., having UniProtAccession Number: Q15116) and 4-1BB-derived polypeptide (e.g., wherein said 4-1BB is also known as CD137 or TNFRS9, which can be used interchangeably, e.g., having UniProt Accession Number: Q07011); further preferably said chimeric co-stimulatory receptor is a fusion polypeptide comprising a CD40L-derived polypeptide and a CD28-derived polypeptide;
   wherein the TCR comprises:
   i) an α chain Complementarity Determining Region 3 (CDR3) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 16; and/or
   ii) a β chain Complementarity Determining Region 3 (CDR3) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 4.
34. The complex and/or the combination of any one of the preceding items and/or the cell population or the cell of any one of the preceding items, wherein the TCR further comprises at least one (e.g., 1, 2, 3, or 4) of the following:
   i) an α chain Complementarity Determining Region 1 (CDR 1) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 14;
   ii) an α chain Complementarity Determining Region 2 (CDR2) comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 15;
   iii) a β chain CDR1 comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 2; and/or
   iv) a β chain CDR2 comprising an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 3.
35. The complex and/or the combination, and/or the cell population or the cell of any one of the preceding items, wherein the TCR has antigenic specificity to an epitope presented in the context of a MHC class I molecule, the epitope comprising the amino acid sequence of VVVGADGVGK (SEQ ID NO: 1), or a fragment thereof (e.g., SEQ ID NO: 35, VVGADGVGK) or a variant thereof comprising one or more (e.g. one or two) conservative amino acid substitutions (preferably corresponding to position 1, 2, 3, 4, 5, 6, 7, 8, 9 and/or 10 and/or SEQ ID NO: 1).
36. The complex and/or the combination, and/or the cell population or the cell of any one of the preceding items, wherein the TCR has antigenic specificity for said epitope presented in the context of an HLA-A molecule.
37. The complex and/or the combination, and/or the cell population or the cell of any one of the preceding items, wherein the TCR has antigenic specificity for said epitope presented in the context of:
   i) an HLA-A molecule, preferably HLA-A*11 molecule;
   ii) an HLA-A*11:01 molecule; and/or
   iii) at least any one of an HLA-A*11 molecule, preferably HLA-A*11:01, HLA-A*11:02, HLA-A*11:03 and/or HLA-A*11:12.
38. The complex and/or the combination, and/or the cell population or the cell of any one of the preceding items, wherein the at least 80% (e.g. at least 81%, 82%, 83%, 84%, 85%, 86%, 87%, 88%, 89%, 90%, 91%, 92%, 93%, 94%, 95%, 96%, 97%, 98%, 99% or 100%) identity comprises conservative and/or highly conservative amino acid substitutions, preferably corresponding to position(s) 1, 2, 3, 4, 5 and/or 6 of SEQ ID NO: 14; position(s) 1, 2, 3, 4, 5, 6, 7 and/or 8 of SEQ ID NO: 15; position(s) 1, 2, 3, 4, 5, 6, 7, 8, 9, 10, 11, 12 and/or 13 of SEQ ID NO: 16; position(s) 1, 2, 3, 4 and/or 5 of SEQ ID NO: 2; position(s) 1, 2, 3, 4, 5 and/or 6 of SEQ ID NO: 3; and/or position(s) 1, 2, 3, 4, 5, 6, 7, 8, 9, 10, 11, 12, 13 and/or 14 of SEQ ID NO: 4.
39. The complex and/or the combination, and/or the cell population or the cell of any one of the preceding items, wherein the TCR comprises at least one (e.g., 1, 2, 3, 4, 5, or 6) of the following:
   i) an α chain CDR 1 comprising the amino acid sequence of SEQ ID NO: 14;
   ii) an α chain CDR2 comprising the amino acid sequence of SEQ ID NO: 15;
   iii) an α chain CDR3 comprising the amino acid sequence of SEQ ID NO: 16;
   iv) a β chain CDR1 comprising the amino acid sequence of SEQ ID NO: 2;
   v) a β chain CDR2 comprising the amino acid sequence of SEQ ID NO: 3; and/or
   vi) a β chain CDR3 comprising the amino acid sequence of SEQ ID NO: 4.
40. The complex and/or the combination, and/or the cell population or the cell of any one of the preceding items, wherein the TCR comprises:
   i) an α chain variable region comprising the amino acid sequence of SEQ ID NO: 20, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 20; and/or
   ii) a β chain variable region comprising the amino acid sequence of SEQ ID NO: 8, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 8.
41. The complex and/or the combination, and/or the cell population or the cell of any one of the preceding items, wherein the TCR further comprises:
   i) an α chain constant region comprising the amino acid sequence of SEQ ID NO: 22, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 22; and/or
   ii) a β chain constant region comprising the amino acid sequence of SEQ ID NO: 10, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 10.
42. The complex and/or the combination, and/or the cell population or the cell of any one of the preceding items, wherein the TCR comprises:
   i) an α chain comprising the amino acid sequence of SEQ ID NO: 24, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 24; and/or
   ii) a β chain comprising the amino acid sequence of SEQ ID NO: 12, or an amino acid sequence having at least 80 % (e.g. at least 81%, 82%, 83%, 84%, 85%, 86%, 87%, 88%, 89%, 90%, 91%, 92%, 93%, 94%, 95%, 96%, 97%, 98%, 99% or 100%) identity to SEQ ID NO: 12.
43. The complex and/or the combination, and/or the cell population or the cell of any one of any one of the preceding items, wherein the TCR has antigenic specificity for said epitope in the absence of the CD8 co-receptor.
44. The complex and/or the combination, and/or the cell population or the cell of any one of the preceding items, wherein the TCR is selected from the group consisting of a native TCR, a TCR variant, a TCR fragment, and a TCR construct.
45. The complex and/or the combination, and/or the cell population or the cell of any one of the preceding items, wherein the TCR construct comprises at least one TCR alpha-chain(s) and at least one TCR beta-chain(s) covalently linked to each other to form TCR heterodimers or multimers.
46. The complex and/or the combination, and/or the cell population or the cell of any one of the preceding items, the TCR further comprising one or more fusion component(s) selected from the group consisting of: any suitable segment or fragment of a protein or polypeptide, preferably a peptide comprising at least six or at least nine amino acids, e.g., one or more of any one of: SEQ ID NOs: 41-44) recognized by the immune system, e.g., by T cells via their TCRs and/or by antibodies, further preferably said segment or fragment comprises an uniTope 9-mer or uniTope 6-mer of one or more of any one of SEQ ID NOs: 41-44 (e.g., according to one or more of any one of SEQ ID NOs: 38, 45 or 46); a Fc receptor; a Fc domain, including IgA, IgD, IgG, IgE, and IgM; a cytokine, including IL-2 or IL-15; a toxin; an antibody or an antigen-binding fragment thereof, including anti-CD3, anti-CD28, anti-CD5, anti-CD 16 or an anti- CD56 antibody or an antigen-binding fragment thereof; and a CD247 (CD3-zeta), CD28, CD137, or a CD134 domain, or combinations thereof, optionally further comprising at least one linker.
47. The complex and/or the combination, and/or the cell population or the cell of any one of the preceding items, wherein the TCR further comprises at least one label or at least one epitope-tag, preferably said at least one label or at least one epitope-tag (e.g., wherein said epitope-tag is not immunogenic and/or detectable, for example, by an antibody) comprising one or more of any one of: SEQ ID NOs: 41-44, optionally according to one or more of any one of: SEQ ID NOs: 38, 45 or 46.
48. The complex and/or the combination, and/or the cell population or the cell of any one of the preceding items, wherein the TCR is soluble.
49. The complex and/or the combination, and/or the cell population or the cell of any one of the preceding items, wherein the chimeric co-stimulatory receptor comprises:
   i) an extracellular domain, preferably comprising a polypeptide derived from PD-1, further preferably at its N-terminus (e.g., SEQ ID NO: 28);
   ii) a transmembrane domain, preferably comprising a polypeptide derived from PD-1 (e.g., SEQ ID NO: 30); and/or
   iii) an intracellular domain, preferably comprising a polypeptide derived from 4-1BB, further preferably at its C-terminus (e.g., SEQ ID NO: 32).
50. The complex and/or the combination, and/or the cell population or the cell of any one of the preceding items, wherein the transmembrane domain comprises a polypeptide derived from PD-1 (e.g., SEQ ID NO: 30).
51. The complex and/or the combination, and/or the cell population or the of any one of the preceding items, wherein
   i) said polypeptide derived from PD-1 comprised by said extracellular and/or by said transmembrane domain is a polypeptide derived from human PD-1; and/or
   ii) said polypeptide derived from 4-1BB comprised by said intracellular domain is a polypeptide derived from human 4-1BB.
52. The complex and/or the combination, and/or the cell population or the cell of any one of the preceding items, wherein
   i) said extracellular domain comprising a polypeptide derived from PD-1 comprises an amino acid sequence with 0, 1, 2, 3, 4, 5, 6, 7, 8, 9, or 10 amino acid substitutions, deletions, and/or insertions compared to SEQ ID NO: 28;
   ii) said extracellular domain comprising a variant of the parent extracellular PD-1 polypeptide of SEQ ID NO: 28, wherein said variant comprising an alteration (e.g., a substitution, deletion and/or insertion) at one or more positions corresponding to positions of the parent polypeptide, wherein said variant having at least 80% (e.g. at least 81%, 82%, 83%, 84%, 85%, 86%, 87%, 88%, 89%, 90%, 91%, 92%, 93%, 94%, 95%, 96%, 97%, 98% or 99%), but less than 100% sequence identity to a parent amino acid sequence of SEQ ID NO: 28;
   iii) said transmembrane domain comprising a polypeptide derived from PD-1 comprises an amino acid sequence with 0, 1, 2, 3, 4, 5, 6, 7, 8, 9, or 10 amino acid substitutions, deletions, and/or insertions compared to the amino acid sequence of SEQ ID NO: 30;
   iv) said transmembrane domain comprising a variant of the parent PD-1 transmembrane polypeptide of SEQ ID NO: 30, wherein said variant comprising an alteration (e.g., a substitution, deletion and/or insertion) at one or more positions corresponding to positions of the parent polypeptide, wherein said variant having at least 80% (e.g. at least 81%, 82%, 83%, 84%, 85%, 86%, 87%, 88%, 89%, 90%, 91%, 92%, 93%, 94%, 95%, 96%, 97%, 98% or 99%), but less than 100% sequence identity to a parent amino acid sequence of SEQ ID NO: 30;
   v) said intracellular domain comprising a polypeptide derived from 4-1BB comprises an amino acid sequence with 0, 1, 2, 3, 4, 5, 6, 7, 8, 9, or 10 amino acid substitutions, deletions, and/or insertions compared to SEQ ID NO: 32;
      and/or
   vi) said intracellular domain comprising a variant of the parent 4-1BB intracellular polypeptide of SEQ ID NO: 32, wherein said variant comprising an alteration (e.g., a substitution, deletion and/or insertion) at one or more positions corresponding to positions of the parent polypeptide, wherein said variant having at least 80% (e.g. at least 81%, 82%, 83%, 84%, 85%, 86%, 87%, 88%, 89%, 90%, 91%, 92%, 93%, 94%, 95%, 96%, 97%, 98% or 99%), but less than 100% sequence identity to a parent amino acid sequence of SEQ ID NO: 32.
53. The complex and/or the combination, and/or the cell population or the cell of any one of the preceding items, wherein
   i) said extracellular domain comprises the amino acid sequence of SEQ ID NO: 28;
   ii) said transmembrane domain comprises the amino acid sequence of SEQ ID NO: 30; and/or
   iii) said intracellular domain comprises the amino acid sequence of SEQ ID NO: 32.
54. The complex and/or the combination, and/or the cell population or the cell of any one of the preceding items, wherein said fusion protein comprises the amino acid sequence of SEQ ID NO: 26 or an amino acid sequence having at least 80% (e.g. at least 81%, 82%, 83%, 84%, 85%, 86%, 87%, 88%, 89%, 90%, 91%, 92%, 93%, 94%, 95%, 96%, 97%, 98% or 99%) identity to SEQ ID NO: 26.
55. The cell population or the cell of any one of the preceding items, wherein the cell is selected from lymphocytes including but not limited to cytotoxic T lymphocytes (CTLs), CD8⁺ T cells, CD4⁺ T cells, natural killer (NK) cells, natural killer T (NKT) cells, and gamma/ delta-T-cells.
56. The cell population or the cell of any one of the preceding items, wherein the cell population comprises cells which secrete at least two proteins or the cell secrets at least two proteins.
57. The cell population or the cell of any one of the preceding items, wherein the proteins are selected from the group consisting of effector proteins, stimulatory cytokines and chemo-attractive cytokines.
58. The complex and/or the combination of any one of the preceding items, wherein the complex comprises a cellular membrane, wherein the TCR and the chimeric co-stimulatory receptor are located in or on said membrane.
59. A nucleic acid comprising a nucleotide sequence encoding the TCR (or fragment thereof, e.g., as depicted in Table 2) as defined by any one of the preceding items and/or a nucleotide sequence encoding the chimeric co-stimulatory receptor as defined by any one of the preceding items (or fragment thereof, e.g., as depicted in Table 2, preferably one or more of SEQ ID NO: 27, 29, 31 and/or 33).
60. A vector comprising the nucleic acid of any one of the preceding items, preferably said vector is an expression vector or a viral vector.
61. A host cell (e.g., isolated and/or recombinant host cell) comprising the nucleic acid of any one of the preceding items, or the vector of any one of the preceding items, preferably said host cell is selected from the group consisting of: a prokaryotic host cell (e.g., bacterial cell), fungal host cell, mammalian host cell, non-human mammalian host cell.
62. A method for obtaining the TCR and the chimeric co-stimulatory receptor as defined by any one of the preceding items, comprising
   (i) incubating the host cell of any one of the preceding items under conditions causing expression of said TCR and of said chimeric co-stimulatory receptor; and
   (ii) purifying said TCR and said chimeric co-stimulatory receptor.
63. A composition, preferably a pharmaceutical or diagnostic composition, comprising one or more of:
   (i) the complex and/or the combination of any one of the preceding items;
   (ii) the cell population or the cell of any one of the preceding items,
   (iii) the nucleic acid of any one of the preceding items;
   (iv) the vector of any one of the preceding items; and/or
   (v) the host cell of any one of the preceding items;
   and, optionally, pharmaceutically excipient(s).
64. The complex and/or the combination, and/or the cell population or the cell any one of the preceding items, the nucleic acid any one of the preceding items, the vector any one of the preceding items, the host cell any one of the preceding items and/or the pharmaceutical composition any one of the preceding items, for use as a medicament and/or for use in therapy.
65. The complex and/or the combination, and/or the cell population or the cell any one of the preceding items, the nucleic acid any one of the preceding items, the vector any one of the preceding items, the host cell any one of the preceding items and/or the pharmaceutical composition any one of the preceding items, for use in a method of detecting, diagnosing, prognosing, preventing and/or treating cancer.
66. The complex and/or the combination, and/or the cell population or the cell, the nucleic acid, the vector, the host cell and/or the pharmaceutical composition for the use of any one of the preceding items, wherein prevention and/or treatment of cancer comprises:
   administering at least any one of the following (i) to (vi):
      (i) the complex and/or the combination any one of the preceding items;
      (ii) the cell population or the cell any one of the preceding items,
      (iii) the nucleic acid any one of the preceding items;
      (iv) the vector any one of the preceding items;
      (v) the host cell any one of the preceding items; and/or
      (vi) the pharmaceutical composition any one of the preceding items;
   to a subject in need thereof.
67. The complex and/or the combination, and/or the cell population or the cell, the nucleic acid, the vector, the host cell and/or the pharmaceutical composition for the use any one of the preceding items, wherein prevention and/or treatment of cancer comprises:
   (a) introducing at least any one of the following (i) to (vi):
      (i) the complex and/or the combination of any one of the preceding items;
      (ii) the cell population or the cell of any one of the preceding items,
      (iii) the nucleic acid of any one of the preceding items;
      (iv) the vector of any one of the preceding items;
      (v) the host cell of any one of the preceding items; and/or
      (vi) the pharmaceutical composition of any one of the preceding items;
      into lymphocytes comprised by a sample obtained from a subject, thereby obtaining modified lymphocytes, and
   (b) administering the modified lymphocytes of step (a) to a subject in need thereof.
68. A method of detecting the presence of a cancer in a subject *in vitro,* comprising:
   (a) contacting a sample obtained from a subject and comprising one or more cells with
      (i) the complex and/or the combination of any one of the preceding items;
      (ii) the cell population or the cell of any one of the preceding items,
      (iii) the nucleic acid of any one of the preceding items;
      (iv) the vector of any one of the preceding items;
      (v) the host cell of any one of the preceding items; and/or
      (vi) the pharmaceutical composition of any one of the preceding items;
      thereby forming a complex, and
   (b) detecting the complex,
   wherein detection of the complex is indicative of the presence of the cancer in the subject.
69. Use of the complex and/or the combination, and/or the cell population or the cell of any one of the preceding items, the nucleic acid of any one of the preceding items and/or the vector of any one of the preceding items for generating modified lymphocytes.
70. A kit comprising the complex and/or the combination, and/or the cell population or the cell of any one of the preceding items, the nucleic acid of any one of the preceding items, the vector of any one of the preceding items, the host cell of any one of the preceding items, and/or the pharmaceutical or diagnostic composition of any one of the preceding items.
71. The complex and/or the combination, and/or the cell population or the cell, the nucleic acid, the vector, the host cell, the pharmaceutical and/or diagnostic composition, method, use or kit according to any one of the preceding items, carried out as shown in Examples (e.g., Figures and/or Tables as depicted herein) and/or herein below.

### EXAMPLES OF THE INVENTION

In order that the invention may be readily understood and put into practical effect, some aspects of the invention are described by way of the following non-limiting examples.

### Materials and Methods

### Materials

### Sequences

**Table 2: TCR sequences and epitopes.**

| **SEQ ID** | **Description** | **Origin** | **Sequence** |
|---|---|---|---|
| **1** | epitope_aa (mKRAS 7-16 G12**D**) | Synthetic derived from human | VVVGA**D**GVGK |
| **2** | CDR1_b_aa | Synthetic derived from human | WNHNN |
| **3** | CDR2_b_aa | Synthetic derived from human | SYGVHD |
| **4** | CDR3_b_aa | Synthetic derived from human | CASSGAGETGELFF |
| **5** | CDR1_b_nt | artificial: codon optimized | tggaaccacaacaac |
| **6** | CDR2_b_nt | artificial: codon optimized | tcttacggcgtgcacgac |
| **7** | CDR3_b_nt | artificial: codon optimized | tgtgcctctagcggagccggcgaaaccggcgaactgtttttc |
| **8** | Vb_aa (TRBV + CDR3 + TRBJ) | Synthetic derived from human | MGTRLFFYVALCLLWAGHRDAEITQSPRHKITET GRQVTLACHQTWNHNNMFWYRQDLGHGLRL IHYSYGVHDTNKGEVSDGYSVSRSNTEDLPLTLES AASSQTSVYFCASSGAGETGELFFGEGSRLTVL |
| **9** | Vb_nt (TRBV + CDR3 + TRBJ) | artificial: codon optimized | atgggcaccagactgttcttttacgtggccctgtgcctgctgt gggccggacatagagatgccgagatcacacagagccccag acacaagatcaccgagacaggcagacaagtgaccctggcc tgtcaccagacctggaaccacaacaacatgttctggtacag acaggacctcggccacggcctgagactgatccactactctta cggcgtgcacgacaccaacaagggcgaagtgtctgacggct acagcgtgtccagaagcaacaccgaggacctgcctctgaca ctggaatctgccgccagctctcagaccagcgtgtacttttgtg cctctagcggagccggcgaaaccggcgaactgtttttcgga gagggcagcagactgaccgtgctc |
| **10** | Cb_aa (TRBC01) | artificial: minimal murinized | EDLNKVFPPEVAVFEPSKAEIAHTQKATLVCLATG FFPDHVELSWWVNGKEVHSGVSTDPQPLKEQP ALNDSRYCLSSRLRVSATFWQNPRNHFRCQVQF YGLSENDEWTQDRAKPVTQIVSAEAWGRADCG ITSASYHQGVLSATILYEILLGKATLYAVLVSALVLM AMVKRKDF |
| **11** | Cb_nt (TRBC01) | artificial: codon optimized | gaggacctgaacaaggtgttccctccggaggtggccgtgttc gagcccagcaaagccgagatcgcgcacacccagaaggcca ccctggtgtgcctggccaccggcttcttccccgaccacgtgg agctgagctggtgggtgaacggcaaggaggtgcacagcgg cgtgagcaccgacccccagcccctgaaggagcagcccgcc ctgaacgacagccgctactgcctgagcagccgcctgcgcgt gagcgccaccttctggcagaacccccgcaaccacttccgctg ccaggtgcagttctacggcctgagcgagaacgacgagtgga cccaggaccgcgccaagcccgtgacccagatcgtgagcgcc gaggcctggggccgcgccgactgcggcattaccagcgcga gctaccatcagggcgtgctgagcgccaccatcctgtacgag atcctgctgggcaaggccaccctgtacgccgtgctggtgagc gccctggtgctgatggcgatggtgaagcgcaaggacttc |
| **12** | TCRb_aa(TRBV + CDR3 + TRBJ +Cb) | | MGTRLFFYVALCLLWAGHRDAEITQSPRHKITET GRQVTLACHQTWNHNNMFWYRQDLGHGLRL IHYSYGVHDTNKGEVSDGYSVSRSNTEDLPLTLES |
| | | artificial: C region minimal murinized | AASSQTSVYFCASSGAGETGELFFGEGSRLTVLE DLNKVFPPEVAVFEPSKAEIAHTQKATLVCLATGF FPDHVELSWWVNGKEVHSGVSTDPQPLKEQPA LNDSRYCLSSRLRVSATFWQNPRNHFRCQVQFY GLSENDEWTQDRAKPVTQIVSAEAWGRADCGI TSASYHQGVLSATILYEILLGKATLYAVLVSALVLM AMVKRKDF |
| **13** | TCRb_nt (TRBV + CDR3 + TRBJ +Cb) | artificial: codon optimized | atgggcaccagactgttcttttacgtggccctgtgcctgctgt gggccggacatagagatgccgagatcacacagagccccag acacaagatcaccgagacaggcagacaagtgaccctggcc tgtcaccagacctggaaccacaacaacatgttctggtacag acaggacctcggccacggcctgagactgatccactactctta cggcgtgcacgacaccaacaagggcgaagtgtctgacggct acagcgtgtccagaagcaacaccgaggacctgcctctgaca ctggaatctgccgccagctctcagaccagcgtgtacttttgtg cctctagcggagccggcgaaaccggcgaactgtttttcgga gagggcagcagactgaccgtgctcgaggacctgaacaagg tgttccctccggaggtggccgtgttcgagcccagcaaagccg agatcgcgcacacccagaaggccaccctggtgtgcctggcc accggcttcttccccgaccacgtggagctgagctggtgggtg aacggcaaggaggtgcacagcggcgtgagcaccgaccccc agcccctgaaggagcagcccgccctgaacgacagccgcta ctgcctgagcagccgcctgcgcgtgagcgccaccttctggca gaacccccgcaaccacttccgctgccaggtgcagttctacgg cctgagcgagaacgacgagtggacccaggaccgcgccaag cccgtgacccagatcgtgagcgccgaggcctggggccgcgc cgactgcggcattaccagcgcgagctaccatcagggcgtgc tgagcgccaccatcctgtacgagatcctgctgggcaaggcc accctgtacgccgtgctggtgagcgccctggtgctgatggcg atggtgaagcgcaaggacttc |
| **14** | CDR1_a_aa | Synthetic derived from human | SSVSVY |
| **15** | CDR2_a_aa | Synthetic derived from human | YLSGSTLV |
| **16** | CDR3_a_aa | Synthetic derived from human | CAVSRSGNTPLVF |
| **17** | CDR1_a_nt | artificial: codon optimized | agcagcgtgtccgtgtac |
| **18** | CDR2_a_nt | artificial: codon optimized | tatctgagcggcagcaccctggtg |
| **19** | CDR3_a_nt | artificial: codon optimized | tgcgccgtgtccagaagcggaaacacccctctggtgttc |
| **20** | Va_aa (TRAV + CDR3 + TRAJ) | Synthetic derived from human | MLLLLVPAFQVIFTLGGTRAQSVTQLDSQVPVFE EAPVELRCNYSSSVSVYLFWYVQYPNQGLQLLLK YLSGSTLVESINGFEAEFNKSQTSFHLRKPSVHIS DTAEYFCAVSRSGNTPLVFGKGTRLSVIAN |
| **21** | Va_nt (TRAV + CDR3 + TRAJ) | artificial: codon optimized | atgcttctgctgctggtgcctgccttccaagtgatctttaccct cggcggcacaagagcccagagcgtgacacagctggatagc caggtgccagtgttcgaggaagcccctgtcgagctgagatg caactacagcagcagcgtgtccgtgtacctgttttggtacgtg cagtaccccaaccagggcctgcagctgctgctcaagtatctg agcggcagcaccctggtggaatccatcaatggcttcgaggc cgagttcaacaagagccagaccagcttccacctgagaaagc ccagcgtgcacatcagcgataccgccgagtacttctgcgccg tgtccagaagcggaaacacccctctggtgttcggcaagggc acaagactgagcgtgatcgccaat |
| **22** | Ca_aa | artificial: minimal murinized | IQNPDPAVYQLRDSKSSDKSVCLFTDFDSQTNVS QSKDSDVYITDKTVLDMRSMDFKSNSAVAWSN KSDFACANAFNNSIIPEDTFFPSSDVPCDVKLVEK SFETDTNLNFQNLSVIGFRILLLKVAGFNLLMTLR LWSS |
| **23** | Ca_nt | artificial: codon optimized | atccagaatccggaccccgccgtgtaccagctgcgcgacag caagagcagcgacaagagcgtgtgcctgttcaccgacttcg acagccagaccaacgtgagccagagcaaggacagcgacgt gtacatcaccgacaagaccgtgctggacatgcgcagcatgg acttcaagagcaacagcgccgtggcctggagcaacaagag cgacttcgcctgcgccaacgccttcaacaacagcatcatccc cgaggacaccttcttccccagcagcgatgtgccgtgcgacgt gaagctggtggagaagagcttcgagaccgacaccaacctg aacttccagaacctgagcgtgatcggcttccgcatcctgctg |
| | | | ctgaaggtggccggcttcaacctgctgatgaccctgcgcctg tggagcagctga |
| **24** | TCRa_aa (TRAV + CDR3 + TRAJ + Ca) | artificial: C region minimal murinized | MLLLLVPAFQVIFTLGGTRAQSVTQLDSQVPVFE EAPVELRCNYSSSVSVYLFWYVQYPNQGLQLLLK YLSGSTLVESINGFEAEFNKSQTSFHLRKPSVHIS DTAEYFCAVSRSGNTPLVFGKGTRLSVIANIQNP DPAVYQLRDSKSSDKSVCLFTDFDSQTNVSQSK DSDVYITDKTVLDMRSMDFKSNSAVAWSNKSD FACANAFNNSIIPEDTFFPSSDVPCDVKLVEKSFE TDTNLNFQNLSVIGFRILLLKVAGFNLLMTLRLW SS |
| **25** | TCRa_nt (TRAV + CDR3 + TRAJ + Ca) | artificial: codon optimized | atgcttctgctgctggtgcctgccttccaagtgatctttaccct cggcggcacaagagcccagagcgtgacacagctggatagc caggtgccagtgttcgaggaagcccctgtcgagctgagatg caactacagcagcagcgtgtccgtgtacctgttttggtacgtg cagtaccccaaccagggcctgcagctgctgctcaagtatctg agcggcagcaccctggtggaatccatcaatggcttcgaggc cgagttcaacaagagccagaccagcttccacctgagaaagc ccagcgtgcacatcagcgataccgccgagtacttctgcgccg tgtccagaagcggaaacacccctctggtgttcggcaagggc acaagactgagcgtgatcgccaatatccagaatccggaccc cgccgtgtaccagctgcgcgacagcaagagcagcgacaag agcgtgtgcctgttcaccgacttcgacagccagaccaacgtg agccagagcaaggacagcgacgtgtacatcaccgacaaga ccgtgctggacatgcgcagcatggacttcaagagcaacagc gccgtggcctggagcaacaagagcgacttcgcctgcgccaa cgccttcaacaacagcatcatccccgaggacaccttcttccc cagcagcgatgtgccgtgcgacgtgaagctggtggagaaga gcttcgagaccgacaccaacctgaacttccagaacctgagc gtgatcggcttccgcatcctgctgctgaaggtggccggcttca acctgctgatgaccctgcgcctgtggagcagc |
| **26** | PD1-41BB_aa | artificial fusion protein | MQIPQAPWPVVWAVLQLGWRPGWFLDSPDR PWNPPTFSPALLVVTEGDNATFTCSFSNTSESFV LNWYRMSPSNQTDKLAAFPEDRSQPGQDCRF RVTQLPNGRDFHMSVVRARRNDSGTYLCGAISL APKAQIKESLRAELRVTERRAEVPTAHPSPSPRPA GQFQTLVVGVVGGLLGSLVLLVWVLAVIKRGRK KLLYIFKQPFMRPVQTTQEEDGCSCRFPEEEEGG CEL |
| **27** | PD1-41BB_nt | artificial: codon optimized | atgcaaattcctcaagctccttggcctgtcgtgtgggccgttc tgcaacttggatggcggcctggctggttcctggactctcctga cagaccctggaatcctccaacattcagccccgctctgctggt ggttaccgagggcgataatgccaccttcacctgtagcttcag caacaccagcgagagcttcgtgctgaactggtacagaatga gccccagcaaccagaccgacaagctggccgcctttcctgag gatagatctcagcccggccaggactgccggttcagagttaca cagctgcccaacggccgggacttccacatgtctgtcgtccgg gccagaagaaacgacagcggcacatatctgtgcggcgccat ttctctggcccctaaggctcagatcaaagagagcctgagag ccgagctgagagtgacagaaagacgggccgaagtgcccac agctcacccttcaccttctccaagacctgccggccagttccag acactggtcgtgggagttgttggcggactgctgggatctctg gtgctgcttgtttgggtgctcgccgtgatcaagcggggcaga aagaagctgctgtacatcttcaagcagcccttcatgcggccc gtgcagaccacacaagaggaagatggctgctcctgcagatt ccccgaggaagaagaaggcggctgcgaactc |
| **28** | PD1 ECD_aa | Synthetic derived from human | MQIPQAPWPVVWAVLQLGWRPGWFLDSPDR PWNPPTFSPALLVVTEGDNATFTCSFSNTSESFV LNWYRMSPSNQTDKLAAFPEDRSQPGQDCRF RVTQLPNGRDFHMSVVRARRNDSGTYLCGAISL APKAQIKESLRAELRVTERRAEVPTAHPSPSPRPA GQFQTLV |
| **29** | PD1 ECD_nt | artificial: codon optimized | atgcaaattcctcaagctccttggcctgtcgtgtgggccgttc tgcaacttggatggcggcctggctggttcctggactctcctga cagaccctggaatcctccaacattcagccccgctctgctggt ggttaccgagggcgataatgccaccttcacctgtagcttcag caacaccagcgagagcttcgtgctgaactggtacagaatga gccccagcaaccagaccgacaagctggccgcctttcctgag gatagatctcagcccggccaggactgccggttcagagttaca cagctgcccaacggccgggacttccacatgtctgtcgtccgg gccagaagaaacgacagcggcacatatctgtgcggcgccat ttctctggcccctaaggctcagatcaaagagagcctgagag ccgagctgagagtgacagaaagacgggccgaagtgcccac agctcacccttcaccttctccaagacctgccggccagttccag acactggtc |
| **30** | PD1 TM_aa | Synthetic derived from human | VGVVGGLLGSLVLLVWVLAVI |
| **31** | PD1 TM_nt | artificial: codon optimized | gtgggagttgttggcggactgctgggatctctggtgctgcttg tttgggtgctcgccgtgatc |
| **32** | 41BB_ICD_aa | Synthetic derived from human | KRGRKKLLYIFKQPFMRPVQTTQEEDGCSCRFPE EEEGGCEL |
| **33** | 41BB_ICD_nt | artificial: codon optimized | aagcggggcagaaagaagctgctgtacatcttcaagcagcc cttcatgcggcccgtgcagaccacacaagaggaagatggct gctcctgcagattccccgaggaagaagaaggcggctgcga actc |
| **34** | epitope_aa (mKRAS₇₋₁₆ **G12C)** | Synthetic derived from human | VVVGACGVGK |
| **35** | epitope_aa (mKRAS ₈₋₁₆ G12**D**) | Synthetic derived from human | VVGADGVGK |
| **36** | epitope_aa (mKRAS₈₋₁₆ G12C) | Synthetic derived from human | VVGACGVGK |
| **37** | epitope_aa (KRAS₁₋₂₄ WT) | Synthetic derived from human | MTEYKLVVVGAGGVGKSALTIQLI |
| **38** | Cb_aa (TRBC01) with uniTope 9_mer | artifical: minimal murinized | EDLNKVFPPEVAVFEPSKAEIAHTQKATLVCLATG FFPDHVELSWWVNGKEVHSGVSTDPQPLKEQP ALNDGSGGEVPKDRFSGSGSRYCLSSRLRVSATF WQNPRNHFRCQVQFYGLSENDEWTQDRAKPV TQIVSAEAWGRADCGITSASYHQGVLSATILYEIL LGKATLYAVLVSALVLMAMVKRKDF |
| **39** | Cb_nt (TRBC01) with uniTope 9_mer | artifical: codon optimized | Gaggacctgaacaaggtgttccctccggaggtggccgtgtt cgagcccagcaaagccgagatcgcgcacacccagaaggcc accctggtgtgcctggccaccggcttcttccccgaccacgtg gagctgagctggtgggtgaacggcaaggaggtgcacagcg gcgtgagcaccgacccccagcccctgaaggagcagcccgc cctgaacgacggatctggcggcgaagtgcccaaggacaga ttttccggatctggaagccgctactgcctgagcagccgcctg cgcgtgagcgccaccttctggcagaacccccgcaaccacttc cgctgccaggtgcagttctacggcctgagcgagaacgacga gtggacccaggaccgcgccaagcccgtgacccagatcgtga gcgccgaggcctggggccgcgccgactgcggcattaccagc gcgagctaccatcagggcgtgctgagcgccaccatcctgtac gagatcctgctgggcaaggccaccctgtacgccgtgctggtg agcgccctggtgctgatggcgatggtgaagcgcaaggactt c |
| **40** | fragment of the human mKRAS G12D | Synthetic derived from human | MTEYKLVVVGADGVGKSALTIQLIQNHFVDEYD PTIEDSYRKQVVIDGETCLLDILDTAGQEEYSAMR DQYMRTGEGFLCVFAINNTKSFEDIHHYREQIKR VKDSEDVPMVLVGNKCDLPSRTVDTKQAQDLA |
| **41** | Exemplary uniTope 9mer including GSG linker | Synthetic derived from human | GSGGEVPKDRFSGSG |
| **42** | Exemplary uniTope 9_mer | Synthetic derived from human | GEVPKDRFS |
| **43** | Exemplary uniTope 6merincluding GSG linker | Synthetic derived from human | GSGEVPKDRGSG |
| **44** | Exemplary uniTope 6_mer | Synthetic derived from human | EVPKDR |
| **45** | KrasG12D(T74.8 -054-14)_TCR16_Beta Constant _Glu202_6-mer_Unitope AA | Synthetic derived from human | EDLNKVFPPEVAVFEPSKAEIAHTQKATLVCLATG FFPDHVELSWWVNGKEVHSGVSTDPQPLKEQP ALNDGSGEVPKDRGSGSRYCLSSRLRVSATFWQ NPRNHFRCQVQFYGLSENDEWTQDRAKPVTQI VSAEAWGRADCGITSASYHQGVLSATILYEILLGK ATLYAVLVSALVLMAMVKRKDF |
| **46** | KrasG12D(T74.8 -054-14)_TCR16_Beta Constant _Glu202_9-mer_Unitope AA | Synthetic derived from human | EDLNKVFPPEVAVFEPSKAEIAHTQKATLVCLATG FFPDHVELSWWVNGKEVHSGVSTDPQPLKEQP ALNDGSGGEVPKDRFSGSGSRYCLSSRLRVSATF WQNPRNHFRCQVQFYGLSENDEWTQDRAKPV TQIVSAEAWGRADCGITSASYHQGVLSATILYEIL LGKATLYAVLVSALVLMAMVKRKDF |
| **47** | KrasG12D(T74.8 -054-14)_TCR16_Beta Constant _Glu202_6-mer_Unitope Nuc | Synthetic derived from human | gaggacctgaacaaggtgttccctccggaggtggccgtgttc gagcccagcaaagccgagatcgcgcacacccagaaggcca ccctggtgtgcctggccaccggcttcttccccgaccacgtgg agctgagctggtgggtgaacggcaaggaggtgcacagcgg cgtgagcaccgacccccagcccctgaaggagcagcccgcc ctgaacgacggatctggcgaagtgcccaaggacagaggat ctggaagccgctactgcctgagcagccgcctgcgcgtgagc gccaccttctggcagaacccccgcaaccacttccgctgccag gtgcagttctacggcctgagcgagaacgacgagtggaccca ggaccgcgccaagcccgtgacccagatcgtgagcgccgag gcctggggccgcgccgactgcggcattaccagcgcgagcta ccatcagggcgtgctgagcgccaccatcctgtacgagatcct gctgggcaaggccaccctgtacgccgtgctggtgagcgccc tggtgctgatggcgatggtgaagcgcaaggacttc |
| **48** | KrasG12D(T74.8 -054-14)_TCR16_Beta Constant _Glu202_9-mer_Unitope Nuc | Synthetic derived from human | gaggacctgaacaaggtgttccctccggaggtggccgtgttc gagcccagcaaagccgagatcgcgcacacccagaaggcca ccctggtgtgcctggccaccggcttcttccccgaccacgtgg agctgagctggtgggtgaacggcaaggaggtgcacagcgg |
| | | | cgtgagcaccgacccccagcccctgaaggagcagcccgcc ctgaacgacggatctggcggcgaagtgcccaaggacagatt ttccggatctggaagccgctactgcctgagcagccgcctgcg cgtgagcgccaccttctggcagaacccccgcaaccacttccg ctgccaggtgcagttctacggcctgagcgagaacgacgagt ggacccaggaccgcgccaagcccgtgacccagatcgtgag cgccgaggcctggggccgcgccgactgcggcattaccagcg cgagctaccatcagggcgtgctgagcgccaccatcctgtacg agatcctgctgggcaaggccaccctgtacgccgtgctggtga gcgccctggtgctgatggcgatggtgaagcgcaaggacttc |

### Methods

### Identification and isolation of mKRAS₇₋₁₆ G12D-specific TCR from T cell repertoires of a healthy donor using in vitro high-throughput T cell priming technology.

This invention describes a TCR with high specificity and sensitivity for mKRAS₇₋₁₆G12D epitope isolated from T cell repertoires of a healthy donor using *in vitro* high-throughput T cell priming technology as follows. Particularly, the priming system employed used monocyte-derived mature dendritic cells (mDCs) isolated from peripheral blood mononuclear cells (PBMC) of HLA-A*11:01-positive healthy donors as antigen-presenting cells and autologous CD8-enriched T cells as responding cells. *In vitro* transcribed RNA (*iv*tRNA) encoding a fragment of the human mKRAS G12D (amino acid sequence MTEYKLVVVGADGVGKSALTIQLIQNHFVDEYDPTIEDSYRKQVVIDGETCLLDILDTAGQEE YSAMRDQYMRTGEGFLCVFAINNTKSFEDIHHYREQIKRVKDSEDVPMVLVGNKCDLPSRT VDTKQAQDLA, SEQ ID NO: 40) served as the source of specific antigen. After electroporation into the mDCs, the mKRAS G12D-encoding ivtRNA was translated into a polypeptide, which was subsequently processed and presented as peptides by HLA-A*11:01 molecules endogenously expressed by mDCs. *In vitro* co-cultures of T cells with ivtRNA-transfected mDCs from the same donor led to *de novo* induction of antigen-specific T cells that served as the source of corresponding TCRs.

Subsequently, mKRASG12D-specificT cells were identified using HLA-A*11:01 mKRAS G12D fluorescently labelled multimers and separated by single cell sorting using fluorescence-activated cell sorting (FACS) technology. After *in vitro* expansion, specific T cell clones of interest were pre-screened for differential mKRAS G12D vs. wild type KRAS recognition on tumor target cells, and further selected using a broader tumor cell panel with multiple mKRAS G12D-endogenously positive tumor cell lines, mKRAS G12D-overexpressing tumor cell lines and KRAS wild type tumor cell lines as negative controls.

Following the identification of promising T cells clones that recognized the desired mKRASG12D epitope presented by HLA-A*11:01 molecules on tumor target cells but not the wildtype KRAS G12 epitope, the corresponding TCR sequences were analyzed by next-generation sequencing (NGS).

The resulting HLA-A*11-restricted mKRAS G12D-specific TCRs identified as described above were reconstructed in silico, cloned in a retroviral vector, transduced into recipient T cells and further tested to confirm their specificity, sensitivity and safety in a transgenic setting.

### TCR sequencing and cloning

TCR-α and TCR-β chains of mKRAS₇₋₁₆ G12D-specific T cell clones were identified by NGS using an established standard protocol for analysis with the MiSeq system (Illumina). For sample preparation, the manufacturer's recommendations were followed using the Dynabeads mRNA DIRECT Kit (Thermo Fisher), SMART Scribe reverse transcriptase (Takara Bio), and the MiSeq V3 Kit (Illumina). To allow transgenic TCR staining and enrichment using anti-human Cβ1 TCR (JOVI.1) antibody, the identified TCR was reconstructed using human Cβ1 as constant beta chain (WO2022/038115A1). Constant regions of all TCR chains were minimally murinized to increase the stability of the TCRs [Reference 1]. The corresponding TCR chains were linked by a P2A peptide linker [Reference 2], codon-optimized (GeneArt, Thermo Fisher) [Reference 3], and cloned into the pES.12-6 self-inactivating gamma-retrovirus vector (TCR construct).
The UniTope tagged TCRs contain a nine amino acid long linear epitope (GEVPKDRFS, SEQ ID NO: 42) which was developed based on consensus amino acid sequences of human TRBV. The UniTope is inserted into the TCR beta constant chain position D85.5 (IMGT unique numbering) via a flanked "GSG" linker (see, for example SEQ ID NOs: 41 or 43) and can be effectively detected using the UniTope specific TraCR antibody.

Additionally, the chimeric co-stimulatory protein (CSP) PD1-41BB, consisting of the extracellular and transmembrane region of PD-1 (CD279, amino acids (aa) 21-191) and the intracellular domain of 4-1BB (CD137, aa 214-255), was added to the vector 5' of the TCR and separated by a T2A element. The sequence was codon-optimized (GeneArt, Thermo Fisher) [Reference 3], ordered from GeneArt as a synthetic DNA string (Thermo Fisher), and cloned into the pES.12-6 self-inactivating gamma-retrovirus vector (TCR + CSP construct).

### Transduction, expansion and enrichment of effector cells used in the functional assays to assess specificity, sensitivity and safety

Fresh blood from healthy donors was collected after informed consent. Peripheral blood mononuclear cells (PBMCs) were isolated from blood samples by density gradient centrifugation using a separating solution containing the synthetic copolymer Biocoll^{®} (BIOSELL) and Leucosep tubes (Greiner Bio-One). CD8⁺ T cells were enriched starting from PBMCs by MACS^{®} technology. The CD8⁺ T cell Isolation kit (Miltenyi) was used to isolate untouched CD8⁺ T cells, avoiding direct labeling of surface molecules on the desired cells that could interfere with downstream applications. The MACS separation was performed according to the manufacturer's protocol. Subsequently, CD8⁺ T cells carrying endogenous Cβ2-TCRs were isolated by depleting T cells carrying endogenous Cβ1-TCRs by MACS^{®} technology in combination with biotinylated anti-human Cβ1 TCR antibody (JOVI.1, Ancell).

The obtained CD8⁺ endogenous Cβ2-TCR⁺ T cells were activated using Dynabeads^{™} Human T-Activator CD3/CD28 for T Cell Expansion and Activation (Gibco). Cell culture medium (RPMI Medium 1640; Gibco) containing 10% human serum, 1x non-essential amino acids (NEAA), 2 mM L-glutamine, and 1 mM sodium-pyruvate, 100 U/ml Penicillin, 100 µg/ml Streptomycin, 10 mM HEPES and 50 µM 2-Mercaptoethanol was supplemented with 50 U/ml IL-2 (PROLEUKIN^{®} S, Clinigen).

Retroviral vector plasmids containing either the TCR alone or the TCR in combination with PD1-41BB CSP were co-transfected in HEK293FT cells with helper plasmids encoding Moloney MLV gag/pol and the GALV env gene to produce amphotropic retroviruses by using the TranslT^{®}-LT1 transfection reagent (Mirus). Retroviral supernatants were harvested 48 h and 72 h after transfection and 2 ml of filtered viral supernatant were added per well to Retronectin (Takara Bio)-coated 24-well plates that were centrifuged at 1000× g at 32 °C for 90 min. For transduction, 0.25-0.5 × 10⁶ activated T cells were added per 24-well. To reach higher transduction rates, the same T cells were transduced in the same manner a second time approximately 18-24 h after the first transduction; 18 to 24 h after transduction, T cells underwent a 10-day-phase of expansion after transfer into G-Rex 24-well plates (Wilson Wolf), during which they were supplied with fresh IL-2 at a final concentration of 50 U/ml every 2-3 days. The cells were harvested from the G-Rex plates, and transduction rates were determined before the cells were enriched to obtain either transgenic TCR⁺ or transgenic TCR⁺PD1-41BB⁺ high-purity T cell populations. Enriched cells were frozen in small aliquots and 10 days prior each functional assay cells were thawed and expanded by a rapid expansion protocol by addition of a re-stimulation cocktail, as previously described [Reference 4].

### Transduction, expansion and enrichment of effector cells used in functional assays to assess CD8 co-receptor independency

Fresh blood from healthy donors was collected after informed consent. PBMCs were isolated from blood samples by density gradient centrifugation using a separating solution containing the synthetic copolymer Biocoll^{®} (BIOSELL) and Leucosep tubes (Greiner Bio-One). CD3⁺ T cells were enriched starting from PBMCs by MACS^{®} technology. The Pan T cell Isolation Kit (Miltenyi) was used to isolate untouched CD3⁺ T cells, avoiding direct labeling of surface molecules on the desired cells that could interfere with subsequent applications. The MACS separation was performed according to the manufacturer's protocol. Subsequently, CD3⁺ T cells carrying endogenous Cβ2-TCRs were isolated by depleting T cells carrying endogenous Cβ1-TCRs by MACS^{®} technology in combination with biotinylated anti-human Cβ1 TCR antibody (JOVI.1, Ancell).

The obtained CD3⁺ endogenous Cβ2-TCR⁺ T cells were activated using Dynabeads^{™} Human T-Activator CD3/CD28 for T Cell Expansion and Activation (Gibco). Cell culture medium (RPMI Medium 1640; Gibco) containing 10% human serum, 1x non-essential amino acids (NEAA), 2 mM L-glutamine, 1 mM sodium-pyruvate, 100 U/ml Penicillin, 100 µg/ml Streptomycin, 10 mM HEPES and 50 µM 2-Mercaptoethanol was supplemented with 50 U/ml IL-2 (PROLEUKIN^{®} S, Clinigen).

Retroviral vector plasmids containing either the coding sequence for TCR alone or for the TCR in combination with PD1-41BB CSP were co-transfected in HEK293FT cells with helper plasmids encoding Moloney MLV gag/pol and the GALV env gene to produce amphotropic retroviruses by using the TranslT^{®}-LT1 transfection reagent (Mirus). Retroviral supernatants were harvested 48 h and 72 h after transfection and 2 ml of filtered viral supernatant were added per well to Retronectin (Takara Bio)-coated 24-well plates that were centrifuged at 1000× g at 32°C for 90 min. For transduction, 0.25-0.5 × 10⁶ activated T cells were added per 24-well. To reach higher transduction rates, the same T cells were transduced in the same manner a second time approximately 18-24 h after the first transduction; 18 to 24 h after transduction, T cells underwent a 10-day-phase of expansion after transfer into G-Rex 24-well plates (Wilson Wolf), during which they were supplied with fresh IL-2 at a final concentration of 50U/ml every 2-3 days. The cells were harvested from the G-Rex plates, and transduction rates were determined before the cells were used to evaluate intracellular IFN-γ stain in response to target stimulation.

To obtain high-purity CD8+ and CD4+ T cell populations expressing the transgenic TCR and PD1-41BB CSP to further test CD8-independency, the desired populations were separated by FACS from transduced CD3+ T cells. Enriched cells were expanded 10 days prior to the functional assay by a rapid expansion protocol through addition of a re-stimulation cocktail, as previously described (Riddell and Greenberg 1990, J. Immunol. Methods;128, 189-201).

### Transduction and expansion of Jurkat Biosensor cells used in functional assay to assess peptide specificity and sensitivity

The Jurkat Biosensor cell line is based on the Jurkat 76 T cell line (obtained from Mirjam H. M. Heemskerk's lab) lacking endogenous expression of TCR alpha and beta chains, which was engineered to stably express CD8 as well as inducible eGFP under the control of a nuclear factor of activated T cells (NFAT) responsive element (Jurkat 76_ieGFP_TCR -/-_CD8). Jurkat Biosensor cells were cultured in RPMI 1640 (+ 10% FBS, 2 mM L-glutamine, 1% NEAA, 100 U/ml Penicillin and 100 µg/ml Streptomycin) at 37°C and 5% CO2 and propagated as required.
Retroviral vector plasmids containing either the coding sequence for TCR alone or for the TCR in combination with PD1-41BB CSP were co-transfected in HEK293FT cells with helper plasmids encoding Moloney MLV gag/pol and the GALV env gene to produce amphotropic retroviruses by using the TranslT^{®}-LT1 transfection reagent (Mirus). Retroviral supernatants were harvested 48 h after transfection and 2 ml of filtered viral supernatant were added per well to Retronectin (Takara Bio)-coated 24-well plates that were centrifuged at 1000 × g at 32°C for 90 min. For transduction, 0.25 × 106 Jurkat Biosensor cells were added per 24-well. Transduced Jurkat Biosensor cells were expanded for 10 - 12 days before transduction rates were determined and eGFP expression in response to target stimulation was evaluated.

### Target cell culture and target cell preparations.

Tumor cell lines were transduced with HLA-A*11:01 molecules to allow surface expression of the desired HLA molecules when they were not endogenously positive for HLA-A*11:01. Additionally, to obtain robust and stable PDL1 expression, all selected tumor cell lines were subsequently further transduced with PDL1.

Retroviral vector plasmids containing the coding sequence for either HLA-A*11:01 molecules, PDL1 molecules, mKRAS G12D gene fragment linked to a reporter gene or KRAS G12 wild type gene fragment linked to a reporter gene were co-transfected in HEK293FT cells with helper plasmids encoding Moloney MLV gag/pol and the GALV env gene to produce amphotropic retroviruses by using the TranslT^{®}-LT1 transfection reagent (Mirus). Retroviral supernatants were harvested 48 h after transfection and 2 ml of filtered viral supernatant were added per well to Poly-L-Lysin (Cultrex) or Retronectin (Takara Bio)-coated 6-well plates that were centrifuged at 2000x g at 4°C for 2 h (Poly-L-Lysin) or 1000× g at 32°C for 90 min (Retronectin). For transduction, 1.0 -5.0 × 10⁵ tumor cells were added per 6-well.

Suspension cells were transduced in the same manner a second time approximately 24 h after the first hit. After transduction and expansion, transduced cells were sorted by FACS on the expression of the respective surface molecules using specific antibodies to obtain high purity populations.

Some tumor cell lines were further transduced with either NucLightRed or NucLight Green reagents (Sartorius) according to manufacturer's instructions. Transduced tumor cells underwent selection using puromycin after 3-5 days of transduction, depending on the fitness of cells.

AsPC-1 cells (ATCC) transduced with either HLA-A*11:01 only or HLA-A*11:01 and PDL1, BxPC-3 cells (DSMZ) transduced with either HLA-A*11:01 only or HLA-A*11:01 and PDL1, HuCC-T1 cells (Riken Biosource Center) transduced with PDL1 only, K562 cells (ATCC) transduced with either HLA-A*11:01 only or HLA-A*11:01 and PDL1 or with HLA-A*11:01 in combination with a gene fragment encoding either mKRAS G12D or KRAS wild type, Mel624.38 cells (kind gift of M. Panelli, National Institutes of Health) transduced with either HLA-A*11:01 only or HLA-A*11:01 and PDL1; SU.86.86 cells (ATCC) transduced with either HLA-A*11:01 only or HLA-A*11:01 and PDL1, were all maintained in RPMI 1640 containing 10% FBS, 2 mM L-glutamine, 1% NEAA, 100 U/ml Penicillin and 100 µg/ml Streptomycin at 37°C and 5% CO₂. HPAF-II cells (ATCC) transduced with either HLA-A*11:01 only or HLA-A*11:01 and PD-L1 were maintained in EMEM containing 10% FBS, 100 U/ml Penicillin and 100 µg/ml Streptomycin at 37°C and 5% CO₂. CL-40 (DSMZ) transduced with either HLA-A*11:01 only or HLA-A*11:01 and PDL1 were maintained in a 1:1 Mixture of DMEM and Ham's F12 media, 20% FBS, 100 U/ml Penicillin and 100 µg/ml Streptomycin at 37°C and 5% CO₂. KYSE-270 cells (DSMZ) transduced with either HLA-A*11:01 only or HLA-A*11:01 and PDL1 were maintained in a 1:1 Mixture of RPMI 1640 and Ham's F12 media, 2% FBS, 2 mM L-glutamine, 100 U/ml Penicillin and 100 µg/ml Streptomycin at 37°C and 5% CO₂. PANC-1 cells (ATCC) transduced with either HLA-A*11:01 only or HLA-A*11:01 and PDL1 were maintained in DMEM High Glucose containing 10% FBS, 2 mM L-glutamine, 1% NEAA, 1 mM sodium-pyruvate, 100 U/ml Penicillin and 100 µg/ml Streptomycin at 37°C and 5% CO₂.

All the above tumor cells - if they were also transduced with NucLightRed or NucLightGreen - were maintained in the stated media with the addition of puromycin for selection.

Primary HLA-A*11:01-positive normal human renal cortical epithelial cells (HRCEpC), primary HLA-A*11:01-positive normal human cardiac fibroblasts (HCF-c, PromoCell), primary HLA-A*11:01-positive normal human atrial cardiac fibroblasts (NHCF-A, Lonza), primary HLA-A*11:01-positive normal human lung fibroblasts (NHLF, Lonza), primary HLA-A*11:01-positive normal human hepatocytes (hepatocytes, Cytes Biotechnologies), induced pluripotent stem cell-derived HLA-A*11:01-transfected iCell Cardiomyocytes (Fujifilm Cellular Dynamics), induced pluripotent stem cell-derived HLA-A*11:01-transduced endothelial cells (Fujifilm Cellular Dynamics), induced pluripotent stem cell-derived HLA-A*11:01-transfected astrocytes (Fujifilm Cellular Dynamics) were cultured according to manufacturers' instructions.

LCL library consists of 63 LCLs (EBV-transformed B cell lines) which are suspension cell lines generated from B cells of healthy donors. Tested LCLs were either bought from a vendor (Fred Hutch Cancer Center) or generated in house. HLA typing of each individual LCL sample was analyzed by sequencing. LCLs were cultured in RPMI 1640 (+ 10% FCS; 1% NEAA; 2 mM L-glutamine 1 mM sodium-pyruvate, 100 U/ml Penicillin and 100 µg/ml Streptomycin) at 37°C and 5% CO₂ and propagated as required.

K562 library consists of 47 K562 cell samples transduced in house with individual HLA molecules. Successful transgenic expression of the introduced HLA molecules was confirmed by surface staining using an anti-human pan HLA class I-APC antibody (Invitrogen) and subsequent flow cytometry analysis. HLA-transduced K562 cells were cultured in RPMI 1640 (+ 10% FCS, 1% NEAA2 mM L-glutamine, 1 mM sodium-pyruvate, 100 U/ml Penicillin and 100 µg/ml Streptomycin) at 37°C and 5% CO₂ and propagated as required.

### Cell surface staining for flow cytometry and FACS

Cell surface marker staining was done according to standard protocols established previously [References 5,6]. The following fluorochrome-labelled antibodies were used according to manufacturers' instructions: anti-human CD8-PB/APC (BD), anti-human CD4-APC/FITC (BD), anti-human PD-1-APC/AF647 (Invitrogen), anti-human Cβ1 TCR-PE (BD). For the detection of HLA-A11, a biotinylated anti-human HLA-A11 antibody (USBio) was used and detected with a streptavidin-BV421 (BioLegend) conjugate. For the detection of the UniTope tag a specific monoclonal antibody was used. It was generated via immunization of rats with a peptide comprising the UniTope tag and followed by screening of hybridoma supernatants for specific binding. Subsequent sequencing of the antibody in the selected hybridoma supernatant revealed the antibody sequence and enabled the recombinant production by a commercial provider (Biointron Biological). The UniTope-specific antibody is hereafter referred to as TraCR. Flow cytometric analysis was performed on a MACSQuant X (Miltenyi Biotec) or a BD LSRFortessa Flow Cytometer (BD). For data analyses, FlowJo software (FlowJo) was used. Multimer staining was performed by using fluorochrome-labelled custom-synthesized tetramers (mKRAS7-16 G12D peptide/HLA-A*11:01, ImmunAware) according to manufacturer's instructions. Cell sorting was conducted using either FACSAria Fusion flow cytometer (BD) or a SH800S cell sorter (Sony Biotechnology).

### IFN-γ release assay.

IFN-γ release by T cells in response to different target cells was assessed in co-culture supernatants after 20 h of stimulation by enzyme-linked immunosorbent assay (ELISA). To assess specificity, sensitivity and safety, co-culture experiments were performed using different panels of target cells comprising tumor cell lines, mKRAS₇₋₁₆ G12D peptide-loaded targets, mKRAS₈₋₁₆ G12D peptide-loaded targets, KRAS₇₋₁₆ wild type peptide-loaded targets, KRAS₈₋₁₆ wild type peptide-loaded targets, LCL library, K562 library, mismatched peptide-loaded targets, *in vitro* transcribed RNA (*iv*tRNA)-transfected target cells and a panel of healthy cells. Peptides were obtained from Peptides&Elephants GmbH. mMESSAGE mMACHINE^{™} T7 kit (Thermo Fisher Scientific) was used to produce ivtRNA according to manufacturer's instructions.

For peptide loading, tumor target cells were harvested, counted and adjusted to 1×10⁶ cells/ml using appropriate medium in a tube. Subsequently, the required amount of peptide was added to the cells to reach the desired concentration. Cells were incubated for 1h and 30 minutes at 37°C and 5% CO₂. After incubation, the appropriate medium was added to a final volume of 15 ml. Cells were centrifuged (350 × g for five minutes) and the supernatant was discarded to remove unbound peptides. Cells were resuspended in fresh medium at the required concentration for the subsequent applications.

Healthy cells were tested with and without mKRAS₇₋₁₆G120 peptide. As positive controls, mKRAS₇₋₁₆ G12D peptide was added at the desired concentration into the wells containing pre-seeded healthy cells, incubated for 1h 30 minutes at 37°C and 5% CO₂ and washed with fresh media prior adding effector T cells.

For ivtRNA transfection, K562 target cells were harvested, counted and washed once with RPMI1640 medium without serum. Cells were adjusted at 2 - 3 × 10⁶ cells/200 µl in medium without serum and 200 µl of the cell suspension were transferred into a pre-cooled electroporation cuvette containing either 20 µg of ivtRNA or 20 µl of water (negative control, mock). The suspension was shortly mixed by pipetting and then quickly electroporated using the Gene Pulser Xcell^{™} device (BioRad). Electroporation was performed using an exponential protocol at 300 V and 150 µF. Immediately after electroporation, cells were transferred into the appropriate fresh medium and placed into the incubator at 37°C and 5% CO₂. After four hours, cells were counted and resuspended at adequate concentrations for further applications.

Co-cultures were performed at effector to target ratios between 2:1 and 1:2 (2 × 10⁴ cells effectors/96-well). Cytokine concentrations were determined using ELISA kits (Thermo Fisher; BD). The OD measurement was performed using the microplate reader Envision 2105 (Revvity). Background-corrected OD values were used for extrapolation using a third-degree polynomial.

### Cytotoxicity assay

Tumor cell lysis was assessed using either IncuCyte S3^{®} or Zoom^{®} device (Sartorius) and following manufacturer's recommendations for real-time quantitative live-cell imaging. Hence, tumor cells were transduced with NucLightRed (Sartorius) and seeded into 96-well flat-bottom plates 48 h prior to the addition of effector T cells at effector to target ratios between 1:3.5 and 1:0.4. Lysis of tumor cells was monitored by imaging the plates at regular intervals (every 4 h). The number of NucLightRed-labelled tumor cells over time was calculated using IncuCyte software (Sartorius).

To determine the cytotoxicity mediated by transduced CD8⁺ T cells in a 3-dimensional (3D) setting, NucLightRed-labeled or NucLightGreen-labeled tumor cells were seeded in 96-well ultra-low attachment plates (ULA; Corning) to form 3D spheroid structures. Three to five days after seeding the tumor cells, 2 × 10⁴ T cells were added to the ULA plates containing either the NucLightRed-labeled tumor spheroids or a combination of NucLightRed-labeled and NucLightGreen-labeled tumor spheroids (for bystander killing assay). Tumor cell lysis was monitored by imaging the plates every 4 h using either IncuCyte S3^{®} or Zoom^{®} device (Sartorius). For repeated challenges of the transduced T cells with tumor cells, pre-seeded tumor cell spheroids were transferred from ULA plates to the co-culture plates at different time points (day 0, 4, 7, 11). Lysis of tumor cell spheroids over time was determined using the spheroid setting in the IncuCyte software (Sartorius).

### Intracellular IFN-γ and IL-2 staining

TCR- or TCR+PD1-41BB-transduced CD3⁺ T cells (2 ×10⁴ cells/well) were co-cultured for 13-15 h with peptide-loaded (10⁻⁵ M) target cells at an E:T ratio of 1:1 in 96-well round bottom wells in the presence of Brefeldin A solution (10 µg/ml, Invitrogen). Frequency of IFN-γ⁺ CD8⁺ and IFN-γ⁺ CD4⁺ T cells was determined by multi-color flow cytometry using combinations of fluorochrome-conjugated antibodies anti-human CD3 PE-Cy7 (BD), anti-human CD4 FITC (BD), anti-human CD8 PerCP (BD), anti-human Cβ1 TCR-PE (BD), anti-human IFN-γ V450 (BD), anti-human IL-2-APC (BioLegend) and live/dead dye FVS780 (BD). After co-culture, cells were washed with PBS and incubated with antibodies at 2-8°C for 30 minutes. Cells were then washed (PBS), fixed (fixation buffer, Invitrogen), permeabilized (permeabilization buffer, Invitrogen) and stained with intracellular IFN-γ antibody at 2-8°C for 30 minutes. Cells were then washed and resuspended in stain buffer BSA (BD Pharmingen). Flow cytometry was performed using LSR Fortessa Flow Cytometer (BD). Data were analyzed using FlowJo software (FlowJo, BD). Intracellular IFN-γ and IL-2 staining were evaluated in gated CD8⁺ and CD4⁺ T cells.

### Biosensor assay

eGFP expression in transduced Jurkat biosensor cells was assessed after co-culture with K562 cells loaded with titrated amounts of mKRAS7-16 G12D peptide (Peptides&Elephants GmbH) ranging from 10-9 to 10-5 M. KRAS7-16 wild type peptide (Peptides&Elephants GmbH)-loaded K562 cells and unloaded K562 cells were used as negative controls. PMA (50 ng/ml; Sigma)/lonomycin (1 µg/ml; Sigma) stimulated Jurkat biosensor cells were used as positive control.

For peptide loading, K562 cells were harvested, counted and adjusted to 1x106 cells/ml using RPMI 1640 (+ 10% FBS, 2 mM L-glutamine, 1% NEAA, 100 U/ml Penicillin and 100 µg/ml Streptomycin). Subsequently, the required amount of peptide was added to the cells to reach the desired concentration. Cells were incubated for 1h and 30 minutes at 37°C and 5% CO2. After incubation, medium was added to a final volume of 15 ml. Cells were centrifuged (350 × g for five minutes) and the supernatant was discarded to remove unbound peptides. Cells were resuspended in fresh medium at 2.5 × 105 cell/ml and distributed to co-culture plates.

Co-culture was performed at an effector to target ratio of 2:1 (5 × 104 Jurkat biosensor cells/96 well) and eGFP expression was evaluated using the microplate reader Envision 2105 (Revvity).

### Results

### High and robust transgenic TCR and PD1-41BB CSP expression.

TCR 16 was expressed either alone or in combination with PD1-41BB CSP in a transgenic setting for vetting specificity, sensitivity and safety. TCR 16 was reconstructed using human constant regions (TRAC and TRBC01 - constant beta 1 region, Cβ1) with minimal murinization (minimal amino acid exchange in human TCR constant regions fosters improved pairing of transgenic TCRα and TCRβ chains over the mixed pairing with the T cell's endogenous TCR and function of TCR gene-modified T cells) and cloned either alone or in combination with PD1-41BB CSP into a retroviral vector. CD8-enriched/Cβ1-depleted T cells derived from healthy donors were activated using anti-CD3/CD28 beads in the presence of IL-2 and transduced with retroviral particles containing either the sequence of the TCR alone or the sequence of the TCR in combination with PD1-41BB CSP. Untransduced CD8⁺ T cells were prepared in the same manner and used as controls. After transduction and expansion, transduced CD8⁺ T cells were enriched using anti-TCR Cβ1 antibody by fluorescence-activated cell sorting (FACS). After enrichment and rapid expansion protocol, effector CD8⁺ T cells were stained with anti-TCR Cβ1, anti-PD1 and anti-CD8 antibodies and analyzed by flow cytometry to assess transduction efficiency and expression levels of the transgenes.

Binding of TCR Cβ1 antibody to TCR 16-transduced CD8⁺ T cells as well as to TCR 16 + PD1-41BB-transduced CD8⁺ T cells indicated efficient transduction with high expression levels of the transgenic TCR (**Figure 1**). Binding of PD1 antibody to TCR 16 + PD1-41BB-transduced CD8⁺ T cells indicated efficient transduction with high expression levels also of the transgenic PD1-41BB CSP which correlated with the expression of the transgenic TCR. Only very low endogenous PD1 expression was detected for TCR 16-transduced CD8⁺ T cells and untransduced CD8⁺ T cells. These results demonstrated that co-expression of the PD1-41BB CSP and the transgenic TCR was possible in CD8⁺ T cells and resulted in similar expression of both constructs on the cell surface.

### High and robust mKRAS₇₋₁₆ G12D (10-mer) multimer binding independently of PD1-41BB CSP co-expression

Multimer binding is the first proof of transgenic TCR functionality. Therefore, after enrichment and rapid expansion protocol, both TCR 16-transduced CD8⁺ T cells and TCR 16 + PD1-41BB-transduced CD8⁺ T cells were stained with mKRAS₇₋₁₆ G12D (10-mer) HLA-A*11:01 multimer and subsequently analyzed by flow cytometry. Results showed high and robust binding of TCR 16 independently of PD1-41BB CSP co-expression to HLA-A*11:01 multimer loaded with mKRAS₇₋₁₆G120 (10-mer) peptide (Figure 2).

### Exquisite mKRAS₇₋₁₆G12D specificity with no KRAS wild type recognition independently of PD1-41BB CSP co-expression

To demonstrate mKRAS₇₋₁₆ G12D peptide specificity, both TCR 16-transduced CD8⁺ T cells and TCR 16 + PD1-41BB-transduced CD8⁺ T cells were co-cultured with K562 cells transduced with HLA-A*11:01 (hereafter designated as K562_A11) loaded with either mKRAS₇₋₁₆ G12D peptide or KRAS₇₋₁₆ wild type peptide at high concentrations (10⁻⁵ M). Additionally, CD8⁺ T cell samples were also co-cultured with K562_A11 cells transduced with either a transgene (gene fragment of 90 base pairs (bp)) encoding mKRAS G12D or a transgene (gene fragment of 90 bp) encoding KRAS wild type. Transduced targets served to assess the capacity of TCR 16 to recognize KRAS epitopes when processed and presented by internal cellular pathways. Unloaded targets as well as untransduced CD8⁺ T cells were included as internal controls. After 20 hours (h) of co-culture, an ELISA was performed to evaluate IFN-γ secretion by T cells.

Both TCR 16-transduced CD8⁺ T cells and TCR 16 + PD1-41BB-transduced CD8⁺ T cells recognized only K562_A11 cells when either loaded with mKRAS₇₋₁₆ G12D peptide or transduced with mKRAS G12D transgene. No IFN-γ was observed in response to target cells overexpressing KRAS wild type (**Figure 3A**). These results showed mKRAS G12D peptide specificity of TCR 16 with no recognition of KRAS wild type and its capacity to recognize the epitope when processed and presented by cellular internal pathways. These TCR properties were not influenced by co-expression of PD1-41BB CSP.

To further evaluate exquisite target epitope specificity of TCR 16, recognition of three additional well described KRAS G12 mutations (G12V, G12C, G12R) was investigated.

TCR 16- and TCR 16 + PD1-41BB-transduced CD8⁺ T cells were co-cultured with target cells either individually loaded with mKRAS₇₋₁₆ G12V, mKRAS₇₋₁₆ G12C, mKRAS₇₋₁₆ G12R peptides (10⁻⁵ M) or individually transfected with ivtRNA encoding gene fragments (~402 bp fragment spanning the specific mutation at position 12 and linked to GFP) of either mKRAS G12V, mKRAS G12C or mKRAS G12R. Loading of mKRAS₇₋₁₆G120 peptide (10⁻⁵ M) and transfection with ivtRNA encoding mKRAS G12D gene fragment (~402 bp fragment spanning the specific mutation at position 12 and linked to GFP) served as positive target controls, whereas loading of KRAS₇₋₁₆ wild type peptide (10⁻⁵ M) and transfection with ivtRNA encoding KRAS wild type gene fragment (~402 bp fragment spanning the position 12 and linked to GFP) were included as negative target controls. In addition, untransduced CD8⁺ T cells and water electroporation of target cells served as internal controls. The transfection efficiency of target cells was evaluated by flow cytometry analyzing GFP signals. IFN-γ secretion was evaluated by ELISA 20 h after setting up the co-culture. This assessment was performed in two independent assays: in assay 1 targets cells prepared as previously described were co-cultured with TCR16-transduced T cells, while in assay 2 target cells prepared as previously described were co-cultured with TCR16+PD1-41BB-transduced T cells.

All *ivt*RNA-transfected target cells showed GFP expression, confirming successful transfection (**Figure 3B**). Both TCR 16-transduced CD8⁺ T cells and TCR 16 + PD1-41BB-transduced CD8⁺ T cells recognized only target cells when either loaded with mKRAS₇₋₁₆G12D peptide or transfected with mKRASG12D ivtRNA. No IFN-γ was observed in response to target cells expressing either other mKRAS variants or KRAS wild type (**Figure 3C** **and** **3D**). These findings further confirmed the exquisite mKRAS G12D specificity of TCR 16 irrespective of PD1-41 BB CSP co-expression.

### High sensitivity for mKRAS₇₋₁₆ G12D (10-mer) peptide independently of PD1-41BB CSP co-expression.

Functional avidity refers to the accumulated strength of multiple affinities of individual non-covalent binding interactions, such as the transgenic TCR and the peptide-MHC complex. As such the functional avidity of TCR-transduced effector T cells serves as a measure of peptide sensitivity. TCRs conferring high peptide sensitivity can recognize lower amounts of peptide. To investigate peptide sensitivity of TCR 16-transgenic CD8⁺ T cells with and without PD1-41 BB CSP, a co-culture experiment was performed using K562_A11 cells loaded with titrated amounts of mKRAS₇₋₁₆ G12D (10-mer) peptide (10⁻⁵ M to 10⁻¹² M). Additionally, titrated amounts of mKRAS₈₋₁₆ G12D (9-mer) peptide (10⁻⁵ M to 10⁻¹² M) were tested for direct comparison of peptide sensitivity for the two peptide length variants. Target cells were also loaded with KRAS₇₋₁₆ wild type (10-mer) peptide and KRAS₈₋₁₆ wild type (9-mer) peptide at 10⁻⁵ M as internal controls. After 20 h of co-culture, an ELISA was performed to evaluate IFN-γ secretion by T cells. Maximal IFN-γ release per effector cell sample was set to 100%. Based on this, the relative IFN-γ release was obtained and used in a non-linear regression analysis.

TCR 16 independently of PD1-41 BB CSP co-expression showed high sensitivity for mKRAS₇₋₁₆ G120 (10-mer) peptide, whereas sensitivity was much lower towards mKRAS₈₋₁₆ G120 (9-mer) peptide (**Figure 4**). These findings confirmed the preferred binding of TCR 16 to mKRAS₇₋₁₆G120 (10-mer) peptide requiring only a very low amount of 10-mer peptide for activation.

Importantly, the 10-mer peptide length variant has been described as the most abundant and stable KRAS G12D peptide variant presented on HLA-A*11:01 molecules on tumor cells by mass spectrometry analyses (Jaewon Choi et al. 2021, Cell Rep Methods*;* Adham S. Bear et al. 2021, Nature Communications).

### Peptide-specific restriction to four different HLA-A*11 subtypes independently of PD1-41BB CSP co-expression

Overall, five HLA*11 subtypes have been described (HLA-A*11:01, HLA-A*11:02, HLA-A*11:03, HLA-A*11:04, HLA-A*11:12) which show only very slight differences in their amino acid sequences. A specific TCR that recognizes its cognate peptide in the context of HLA-A*11:01 might also recognize the peptide presented by another HLA-A*11 subtype.

To determine target peptide-specific HLA restriction and potentially broaden the patient cohort, TCR 16 was characterized in the context of the different HLA-A*11 subtypes. CD8⁺ T cells expressing either no transgenic TCR, the transgenic TCR 16 only or the combination of TCR 16 and PD1-41BB CSP were co-cultured with HLA-transduced K562 cell samples. Each K562 cell sample expressed individual transgenic HLA molecules previously described and was tested unloaded as well as after mKRAS₇₋₁₆G120 peptide loading (10⁻⁵ M).

Untransduced K562 cells served as internal controls. IFN-γ release was evaluated by ELISA 20 h after setting up the co-culture.

TCR 16-transduced CD8⁺ T cells recognized mKRAS₇₋₁₆ G12D peptide presented by HLA molecules encoded by HLA-A*11:02, HLA-A*11:03 and HLA-A*11:12 at similar levels compared to HLA-A*11:01 (**Figure 5**). This recognition pattern was not altered by the co-expression of PD1-41 BB CSP. No recognition of any unloaded HLA-transduced K562 sample was observed. In conclusion, these results demonstrated that TCR 16 presented peptide-specific restriction to four different HLA-A*11 subtypes independently of PD1-41BB CSP co-expression.

### High and specific IFN-γ and IL-2 secretion in response to mKRAS G12D-positive tumor cells independently of PD1-41BB CSP co-expression

To be potentially useful in a clinical setting, it is essential that T cells expressing the candidate TCR are able to specifically recognize tumor cells expressing the target antigen. A standard way to assess tumor cell recognition by TCR-transgenic T cells is the evaluation of cytokine release (e.g. IFN-γ and IL-2 secretion) by T cells upon target antigen-specific stimulation.

Tumor cell lines derived from various cancers (pancreatic, colorectal, bile duct tumors) expressing mKRAS G12D at different levels (two homozygous cell lines (AsPC-1, SU.86.86), three heterozygous cell lines (PANC-1, HPAF-II, HuCC-T1), one unknown (CL-40)) were selected as target cells for this co-culture experiment. In addition, three tumor cell lines expressing KRAS wild type (MeI624.38, KYSE-270, BxPC-3) served as mKRAS G12D-negative tumor target cells. All tumor cell lines, except for HuCC-T1 and KYSE-270 cell lines, were stably transduced with HLA-A*11:01-encoding transgene to allow surface expression of the desired HLA molecules.

The co-culture experiment was set up by using the described tumor cell lines and TCR 16-transduced CD8⁺ T cells with and without PD1-41BB CSP Untransduced CD8⁺ T cells served as negative control. IFN-γ secretion was assessed 20 h after setting up the co-culture.

IFN-γ release was observed only when TCR 16- and TCR16+PD1-41BB-transduced CD8⁺ T cells were stimulated with mKRAS G12D-positive tumor cells (Figure 6A). Importantly, no recognition of any KRAS wild type tumor cells was observed.

Additionally, a similar co-culture experiment was set up with a reduced tumor cell panel, three mKRAS G12D positive (SU.86.86, HPAF-II, CL-40) and three KRAS wild type tumor cell lines (MeI624.38, KYSE-270, BxPC-3), to assess capacity of TCR 16-transduced CD8⁺ T cells (with and without PD1-41BB CSP) to also release IL-2 after antigen-specific activation. Untransduced CD8⁺ T cells served as negative control. IL-2 secretion was assessed 20 h after setting up the co-culture. Similarly to the results obtained for IFN-γ, IL-2 release was observed only when TCR 16- and TCR 16 + PD1-41BB-transduced CD8⁺ T cells were stimulated with mKRAS G12D-positive tumor cells (**Figure 6B**). No IL-2 secretion was detected in response to any KRAS wild type tumor cells.

Overall, these findings confirmed mKRAS G12D specificity of TCR 16 independently of PD1-41BB CSP co-expression and its capacity to be activated and subsequently mediate release of both IFN-γ and IL-2 in response to tumor cells expressing endogenous levels of mKRAS G12D.

### Strong and specific cytotoxic response against mKRAS G12D-positive tumor cells independently of PD1-41BB CSP co-expression even after multiple tumor challenges

Tumor cell recognition by TCR-transgenic CD8⁺ T cells can be addressed not only by evaluating cytokine release upon target antigen-specific stimulation, but also by testing the cytotoxic response against tumor cells expressing the target tumor antigen.

To evaluate cytotoxic capacity of TCR 16- and TCR 16 + PD1-41BB-transduced CD8⁺ T cells, both effector cells and untransduced CD8⁺ T cells were co-cultured with tumor cell lines and monitored over time by a live-cell imaging system. Some of the previously described tumor cell lines (four mKRAS G12D-positive (AsPC-1, HPAF-II, CL-40, SU.86.86) and two KRAS wild type cell lines (Mel624.38, KYSE-270)) were used as target cells for this assay. All tumor cells stably expressed a red fluorescent protein for tumor cell tracking by a live-cell imaging system. Cytotoxicity against tumor cells was determined by a reduction in the absolute number of red fluorescent tumor cells over time.

Co-cultures containing TCR 16- as well as TCR 16 + PD1-41BB-transduced CD8⁺ T cells and mKRAS G12D-positive tumor cells showed decreasing absolute numbers of red fluorescent tumor cells, indicating a robust and strong cytotoxic response (**Figure 7A**). In contrast, proliferation and expansion of KRAS G12D-positive tumor cells were seen in co-cultures with the untransduced CD8⁺ T cells as well as when targets were kept alone in the wells (only targets). Importantly, no reduction in numbers of KRAS wild type tumor cells was observed in any co-cultures (**Figure 7B**). These results showed the exclusive mKRAS G12D-specificity of TCR 16 cytotoxic response irrespective of PD1-4BB CSP co-expression.

To investigate T cell fitness and resilience, effector cells were co-cultured with 3-dimensional (3D) tumor cell spheroids and multiple challenges with fresh tumor cell spheroids were performed by transferring the tumor cells to the co-culture plates at different time points. These 3D tumor cell spheroids served as an *in vitro* model for solid tumors. Out of the previously described tumor cell panel, two mKRAS G12D-positive tumor cells (HPAF-II, SU.86.86) were selected as target cells for this assay. All tumor cells expressed a red fluorescent protein for cell tracking over time. Cytotoxicity against tumor cell spheroids was determined by the reduction of red fluorescence signal over time using a live-cell imaging system. Robust and strong cytotoxic responses mediated by both TCR 16- and TCR 16 + PD1-41BB-transduced CD8⁺ T cells were observed against mKRAS G12D-positive tumor cell spheroids, which were maintained in the challenging environment with repeated exposure to tumor cells (Figure 7C).

### TCR-gated control of PD1-41BB CSP activation in TCR + PD1-41BB-transduced T cells

PDL1 is expressed on tumor cells as well as on various cell types within the tumor microenvironment and binds to PD1 expressed on the surface of activated T cells, thereby inducing T cell exhaustion and mediating tumor immune escape. PD1-41BB CSP is designed to turn the inhibitory signal mediated via the PD1/PDL1 axis into a costimulatory response. The CSP-mediated costimulatory signal is TCR-gated, whereby costimulation can only occur if the specific peptide-HLA complex present on target cells interacts with the recombinant TCR expressed in T cells.

To demonstrate that PD1-41BB CSP activation in TCR 16 + PD1-41BB-transduced T cells is TCR-gated and cannot be triggered via PDL1 in the absence of target antigen, PDL1 molecules were overexpressed in target cells via retroviral transduction to obtain stable and robust expression and subsequently used to evaluate maximum effect mediated by PD1-41BB CSP in co-culture experiments.

TCR 16- and TCR 16 + PD1-41BB-transduced CD8⁺ T cells were tested with PDL1-transduced K562_A11 cells (hereafter designated as K562_A11_PDL1) transfected with either *ivt*RNA encoding a fragment of mKRAS G12D gene (~402 bp) or *ivt*RNA encoding a fragment of KRAS wild type gene (~402 bp). K562_A11_PDL1 cells electroporated with water (mock) as well as untransduced CD8⁺ T cells were included as negative controls. After 20 h of co-culture, an ELISA was performed to evaluate IFN-γ secretion by T cells.

Both TCR 16- and TCR 16 + PD1-41BB-transduced CD8⁺ T cells recognized only K562_A11_PDL1 cells when transfected with mKRASG12D *ivt*RNA. Importantly, no IFN-γ was observed in response to K562_A11_PDL1 cells transfected with KRAS wild type *ivt*RNA, showing that TCR 16 + PD1-41BB-transduced CD8⁺ T cells do not get activated via PDL1-overexpressing target cells in the absence of target antigen (**Figure 8A**).

To further demonstrate that PD1-41BB activation in TCR+PD1-41BB-transduced T cells is TCR-gated, an *in vitro* co-culture experiment was set up to evaluate bystander killing of KRAS wild type PDL1-overexpressing tumor cell spheroids in the presence of ongoing lysis of mKRAS G12D-positive PDL1-overexpressing tumor cell spheroids by CD8⁺ T cells transduced with either TCR 16 or TCR 16 + PD1-41BB.

Two tumor cell lines (one mKRAS G12D-positive and one KRAS wild type) expressing high levels of transgenic PDL1 molecules were additionally transduced to express either a red fluorescent protein (HPAF-II cells, mKRAS G12D-positive) or a green fluorescent protein (BxPC-3, KRAS wild type) for tumor cell tracking by a live-cell imaging system. Red and green fluorescent tumor cells were seeded individually and incubated for three days to allow 3D tumor cell spheroid formation. On the day prior co-culture, red and green fluorescent tumor cell spheroids were combined and effector cells were added. Untransduced CD8⁺ T cells served as internal negative controls. The co-culture plates were monitored over time by a live-cell imaging system.

Cytotoxicity against mKRAS G12D-positive and KRAS wild type tumor cell spheroids was detected by the reduction of red fluorescence signal and green fluorescence signal over time, respectively.

Robust and strong cytotoxic responses mediated by both TCR 16- and TCR 16 + PD1-41BB-transduced CD8⁺ T cells were observed against mKRAS G12D-positive tumor cell spheroids (reduction of red fluorescence signal) (**Figure 8B**). By contrast, activated TCR 16- and TCR 16 + PD1-41BB-transduced T cells did not show any bystander killing of KRAS wild type tumor cell spheroids (stable green fluorescent signal over time) carrying PDL1 in the presence of antigen positive tumor cells.

Overall, these results confirmed TCR-gated functionality and a favorable safety profile of PD1-41BB CSP-armored TCR 16-transduced T cells.

### No signs of target peptide-independent cross-recognition of frequent HLA allotypes independently of PD1-41BB CSP co-expression

To investigate potential target peptide-independent cross-recognition of frequent HLA allotypes, two cellular libraries were used as target cells in a co-culture experiment. The first library comprises 63 lymphoblastoid cell lines (LCLs, LCL library) and the second one comprises 47 K562 cell samples transduced with individual HLA molecules (K562 library). All together these samples cover the most frequent HLA-A, -B and -C allotypes in Asian, US and European populations.

TCR 16-transduced CD8⁺ T cells, TCR 16 + PD1-41BB-transduced CD8⁺ T cells and untransduced CD8⁺ T cells were co-cultured with the two libraries. K562_A11 cells loaded with mKRAS₇₋₁₆ G12D peptide (10⁻⁵ M) served as internal positive controls. IFN-γ secretion was evaluated by ELISA 20 h after setting up the co-culture.

No recognition of any LCL and K562 sample was observed by CD8⁺ T cells transduced with either TCR 16 alone or TCR 16 + PD1-41BB CSP (**Figure 9A** **and** **9B**). Only positive controls led to activation of TCR 16-transgenic CD8⁺ T cells with and without PD1-41BB CSP. Importantly, untransduced CD8⁺ T cells released IFN-γ to some extent in response to certain LCL and K562 cells which was potentially due to the reactivity of the endogenous TCRs either to allogeneic HLA molecules expressed on LCL/K562 cells or to peptides derived from Epstein-Barr-Virus (which was used to immortalize B cells and generate the required LCL) expressed in LCL samples. The levels of IFN-γ released by untransduced T cells in response to individual target cells were considered as individual cutoff values for the corresponding co-culture with TCR 16 / TCR 16 + PD1-41BB CSP-transduced T cells. None of the TCR 16 / TCR16 + PD1-41BB-expressing effectors released higher levels of IFN-γ than the corresponding untransduced T cells, confirming no recognition of any LCL/K562 cells due to the transgenic TCR 16 / TCR 16 + PD1-41BB.

Overall, these data demonstrated the absence of target peptide-independent cross-recognition of frequent HLA allotypes mediated by TCR 16 independently of PD1-41BB CSP co-expression.

### No signs of off-target toxicity against healthy cell types independently of PD1-41BB CSP co-expression

The aim of this experiment was to assess potential off-target toxicities that could be caused by TCR 16-transduced CD8⁺ T cells independently of PD1-41BB CSP co-expression. HLA-A*11:01-positive primary healthy cells and induced pluripotent stem cell (iPS)-derived cell lines representing essential organs were tested for recognition by TCR-transduced T cells. These healthy cell types were either endogenously or transgenically expressing HLA-A*11:01 molecules on their cell surface. In line with the properties of the individual targets, healthy cells were seeded one to seven days prior to start of the co-culture at cell densities as per manufacturer's instructions and cultivated in monolayers in flat bottom wells. Target cells loaded with mKRAS₇₋₁₆ G12D peptide (10⁻⁵ M) were included as internal positive controls. IFN-γ secretion was evaluated by ELISA 20 h after setting up the co-culture.

None of the tested target cells was recognized by TCR 16-transduced CD8⁺ T cells independently of the presence of PD1-41 BB CSP (**Figure 10**). Only peptide-loaded target cells (positive controls) led to activation of T cells and subsequent IFN-γ release. These results demonstrated no signs of off-target toxicities caused by the recognition of endogenous peptides.

### No signs of off-target toxicity against a library of self-peptides with a high sequence similarity to the target peptide independently of PD1-41BB CSP co-expression

Off-target toxicities can arise when TCRs recognize not only the specific target peptide but also other peptides that possess a high sequence homology with the original peptide. To identify peptide candidates that show a high sequence similarity with the specific target peptide and are likely to be found in the processed HLA-A*11:01-presented human peptidome, computational tools have to be employed to generate a list of potentially cross-recognized peptides that can subsequently be synthesized and tested for recognition by TCR-transduced effector T cells in co-culture experiments.

By using the computational tool Expitope 2.0^{®} (Jaravine V, Mösch A, Raffegerst S, et al. 2017; BMC Cancer; 17(1):892), 240 candidate mismatched peptides could be identified containing up to 4 amino acid differences compared to the target mKRAS₇₋₁₆G12D peptide.

A co-culture experiment was performed using target cells loaded with the selected 240 mismatched peptides individually (two concentrations were tested: 10⁻⁵ M and 10⁻⁶ M). K562_A11 were used as target cells in a co-culture with TCR 16-transduced T cells, while K562_A11_PDL1 were used as target cells in co-culture with TCR 16 + PD1-41BB-transduced T cells. Target cells loaded with mKRAS₇₋₁₆ G12D peptide served as positive controls, while unloaded targets served as negative controls. TCR 16-transduced CD8⁺ T cells, TCR 16+PD1-41BB-transduced CD8⁺ T cells as well as untransduced CD8⁺ T cells were utilized as effector cells. IFN-γ secretion was assessed by ELISA 20 h after co-culture.

The majority of the tested peptides did not lead to activation of any effector T cells, showing they were not likely to cause off-target toxicities. However, 7 out of 240 tested mismatched peptides were recognized by TCR 16-transduced CD8⁺ T cells and TCR 16 + PD1-41BB-transduced CD8⁺ T cells to some degree at the tested peptide concentrations and therefore were further investigated. Since exogenous loading of peptides at high concentration does not necessarily translate into physiological recognition of endogenously processed and presented peptides, the identified recognized peptides were tested for their potential to induce IFN-γ release by TCR (± PD1-41 BB)-transgenic CD8⁺ T cells when the peptides were delivered intracellularly as ivtRNA and thus undergoing translation, processing and presentation by the endogenous cellular machinery of target cells. Midi-gene fragments were designed to cover ~200 bp of the original gene sequence 5' and 3' of the respective peptide-coding region and linked to a GFP reporter gene. Cross-recognized peptides can be de-risked if they are not processed from the original protein, fail to be loaded on HLA molecules endogenously after overexpression in target cells or are not able to trigger activation of TCR (± PD1-41BB)-transgenic CD8⁺ T cells.

Co-culture experiments were established by using TCR 16- and TCR 16 + PD1-41BB-transduced CD8⁺ T cells and mismatched peptide midi-gene fragment ivtRNA-transfected target cells (K562_A11 were used as target cells in co-culture with TCR 16-transduced T cells, while K562_A11_PDL1 were used as target cells in co-culture with TCR 16 + PD1-41BB-transduced T cells). Target cells transfected with mKRAS G12D midi-gene fragment ivtRNA (fragment spanning mKRAS₇₋₁₆ G12D peptide-coding region and linked to GFP) were included as positive control. Water electroporation of target cells served as negative control. The transfection efficiency of target cells was evaluated by flow cytometry analyzing GFP signals. IFN-γ secretion was evaluated by ELISA 20 h after setting up the co-culture.

All ivtRNA-transfected target cells showed GFP expression, confirming successful transfection and translation of the ivtRNA (**Figure 11A** **and** **11B**). As expected, target cells transfected with the ivtRNA construct encoding mKRAS G12D peptide were recognized by both TCR 16- and TCR 16+PD1-41BB-transgenic CD8⁺ T cells. By contrast, none of the intracellularly processed mismatched peptides were recognized by any effector T cells (**Figure 11C** **and** **11D**). Therefore, all mismatched peptides were successfully de-risked indicating that they were not likely to cause off-target toxicities.

### Signs of TCR functionality in CD4⁺ T cells independently of PD1-41BB CSP co-expression

The CD8 co-receptor is known to directly bind to HLA class I molecules and to be critical for the development of CD8⁺ T cells. The CD8 co-receptor stabilizes the binding of a TCR to the HLA-peptide complex and facilitates early events in the TCR signaling cascade. To evaluate the impact of the CD8 co-receptor on TCR functionality, either TCR 16 alone or TCR 16 in combination with PD1-41BB CSP was transduced in CD3⁺ T cells derived from healthy donors (mixed population containing both CD4⁺ and CD8⁺ T cells) and utilized as effector samples in a co-culture experiment. Corresponding untransduced CD3⁺ T cells served as controls. Co-culture experiments were established using HLA-A*11:01-positive target cells loaded with either KRAS₇₋₁₆ wild type (10⁻⁵ M) or mKRAS₇₋₁₆ G12D peptide (10⁻⁵ M) as target cells (K562_A11 were used as target cells in co-culture with TCR 16-transduced CD3⁺ T cells, while K562_A11_PDL1 were used as target cells in co-culture with TCR 16 + PD1-41BB-transduced CD3⁺ T cells). After overnight incubation in the presence of Brefeldin A, co-culture samples were assessed for CD3, CD8, CD4, Cβ1 surface expression as well as for IFN-γ and IL-2 intracellular accumulation by flow cytometry. Samples were analyzed to assess IFN-γ-positive and IL-2-positive cells contained in CD8⁺ and in CD4⁺ T cell populations.

As expected, TCR 16 as well as TCR 16 + PD1-41BB CSP expressed in CD8⁺ T cells were activated and resulted in a high and robust fraction of IFN-γ-positive and IL-2-positive cells after co-culture with targets loaded with mKRAS₇₋₁₆ G12D peptide (**Figure 12A**).

No recognition of target cells loaded with KRAS wild type peptide was observed. Interestingly, TCR 16 expressed in CD4⁺ T cells, independently of PD1-41BB CSP co-expression, also recognized mKRAS₇₋₁₆ G12D peptide-loaded target cells (high fraction of IFN-γ-positive and IL-2-positive cells), showing that the functionality of the transgenic TCR 16 (with and without PD1-41BB CSP) was not dependent on the presence of the CD8 co-receptor (**Figure 12B**).

### Co-expression of PD1-41BB CSP enhanced the specific release of IFN-γ in response to mKRAS G12D-positive tumor cells overexpressing PDL1

The interaction of PDL1 on tumor cells with PD1 on T cells usually leads to an inhibitory signal that reduces T cell activity. PD1-41BB CSP is designed to reverse this signal and result in increased T cell reactivity when the transgenic TCR binds to its cognate peptide-HLA complex.

To test the impact of PD1-41BB CSP co-expression on the T cell reactivity, effector T cells expressing either TCR 16 alone or TCR 16 in combination with PD1-41BB CSP were co-cultured with a panel of tumor cells expressing either mKRAS G12D or KRAS wild type. To obtain robust and stable PDL1 expression, all selected tumor cell lines were transduced with PDL1. mKRAS G12D-positive tumor cell lines transduced with PDL1 served to evaluate maximum effect mediated by PD1-41BB CSP in an antigen specific manner. On the other hand, KRAS wild type tumor cell lines transduced with PDL1 served as negative controls to ensure that co-expression of PD1-41BB CSP did not negatively impact the specificity of the transgenic TCR-transduced T cells. Untransduced CD8⁺ T cells served as negative control. IFN-γ secretion was assessed 20 h after setting up the co-culture.Overall, it was observed that co-expression of PD1-41BB CSP enhanced the release of IFN-γ in response to PDL1-overexpressing mKRAS G12D-positive tumor cells, whereas PDL1-overexpressing KRAS wild type tumor cells remained not recognized by any of the tested T cells (**Figure 13**). These findings confirmed that the co-expression of PD1-41BB CSP improved CD8⁺ T cell reactivity in response to PDL1-positive tumor cells and it did not impact the specificity of TCR 16.

### Cytotoxic response against tumor cells mediated by TCR-transduced CD4⁺ T cells independently of PD1-41BB CSP co-expression

To further investigate CD8 co-receptor independence, TCR 16 alone as well as in combination with PD1-41BB CSP were individually expressed in both CD8⁺ T cells and CD4⁺ T cells. CD8⁺ and CD4⁺ TCR-expressing T cells were then enriched by fluorescence-activated cell sorting (FACS). After enrichment and rapid expansion protocol, CD8/CD4 purity and transgenic TCR expression (anti-Cb1 antibody) was verified by flow cytometry analysis. Additionally, T cell samples were stained with mKRAS₇₋₁₆ G12D (10-mer) HLA-A*11:01 multimer and analyzed by flow cytometry. The results showed high and robust 10-mer multimer binding by TCR 16 alone as well as in combination with PD1-41BB CSP when expressed not only in CD8⁺ T cells but also in CD4⁺ T cells, confirming signs of TCR functionality in CD4⁺ T cells (**Figure 14A**).

Enriched CD8⁺ and CD4⁺ T cell samples were co-cultured with either mKRAS G12D-positive or KRAS wild type tumor cell spheroids. Tumor spheroids expressed a red fluorescent protein for cell tracking and quantification of target cell killing over time. Red fluorescent tumor cells were seeded three days prior to the start of the co-culture experiment. After addition of effector cells, the co-culture plates were monitored over time by a live-cell imaging system to evaluate the reduction of red fluorescent tumor cell spheroids, indicating cytotoxicity mediated by TCR ± PD1-41BB-transgenic T cells. TCR-transduced CD4⁺ and CD8⁺ T cells exhibited comparable capacities to lyse mKRAS G12D-positive tumor cell spheroids. In contrast, no cytotoxic activity against KRAS wild type tumor spheroids was observed, proving that the reactivity of TCR-transduced CD4⁺ T cells is also TCR-gated (**Figure 14B**).

These results confirmed the functionality of the transgenic TCR in CD4⁺ T cells irrespective of PD1-41BB co-expression, hence potentially broadening the therapeutically active cell population in a non-enriched clinical T cell product.

### Transgenic TCR and PD1-41BB CSP expression is not affected by UniTope presence

To allow identification and precise tracking of TCR 16 in research and clinical settings, TCR 16 was combined with the UniTope epitope tag. The UniTope is a nine amino acid long linear epitope (GEVPKDRFS, SEQ ID NO: 42) inserted into the TCR beta constant chain which can be effectively detected using an antibody that specifically binds to the Unitope tag (TraCR). Here, the influence of the UniTope insertion on TCR 16 expression and functionality was analyzed by expressing the UniTope tagged TCR 16 construct (± PD1-41BB CSP co-expression) in an engineered Jurkat 76_ieGFP_TCR -/-_CD8 reporter cell line (hereafter termed Jurkat biosensor cell line) and comparing it to the untagged TCR 16 construct (± PD1-41BB CSP co-expression). Since Jurkat biosensor cells lack expression of an endogenous TCR, transgenic TCR expression can be directly assessed by analyzing TCR Cβ1 expression. To this end, transduced Jurkat biosensor cells were stained with anti-TCR Cβ1 and anti-PD1 antibodies and analyzed by flow cytometry.

Binding of TCR Cβ1 antibodies to transduced Jurkat biosensor cells indicated efficient transduction with high expression levels of TCR 16 alone as well as in combination with PD1-41BB CSP independently of UniTope inclusion (**Figure 15**). Also binding of PD1 antibody was independent of UniTope inclusion and restricted to TCR 16 + PD1-41BB-transduced Jurkat biosensor cells. These results demonstrated that co-expression of the PD1-41BB CSP and the transgenic TCR was possible in Jurkat biosensor cells and not influenced by the presence of the UniTope.

### mKRAS₇₋₁₆ G12D (10-mer) multimer binding is not affected by UniTope presence

Multimer binding was performed to gain first insights into transgenic TCR functionality after UniTope insertion. Therefore, untagged TCR 16-transduced (± PD1-41BB CSP co-expression) and UniTope tagged TCR 16-transduced (± PD1-41BB CSP co-expression) Jurkat biosensor cells were stained with mKRAS₇₋₁₆G120 (10-mer) HLA-A*11:01 multimer and analyzed by flow cytometry.

Results showed high and robust binding of TCR 16 (± PD1-41BB CSP co-expression) independently of UniTope presence to HLA-A*11:01 multimer loaded with mKRAS₇₋₁₆ G12D (10-mer) peptide, while untransduced Jurkat biosensor cells were not labelled by multimer staining (**Figure 16**).

### Specific and reliable detection of UniTope tagged TCR-transduced Jurkat biosensor cells

The insertion of the UniTope allows specific detection and reliable tracking of UniTope tagged TCR 16-expressing effector cells. Therefore, untagged TCR 16-transduced (± PD1-41BB CSP co-expression) and UniTope tagged TCR 16-transduced (± PD1-41BB CSP co-expression) Jurkat biosensor cells were stained with the UniTope-specific TraCR antibody and analyzed for specificity of the staining by flow cytometry.

Detection was highly specific for Jurkat biosensor cells transduced with UniTope tagged TCR 16 constructs (± PD1-41BB CSP co-expression), while no staining was observed for untransduced Jurkat biosensor cells or Jurkat biosensor cells transduced with untagged TCR 16 constructs (± PD1-41BB CSP co-expression) (**Figure 17**).

### Specificity and sensitivity for mKRAS₇₋₁₆ G12D (10-mer) peptide are not affected by UniTope presence.

In the Jurkat biosensor cell line peptide sensitivity can be easily evaluated by measuring the expression of eGFP in response to binding of the transgenic TCR to a specific peptide-MHC complex. In detail, the Jurkat biosensor cell line contains an inducible eGFP gene expression cassette under the control of a nuclear factor of activated T cells (NFAT) responsive element. Upon activation of the Jurkat biosensor cell line by binding of the transgenic TCR to a specific peptide-MHC complex, eGFP is expressed and can be measured spectrofluorometrically. Since the degree of activation and thus the peptide sensitivity directly correlates with the amount of expressed eGFP, Jurkat biosensor cells are a simple way to compare the functionality of different transgenic TCRs. Here, the Jurkat biosensor assay was used to demonstrate comparable functionality of UniTope tagged TCR 16 (± PD1-41BB CSP co-expression) and untagged TCR 16 (± PD1-41BB CSP co-expression).

Therefore, TCR 16- and TCR 16 + PD1-41 BB-transgenic Jurkat biosensor cells with and without UniTope were co-cultured with K562_A11 cells loaded with titrated amounts of mKRAS₇₋₁₆ G12D (10-mer) peptide (10⁻⁵ M to 10⁻⁹ M). Target cells were also assessed either without peptide loading or loaded with KRAS₇₋₁₆ wild type (10-mer) peptide as internal controls. PMA/lono stimulation of Jurkat biosensor cells served as positive control. eGFP expression was analyzed after 20h of co-culture.
Both, TCR 16 and TCR 16 + PD1-41BB showed high sensitivity for the mKRAS₇₋₁₆ G12D (10-mer) peptide independently of UniTope presence (**Figure 18A**), while no recognition of KRAS₇₋₁₆wild type (10-mer) was observed (**Figure 18B**). These findings confirmed the maintenance of specificity of TCR 16 (± PD1-41BB CSP co-expression) as well as high sensitivity towards the mKRAS₇₋₁₆ G12D (10-mer) peptide after UniTope insertion.

### References

1. Sommermeyer, D.; Uckert, W. Minimal Amino Acid Exchange in Human TCR Constant Regions Fosters Improved Function of TCR Gene-Modified T Cells. J. Immunol. 2010, 184, 6223-6231.
2. Leisegang, M.; Engels, B.; Meyerhuber, P.; Kieback, E.; Sommermeyer, D.; Xue, S.-A.; Reuß, S.; Stauss, H.; Uckert, W. Enhanced functionality of T cell receptor-redirected T cells is defined by the transgene cassette. J. Mol. Med. 2008, 86, 573-583.
3. Scholten, K.B.; Kramer, D.; Kueter, E.W.; Graf, M.; Schoedl, T.; Meijer, C.J.; Schreurs, M.W.; Hooijberg, E. Codon modification of T cell receptors allows enhanced functional expression in transgenic human T cells. Clin. Immunol. 2006, 119, 135-145.
4. Riddell, S.R.; Greenberg, P.D. The use of anti-CD3 and anti-CD28 monoclonal antibodies to clone and expand human antigen-specific T cells. J. Immunol. Methods 1990, 128, 189-201.
5. Wilde, S.; Sommermeyer, D.; Frankenberger, B.; Schiemann, M.; Milosevic, S.; Spranger, S.; Pohla, H.; Uckert, W.; Busch, D.H.; Schendel, D.J. Dendritic cells pulsed with RNA encoding allogeneic MHC and antigen induce T cells with superior anti-tumor activity and higher TCR functional avidity. Blood 2009, 114, 2131-2139.
6. Wilde, S.; Geiger, C.; Milosevic, S.; Mosetter, B.; Eichenlaub, S.; Schendel, D.J. Generation of allo-restricted peptide-specific T cells using RNA-pulsed dendritic cells: A three phase experimental procedure. Oncoimmunology 2012, 1, 129-140.

## Claims

1. A T-cell receptor (TCR) comprising:
i) an α chain Complementarity Determining Region 3 (CDR3) comprising an amino acid sequence having at least 80 % identity to SEQ ID NO: 16 (CAVSRSGNTPLVF);
ii) a β chain Complementarity Determining Region 3 (CDR3) comprising an amino acid sequence having at least 80 % identity to SEQ ID NO: 4 (CASSGAGETGELFF);
iii) an α chain Complementarity Determining Region 1 (CDR1) comprising an amino acid sequence having at least 80 % identity to SEQ ID NO: 14 (SSVSVY);
iv) an α chain Complementarity Determining Region 2 (CDR2) comprising an amino acid sequence having at least 80 % identity to SEQ ID NO: 15 (YLSGSTLV);
v) a β chain CDR1 comprising an amino acid sequence having at least 80 % identity to SEQ ID NO: 2 (WNHNN); and
vi) a β chain CDR2 comprising an amino acid sequence having at least 80 % identity to SEQ ID NO: 3 (SYGVHD).

2. The TCR of claim 1, comprising:
i) an α chain Complementarity Determining Region 3 (CDR3) comprising an amino acid sequence having SEQ ID NO: 16 (CAVSRSGNTPLVF); and
ii) a β chain Complementarity Determining Region 3 (CDR3) comprising an amino acid sequence having SEQ ID NO: 4 (CASSGAGETGELFF);
wherein said TCR further comprises the following:
a) an α chain Complementarity Determining Region 1 (CDR 1) comprising an amino acid sequence having SEQ ID NO: 14 (SSVSVY);
b) an α chain Complementarity Determining Region 2 (CDR2) comprising an amino acid sequence having SEQ ID NO: 15 (YLSGSTLV);
c) a β chain CDR1 comprising an amino acid sequence having SEQ ID NO: 2 (WNHNN); and
d) a β chain CDR2 comprising an amino acid sequence having SEQ ID NO: 3 (SYGVHD).

3. The TCR of any one of the preceding claims, comprising:
i) an α chain variable region comprising the amino acid sequence of SEQ ID NO: 20, or an amino acid sequence having at least 80 % identity to SEQ ID NO: 20; and/or
ii) a β chain variable region comprising the amino acid sequence of SEQ ID NO: 8, or an amino acid sequence having at least 80 % identity to SEQ ID NO: 8.

4. The TCR of any one of the preceding claims, having antigenic specificity to an epitope presented in the context of a MHC class I molecule, the epitope comprising the amino acid sequence of VVVGADGVGK (SEQ ID NO: 1), or a fragment thereof or a variant thereof comprising one or more (e.g. one or two) conservative amino acid substitutions (preferably corresponding to position(s) 1, 2, 3, 4, 5, 6, 7, 8, 9 and/or 10 of SEQ ID NO: 1).

5. The TCR of claim 4, wherein the TCR has antigenic specificity for said epitope presented in the context of:
iv) an HLA-A molecule, preferably HLA-A*11 molecule;
v) an HLA-A*11:01 molecule; and/or
vi) at least any one of an HLA-A*11 molecule, preferably HLA-A*11:01, HLA-A*11:02, HLA-A*11:03 and/or HLA-A*11:12.

6. A nucleic acid and/or vector, preferably expression vector, comprising a nucleotide sequence encoding the TCR of any one of the preceding claims.

7. A host cell (e.g., isolated and/or recombinant host cell) comprising the TCR of any one of claims 1 to 6 and/or the nucleic acid or vector of claim 6.

8. A method for obtaining the TCR according to any one of claims 1 to 5, comprising
a) incubating the host cell of claim 7 under conditions causing expression of said TCR; and
b) purifying said TCR.

9. A composition and/or kit, preferably a pharmaceutical and/or diagnostic composition, comprising one or more of the following:
i) the TCR of any one of claims 1 to 5;
ii) the nucleic acid and/or vector of claim 6; and/or
iii) the host cell of claim 7;
and, optionally, pharmaceutically excipient(s).

10. The TCR of any one of claims 1 to 5, the nucleic acid and/or vector of claim 6, the host cell of claim 7 and/or the composition of claim 9 for use as a medicament and/or for use in therapy.

11. A method of detecting the presence of a cancer in a subject *in vitro,* comprising:
(a) contacting a sample obtained from a subject and comprising one or more cells with:
i) the TCR of any one of claims 1 to 5;
ii) the nucleic acid and/or vector of claim 6;
iii) the host cell of claim 7, and/or
iv) the composition of claim 9, preferably a pharmaceutical composition;
thereby forming a complex, and
(b) detecting the complex,
wherein detection of the complex is indicative of the presence of the cancer in the subject.

12. A T-cell receptor (TCR) polypeptide complex and/or a combination comprising:
a) a TCR, preferably the TCR of any one of claims 1 to 5; and
b) a chimeric co-stimulatory receptor,
wherein the TCR comprises:
i) an α chain Complementarity Determining Region 3 (CDR3) comprising an amino acid sequence having at least 80 % identity to SEQ ID NO: 16;
ii) a β chain Complementarity Determining Region 3 (CDR3) comprising an amino acid sequence having at least 80 % identity to SEQ ID NO: 4;
iii) an α chain Complementarity Determining Region 1 (CDR 1) comprising an amino acid sequence having at least 80 % identity to SEQ ID NO: 14 (SSVSVY);
iv) an α chain Complementarity Determining Region 2 (CDR2) comprising an amino acid sequence having at least 80 % identity to SEQ ID NO: 15 (YLSGSTLV);
v) a β chain CDR1 comprising an amino acid sequence having at least 80 % identity to SEQ ID NO: 2 (WNHNN); and
vi) a β chain CDR2 comprising an amino acid sequence having at least 80 % identity to SEQ ID NO: 3 (SYGVHD).

13. A cell population comprising cells or a cell expressing:
a) a TCR, preferably the TCR of any one of claims 1 to 5; and
b) a chimeric co-stimulatory receptor,
wherein the TCR comprises:
i) an α chain Complementarity Determining Region 3 (CDR3) comprising an amino acid sequence having at least 80 % identity to SEQ ID NO: 16;
ii) a β chain Complementarity Determining Region 3 (CDR3) comprising an amino acid sequence having at least 80 % identity to SEQ ID NO: 4;
iii) an α chain Complementarity Determining Region 1 (CDR1) comprising an amino acid sequence having at least 80 % identity to SEQ ID NO: 14 (SSVSVY);
iv) an α chain Complementarity Determining Region 2 (CDR2) comprising an amino acid sequence having at least 80 % identity to SEQ ID NO: 15 (YLSGSTLV);
v) a β chain CDR1 comprising an amino acid sequence having at least 80 % identity to SEQ ID NO: 2 (WNHNN); and
vi) a β chain CDR2 comprising an amino acid sequence having at least 80 % identity to SEQ ID NO: 3 (SYGVHD).

14. The complex and/or the combination, and/or the cell population or the cell of any one of the preceding claims, wherein the TCR comprises:
i) an α chain variable region comprising the amino acid sequence of SEQ ID NO: 20, or an amino acid sequence having at least 80 % identity to SEQ ID NO: 20; and/or
ii) a β chain variable region comprising the amino acid sequence of SEQ ID NO: 8, or an amino acid sequence having at least 80 % identity to SEQ ID NO: 8.

15. The complex and/or the combination, and/or the cell population or the cell of any one of the preceding claims, wherein the TCR has antigenic specificity to an epitope presented in the context of a MHC class I molecule, the epitope comprising the amino acid sequence of VVVGADGVGK (SEQ ID NO: 1), or a fragment thereof or a variant thereof comprising one or more (e.g. one or two) conservative amino acid substitutions (preferably corresponding to position 1, 2, 3, 4, 5, 6, 7, 8, 9 and/or 10 and/or SEQ ID NO: 1), optionally wherein the TCR has antigenic specificity for said epitope presented in the context of:
iv) an HLA-A molecule, preferably HLA-A*11 molecule;
v) an HLA-A*11:01 molecule; and/or
vi) at least any one of an HLA-A*11 molecule, preferably HLA-A*11:01, HLA-A*11:02, HLA-A*11:03 and/or HLA-A*11:12.

16. The complex and/or the combination and/or the cell population or the cell of any one of the preceding claims, wherein the chimeric co-stimulatory receptor comprises:
i) an extracellular domain comprising a polypeptide derived from PD-1 at its N-terminus (e.g., SEQ ID NO: 28);
ii) a transmembrane domain; and/or
iii) an intracellular domain comprising a polypeptide derived from 4-1BB at its C-terminus (e.g., SEQ ID NO: 32).
